# EUROPÄISCHE PATENTSCHRIFT

(11) **EP 1 599 453 B1**
(45) Veröffentlichungstag und Bekanntmachung des Hinweises auf die Patenterteilung: **06.05.2009**
(21) Anmeldenummer: 04712494.6
(22) Anmeldetag: 19.02.2004
(51) Int. Cl.: C07D 263/32, A61K 31/421, A61P 3/10, C07D 413/12, A61K 31/422, A61P 9/10

(54) **3-METHYL-2- (3- (2-PHENYL-OXAZOL-4-YLMETHOXY)-CYCLOHEXANCARBONYL-AMINO)-BUTTERSÄUREDERIVATE UND VERWANDTE VERBINDUNGEN ALS PPAR MODULATOREN ZUR BEHANDLUNG VON TYP 2 DIABETES UND ATHEROSKLEROSE**
3-METHYL-2- (3- (2-PHENYL-OXAZOL-4-YLMETHOXY)-CYCLOHEXANECARBONYL-AMINO BUTYRIC ACID DERIVATIVES AND RELATED COMPOUNDS AS PPAR MODULATORS FOR THE TREATMENT OF TYPE 2 DIABETES AND ATHEROSCLEROSIS
DERIVES D'ACIDE 3-METHYL-2- (3- (2-PHENYL-OXAZOL-4-YLMETHOXY)-CYCLOHEXANCARBONYL-AMINO)-BUTYRIQUE ET COMPOSES APPARENTES SERVANT DE MODULATEURS DE PPAR POUR TRAITER DES DIABETES DE TYPE 2 ET DES ATHEROSCLEROSES

(30) Priorität: 27.02.2003 DE 10308355
(43) Veröffentlichungstag der Anmeldung: 30.11.2005
(73) Patentinhaber: Sanofi-Aventis Deutschland GmbH, 65929 Frankfurt am Main (DE)
(72) Erfinder: STAPPER, Christian, 55118 Mainz (DE); GRETZKE, Dirk, 60596 Frankfurt (DE); FALK, Eugen, 60529 Frankfurt (DE); GOERLITZER, Jochen, 60594 Frankfurt am Main (DE); KEIL, Stefanie, 65719 Hofheim (DE); SCHAEFER, Hans-Ludwig, 65239 Hochheim (DE); GLOMBIK, Heiner, 65719 Hofheim (DE); WENDLER, Wolfgang, 65618 Selters (DE)
(86) Internationale Anmeldenummer: PCT/EP2004/001578
(87) Internationale Veröffentlichungsnummer: WO 2004/076426

(56) Entgegenhaltungen:
- WO-A-00/64876
- WO-A-02/100403

## Beschreibung

Die Erfindung betrifft Cycloalkyl substituierte Aminosäurederivate sowie deren physiologisch verträgliche Salze.

Es sind bereits strukturähnliche Verbindungen zur Behandlung von Hyperlipidämie und Diabetes im Stand der Technik beschrieben (WO 2000/64876). PPAR Agonisten enthaltend einen Phenyl-Zentralbaustein sind aus WO02/100403 bekannt.

Der Erfindung lag die Aufgabe zu Grunde, Verbindungen zur Verfügung zu stellen, die eine therapeutisch verwertbare Modulation des Lipid- und/oder Kohlehydratstoffwechsels erlauben und sich somit zur Prävention und/oder Behandlung von Krankheiten wie Typ 2 Diabetes und Atherosklerose sowie deren vielfältigen Folgeerkrankungen eignen.

Überraschenderweise wurde eine Serie von Verbindungen gefunden, die die Aktivität von PPAR Rezeptoren modulieren. Insbesondere eignen sich die Verbindungen zur Aktivierung von PPARalpha und PPARgamma, wobei das Ausmaß der relativen Aktivierung je nach Verbindungen unterschiedlich sein kann.

Die Erfindung betrifft daher Verbindungen der Formel I 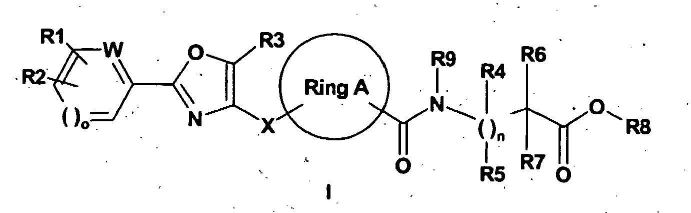 worin bedeuten:
- Ring A: Cyclohexan-1,3-diyl;
- R1, R2: unabhängig voneinander H, F, Cl, Br, CF₃, OCF₃, (C1-C6)-Alkyl, O-(C1- C6)-Alkyl, SCF₃, SF₅, OCF₂-CHF₂, (C6-C10)-Aryl, (C6-C10)-Aryloxy, OH, NO₂; oder
- R1 und R2: zusammen genommen mit dem Phenyl-, Pyridin-, 1-H-Pyrrol-, Thiophen- oder Furan-Ring kondensiertes, teilweise oder nicht gesättigtes bicyclisches (C6-C10)-Aryl, (C5-C11)-Heteroaryl;
- R3: H, (C1-C6)-Alkyl, (C3-C8)-Cycloalkyl, (C1-C3)-Alkyl-(C3-C8)-Cycloalkyl, Phenyl, (C1-C3)-Alkyl-Phenyl, (C5-C6)-Heteroaryl, (C1-C3)-Alkyl-(C5-C6)- Heteroaryl oder (C1-C3)-Alkyl, das durch F ganz oder teilweise substituiert ist;
- O: O oder 1;
- W: CH, N, falls o = 1;
- W: O, S, NR10, falls o = 0;
- X: (C1-C6)-Alkandiyl, wobei in der Alkandlylgruppe ein oder mehrere Kohlenstoffatome durch Sauerstoffatome ersetzt sein können;
- n: 0 - 2;
- R4: H, (C1-C6)-Alkyl;
- R5: H, (C1-C6)-Alkyl;
- R6: H, (C1-C6)-Alkyl, F;
- R7: H, F, (C1-C6)-Alkyl, (C1-C6)-Alkoxy, (C2-C6)-Alkenyl, (C2-C6)-Alkinyl, (C3-C8)-Cycloalkyl, Phenyl, wobei Alkyl gegebenenfalls substituiert sein kann durch ein oder mehrere Reste aus der Reihe Hydroxy, Phenyl, (C5- C11)-Heteroaryl, (C1-C6)-Alkoxy und NR11R12, und Phenyl gegebenfalls substituiert sein kann durch ein oder mehrere Reste aus der Reihe Hydroxy, (C1-C6)-Alkoxy, F und CF₃; mit der Maßgabe, dass R7 ungleich NR11R12 oder (C1-C6)-Alkoxy, falls R6 = F ist;
- R7 und R9: zusammen mit den sie tragenden Atomen Pyrrolidin oder Piperidin, falls n = 0;
- R6 und R7: zusammen mit dem sie tragenden C-Atom (C3-C8)-Cycloalkyl;
- R8: H, (C1-C6)-Alkyl;
- R9: H, (C1-C6)-Alkyl, (C2-C6)-Alkenyl, (C2-C6)-Alkinyl, (C1-C4)-Alkyl-(C6- C10)-Aryl, (C1-C4)-Alkyl-(C5-C11)-Heteroaryl, (C1-C4)-Alkyl-O-(C1-C4)- Alkyl, Phenyl-(C1-C4)-Alkyl, (C5-C6)-Heteroaryl-(C1-C4)-Alkyl;
- R10: H, (C1-C6)-Alkyl-Phenyl, (C1-C6)-Alkyl;
- R11: H, (C1-C6)-Alkyl-Phenyl, (C1-C6)-Alkyl;
- R12: H, (C1-C6)-Alkyl-Phenyl, (C1-C6)-Alkyl;
sowie deren physiologisch verträgliche Salze.

Bevorzugt sind Verbindungen der Formel I, in denen
- Ring A: Cyclohexan-1,3-diyl;
- X: (C1-C6)-Alkandiyl, wobei in der Alkandiylgruppe das C1- oder C2- Kohlenstoffatom (zum Ring A) durch Sauerstoffatom ersetzt ist.

Besonders bevorzugt sind die Verbindungen der Formel I, worin ein oder mehrere Reste die folgenden Bedeutungen haben:
- Ring A: Cyclohexan-1,3-diyl; oder
- R1: F, Br, CF₃, OCF₃, (C1-C6)-Alkyl, O-(C1-C6)-Alkyl, Phenyl; oder
solche, in denen der Substituent
- R1: in meta- oder in para-Stellung steht; oder
- R2: Wasserstoff ist; oder
- R1 und R2: zusammen mit dem Phenylring = Naphthyl; oder
- R3: H, (C1-C6)-Alkyl, (C3-C8)-Cycloalkyl, (C1-C3)-Alkyl-(C5-C6)-Cycloalkyl, Phenyl, (C1-C3)-Alkyl-Phenyl; oder
- W: CH, falls o = 1; oder
- X: CH₂-O oder CH₂-O-CH₂; oder
- n: 0; oder
- R6: H, (C1-C6)-Alkyl; oder
- R6 und R7: zusammen mit dem sie tragenden C-Atom (C3-C6)-Cycloalkyl, besonders Cyclopentyl;
- R7: (C1-C6)-Alkyl, wobei Alkyl gegebenenfalls substituiert sein kann durch ein oder mehrere Reste aus der Reihe Hydroxy, Phenyl, (C5-C11)-Heteroaryl, (C1-C6)-Alkoxy und NR11R12, mit
- R11 und R12: H, (C1-C6)-Alkyl.

Besonders bevorzugt sind ferner die Verbindungen der Formel I, worin bedeuten
- R7: (C1-C4)-Alkyl, (C1-C4)-Alkyl-O-(C1-C4)-Alkyl oder Benzyl;
ganz besonders bevorzugt
- R7: (C1-C4)-Alkyl oder Benzyl.

Ganz besonders bevorzugt sind auch Verbindungen der Formel I,
worin bedeuten
- Ring A: cis-Cyclohexan-1,3-diyl;
- R1: Br, CF₃, OCF₃, (C1-C6)-Alkyl, O-(C1-C6)-Alkyl;
- R2: H, (C1-C6)-Alkyl, O-(C1-C6)-Alkyl oder
- R1 und R2: zusammen mit dem Phenylring = Naphthyl;
- R3: CF₃, (C1-C6)-Alkyl, (C3-C6)-Cycloalkyl, Phenyl;
- W: CH, falls o = 1;
- X: CH₂O, CH₂-O-CH₂;
- n: 0
- R6: H, (C1-C6)-Alkyl;
- R7: (C1-C6)-Alkyl, wobei Alkyl gegebenenfalls substituiert sein kann durch Phenyl;
- R7 und R9: zusammen mit den sie tragenden Atomen Pyrrolidin, falls n = 0;
- R6 und R7: zusammen mit dem sie tragenden C-Atom (C3-C6)-Cycloalkyl;
- R8: H,
- R9: H, (C1-C6)-Alkyl, Benzyl;
sowie deren physiologisch verträgliche Salze.

Die Alkylreste in den Substituenten R1, R2, R3, R4, R5 , R6, R7, R8, R9, R10, R11 und R12 können sowohl geradkettig wie verzweigt sein.

Unter Aryl wird ein aromatisches carbocyclisches mono- oder bicyclisches RingSystem verstanden, das 6 bis 10 Atome im Ring oder in den Ringen enthält.

Heteroaryl ist ein mono- oder bicyclisches aromatisches Ringsystem mit 4 bis 11 Ringgliedern, worin mindestens ein Atom im Ringsystem ist ein Heteroatom aus der Reihe N, O und S.

Die Verbindungen der Formel I enthalten mindestens zwei Assymmetriezentren und können darüber hinaus mehr enthalten. Daher können die Verbindungen der Formel I in Form ihrer Racemate, racemischen Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organische Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel' Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

### Verwendung

Diese Erfindung bezieht sich weiterhin auf die Verwendung von Verbindungen der Formeln I und ihren pharmazeutischen Zusammensetzungen als PPAR-Rezeptor-Liganden. Die erfindungsgemäßen PPAR-Rezeptor-Liganden eignen sich als Modulatoren der Aktivität der PPAR Rezeptoren.

Peroxisomen-Proliferatoren-Aktivierte Rezeptoren (PPAR) sind durch Liganden aktivierbare Transkriptionsfaktoren, die zur Klasse der Kern-Hormon-Rezeptoren gehören. Es existieren drei PPAR Isoformen, PPARalpha, PPARgamma und PPARdelta, die von verschiedenen Genen kodiert werden (Peroxisome proliferator-activated receptor (PPAR): structure, mechanisms of activation and diverse functions: Motojima K, Cell Struct Funct. 1993 Oct; 18(5): 267-77).

Von PPARgamma existieren zwei Varianten, PPARgamma₁ und gamma₂, die das Ergebnis eines alternativen Einsatzes von Promotoren und einer differenziellen mRNA-Spleißung (Vidal-Puig et al. J. Clin. Invest., 97:2553-2561, 1996) sind. Die verschiedenen PPAR Rezeptoren besitzen eine unterschiedliche Gewebsverteilung und modulieren unterschiedliche physiologische Funktionen. Die PPAR-Rezeptoren spielen eine Schlüsselrolle bei unterschiedlichen Aspekten der Regulation einer Vielzahl von Genen, deren Genprodukte direkt oder indirekt am Lipid- und Kohlehydratstoffwechsel entscheidend beteiligt sind. So spielen z. B. PPARalpha Rezeptoren bei der Regulation des Fettsäurekatabolismus oder des Lipoproteinstoffwechsels in der Leber eine wichtige Rolle, während PPARgamma zum Beispiel bei der Regulation der Fettzelldifferenzierung entscheidend beteiligt ist. Darüberhinaus sind PPAR Rezeptoren aber auch an der Regulation vieler weiterer physiologischer Prozesse, auch solcher die nicht direkt mit dem Kohlehydrat- oder Lipidstoffwechsel in Verbindung stehen, beteiligt. Die Aktivität der unterschiedlichen PPAR Rezeptoren kann durch verschiedene Fettsäuren, Fettsäurederivate sowie synthetische Verbindungen in unterschiedlichem Ausmaß moduliert werden.

Entsprechende Reviews über Funktionen, physiologische Wirkung und Pathophysiologie siehe: Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63.

Die vorliegende Erfindung betrifft Verbindungen der Formel I, die sich zur Modulierung der Aktivität von PPAR Rezeptoren eignen, insbesondere der Aktivität von PPARalpha und PPARgamma. Je nach Profil der Modulation sind die Verbindungen der Formel I zur Behandlung, Kontrolle und Prohylaxe nachfolgend beschriebener Indikationen, sowie einer Reihe anderer, damit verbundener pharmazeutischer Anwendungen geeignet (siehe z.B. Joel Berger et al., Annu. Rev. Med. 2002, 53, 409 - 435; Timothy Wilson et al. J. Med. Chem., 2000, Vol. 43, No. 4, 527-550; Steven Kliewer et al., Recent Prog Horm Res. 2001; 56: 239-63; Jean-Charles Fruchart, Bart Staels and Patrick Duriez: PPARS, Metabolic Disease and Arteriosclerosis, Pharmacological Research, Vol. 44, No. 5, 2001; Sander Kersten, Beatrice Desvergne & Walter Wahli: Roles of PPARs in health and disease, NATURE, VOL 405 ,25 MAY 2000 ; Ines Pineda Torra, Giulia Chinetti, Caroline Duval, Jean-Charles Fruchart and Bart Staels: Peroxisome proliferator-activated receptors: from transcriptional control to clinical practice, Curr Opin Lipidol 12: 2001 , 245-254).

Besonders geeignet sind solche Verbindungen zur Behandlung und/oder Prävention von
1. Störungen des Fettsäurestoffwechsels und Glucoseverwertungsstörungen - Störungen, bei denen Insulin Resistenz eine Rolle spielt
2. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der β-Zellen der Bauclispeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
4. Verschiedene andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
5. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzeti-Karzimone, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und hamableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminatä
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrome X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg und 50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch Verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formeln I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10.Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glylcogenolyse beteiligt sind, Modulatoren der Glukoseaufnahme, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I in Kombination mit Insulin verabreicht.

Bei einer Ausführungsform der Erfindung werden die Verbindungen der Formel I in Kombination mit Substanzen, die Einfluss haben auf die hepatische Glukoseproduktion, wie z.B. Glycogen Phosphorylase Inhibitoren (siehe: WO 01/94300, WO 02/096864, WO 03/084923, WO 03/084922, WO 03/104188) Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform werden die Verbindungen der Formel I in Kombination mit einem DPPIV Inhibitor, wie z.B. in WO98/19998, WO99/61431, WO99/67278, WO99/67279, WO01/7229 WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel I in Kombination mit einem PPARgamma Agonist, wie z.B. Rosiglitazon, Pioglitazon, verabreicht.

Bei einer Aufführungsform werden die Verbindungen der Formel I in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor (siehe z.B. US 6,245,744, US 6,221,897, US 6,277,831, EP 0683 773, EP 0683 774) verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin. Bei noch einer Ausführungsform ist der weitere Wirkstoff Sibutramin.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-hamstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten) verabreicht.

Bei einer Ausführungsform der Erfindung ist der weitere Wirkstoff Leptin.

Bei einer Ausführungsform ist der weitere Wirkstoff Dexamphatamin, Amphetamin, Mazindol oder Phentermin.

Bei einer Ausführungsform werden die Verbindungen der Formel I in Kombination mit Medikamenten mit Wirkungen auf das Herz-Kreislauf- und das Blutgefäß-System, wie verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der Verbindungen wurde wie folgt getestet:

### Bestimmung von EC50-Werten von PPAR-Agonisten im zellulären PPARalpha-Test

### Prinzip

Für die Analyse der Wirkstärke von Substanzen, die an humanes PPARalpha binden und es in agonistischer Weise aktivieren, wird eine stabil transfizierte HEK-Zellinie (HEK= human embryo kidney) benutzt, die hier als PPARalpha-Reporterzellinie bezeichnet wird. Sie enthält zwei genetische Elemente, ein Luziferase-Reporterelement (pdeltaM-GAL4-Luc-Zeo) und ein PPARalpha-Fusionsprotein (GR-GAL4-humanPPARalpha-LBD), welches die Expression des Luziferase-Reporterelementes in Abhängigkeit eines PPARalpha-Liganden vermittelt. Das stabil und konstitutiv exprimierte Fusionsprotein GR-GAL4-humanPPARalpha-LBD bindet im Zellkern der PPARalpha-Reporterzellinie über den GAL4-Proteinanteil an die GAL4-DNA-Bindungsmotife 5'-oberhalb des Luziferase-Reporterelementes, das im Genom der Zellinie integriert ist. Ohne Zugabe eines PPARalpha-Liganden ist die Expression des Luziferase-Reportergens nur gering, sofern im Test fettsäuredepletiertes fötales Kälberserum (cs-FKS) verwendet wird. PPARalpha-Liganden binden und aktivieren das PPARalpha-Fusionsprotein und bewirken darüber die Expression des Luciferasereportergens. Die gebildete Luziferase lässt sich über ein entsprechendes Substrat mittels Chemilumineszenz nachweisen.

### Konstruktion der Zelllinie

Die PPARalpha-Reporterzellinie wurde in 2 Stufen hergestellt: Zuerst wurde das Luziferase-Reporterelement konstruiert und stabil in HEK-Zellen transfiziert. Dazu wurden fünf Bindungsstellen des Hefe-Transkriptionsfaktors GAL4 (jeweils 5'-CGGAGTACTGTCCTCCGAG-3') 5'-oberhalb eines 68 bp langen minimalen MMT\/- Promotors (Genbank-Accession # V01175) einkloniert. Der minimale MMTV-Promotörabschnitt enthält eine CCAAT-Box und ein TATA-Element, um eine effiziente Transkription durch die RNA-Polymerase II zu ermöglichen. Die Klonierung und Sequenzierung des GAL4-MMTV-Konstruktes erfolgte analog wie bei Sambrook J. et. al. beschrieben (Molecular cloning, Cold Spring Harbor Laboratory Press, 1989). Danach wurde 3'-unterhalb des GAL4-MMTV-Elementes das gesamte Luziferasegen von Photinus pyralis (Genbank-Accession # M15077) einkloniert. Nach der Sequenzierung wurde das aus fünf GAL4-Bindungsstellen, MMTV-Promotor und Luziferasegen bestehende Luziferase-Repprterelement in ein Zeozin-Resistenz vermittelndes Plasmid umkloniert, um zu dem Plasmid pdeltaM-GAL4-Luc-Zeo zu gelangen. Dieser Vektor wurde nach den Angaben in Ausubel, F.M. et al. (Current protocols in molecular biology, Vol. 1-3, John Wiley & Sons, Inc., 1995) in HEK-Zellen transfiziert. Danach wurde unter Verwendung von zeozinhaltigem Medium (0,5 mg/ml) ein geeigneter stabiler Zellklon selektioniert, der eine möglichst niedrige Basalexpression des Luziferasegens zeigte.

In einem zweiten Schritt wurde das PPARalpha-Fusionsprotein (GR-GAL4-humanPPARalpha-LBD in den beschriebenen stabilen Zellklon eingebracht. Dazu wurde zunächst die für die N-terminalen 76 Aminosäuren des Glukocorticoid-Rezeptors (Genbank-Accession # P04150) kodierende cDNA mit dem für die Aminosäuren 1-147 des Hefetranskriptionsfaktors GAL4 (Genbank-Accession # P04386) kodierenden cDNA-Abschnitt verknüpft. Am 3'-Ende dieses GR-GAL4-Konstruktes wurde die cDNA der Ligandenbindungsdomäne des humanen PPARalpha-Rezeptors einkloniert (Aminosäuren S167-Y468; Genbank-Accession # S74349). Das so hergestellte Fusionskonstrukt (GR-GAL4-humanPPARalpha-LBD) wurde in das Plasmid pcDNA3 (Firma Invitrogen) umkloniert, um darin eine konstitutive Expression durch den Cytomegalovirus-Promotor zu ermöglichen. Dieses Plasmid wurde mit einer Restriktionsendonuklease linearisiert und stabil in den bereits beschriebenen, das Luziferase-Reporterelement enthaltenden Zellklon transfizierf. Durch Selektion mit Zeozin (0,5 mg/ml) und G418 (0,5 mg/ml) wurde die fertige PPARalpha-Reporterzellinie isoliert, die ein Luziferase-Reporterelement enthält und konstitutiv das PPARalpha-Fusionsprotein (GR-GAL4-humanPPARalpha-LBD) exprimiert.

### Durchführung des Tests

Die Aktivität von PPARalpha-Agonisten wird in einem 3-Tagestest bestimmt, der nachfolgend beschrieben ist:

### Tag 1

Die PPARalpha-Reporterzellinie wird bis zu einer 80 %-igen Konfluenz in DMEM-Medium (# 41965-039, Invitrogen) kultiviert, das mit folgenden Zusätzen versetzt ist: 10% cs-FKS (fötales Kälberserum; #SH-30068.03, Hyclone), 0,5 mg/ml Zeozin (#R250-01, Invitrogen), 0,5 mg/ml G418 (#10131-027, Invitrogen), 1% Penicillin-Streptomycin-Lösung (#15140-122, Invitrogen) und 2 mM L-Glutamin (#25030-024, Invitrogen). Die Kultivierung erfolgt in Standard-Zellkulturflaschen (# 353112, Becton Dickinson) in einem Zellkulturbrutschrank bei 37°C in Anwesenheit von 5% CO₂. Die zu 80% konfluenten Zellen werden einmal mit 15 ml PBS gewaschen (#14190-094, Invitrogen), mit 3 ml Trypsinlösung (#25300-054, Invitrogen) für 2 min bei 37°C behandelt, in 5 ml des beschriebenen DMEM-Mediums aufgenommen und in einem Zellzählgerät gezählt. Nach der Verdünnung auf 500.000 Zellen/ml werden jeweils 35.000 Zellen pro well einer 96 well-Mikrotiterplatte mit klarem Plastikboden (#3610, Corhing Costar) ausgesät. Die Platten werden für 24 h in einem Zellkulturbrutschrank bei 37°C und 5% CO₂ inkubiert.

### Tag 2

Zu testende PPARalpha-Agonisten werden in einer Konzentration von 10 mM in DMSO gelöst. Diese Stocklösung wird in DMEM-Medium (#41965-039, Invitrogen) verdünnt, das mit 5 % cs-FKS (#SH-30068.03, Hyclone), 2 mM L-Glutamin (#25030-024, Invitrogen) und den bereits beschriebenen Antibiotika (Zeozin, G418, Penicillin und Streptomycin) versetzt ist.

Testsubstanzen werden in 11 verschiedenen Konzentrationen im Bereich von 10 µM bis 100 pM getestet. Potentere Verbindungen werden in Konzentrationsbereichen von 1 µM bis 10 pM oder zwischen 100 nM und 1 pM geprüft.

Das Medium der an Tag 1 ausgesäten PPARalpha-Reporterzellinie wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Die DMSO-Konzentration in dem Test beträgt unter 0.1 % v/v, um zelltoxische Effekte des Lösungsmittels zu vermeiden. Jede Platte wurde mit einem Standard PPARalpha-Agonisten belegt, der ebenfalls in 11 verschiedenen Konzentrationen verdünnt wird, um die Funktionsfähigkeit des Tests in jeder Einzelplatte nachzuweisen. Die Testplatten werden für 24 h in einem Brutschrank bei 37 °C und 5 % CO₂ inkubiert.

### Tag 3

Die mit den Testsubstanzen behandelten PPARalpha-Reporterzellen werden aus dem Brutschrank entnommen und das Medium abgesaugt. Zur Lyse der Zellen werden 50 µl Bright Glo Reagens (Firma Promega) pro well einer 96-well Mikrotiterplatte zupipettiert. Nach einer 10 minütigen Inkubation im Dunkeln bei Raumtemperatur werden die Mikrotiterplatten im Lumineszenzmeßgerät (Trilux der Firma Wallac) gemessen. Die Messzeit pro well einer Mikrotiterplatte beträgt 1 sec.

### Auswertung

Die Rohdaten des Lumineszenzmeßgerätes werden in ein Microsoft Excel-File transferiert. Dosis-Wirkungskurven und EC50-Werte von PPAR-Agonisten werden mit dem Program XL.Fit nach Vorgabe des Herstellers (Firma IDBS) berechnet.

Die PPARalpha-EC50-Werte für die Verbindungen der Beispiele 1 bis 50 liegen in diesem Assay im Bereich von 1 nM bis >10 µM.

Die Ergebnisse für die Aktivität einiger erfindungsgemäßer Verbindungen der Formel I sind in der folgenden Tabelle I angegeben:

**Tabelle I**

| Beispiel Nr. | EC50 PPARalpha [nM] |
|---|---|
| 3 | 99 |
| 5 | 29 |
| 11 | 15 |
| 21a | 5,0 |
| 25 | 7,2 |
| 43 | 32 |
| 44 | 4,5 |
| 50 | 1,2 |
| | |

Aus der Tabelle I ist ersichtlich, dass die erfindungsgemäßen Verbindungen der Formel I den PPARalpha-Rezeptor aktivieren und damit zum Beispiel analog zu klinisch verwendeten Fibraten im Organismus eine Triglyceridsenkung bewirken (siehe z.B. J.-Ch. Fruchard et al .,: PPARS, Metabolic Disease and Atherosclerosis, Pharmacological Research, Vol. 44, No. 5, 2001; S. Kersten et al.: Roles of PPARs in health and disease, NATURE, VOL 405 ,25 MAY 2000 ;I. Pineda et al.: Peroxisome proliferator-activated receptors: from transcriptional control to clinical practice,Curr Opin Lipidol 12: 2001, 245-254).

### Bestimmung von EC50-Werten von PPAR-Agonisten im zellulären PPARgamma-Test

### Prinzip

Zur Bestimmung der zellulären PPARgamma Aktivität von PPAR-Agonisten wird ein transientes Transfektionssystem eingesetzt. Es basiert auf der Verwendung eines Luziferase-Reporterplasmides (pGL3basic-5xGAL4-TK) und eines PPARgamma-Expressionsplasmides (pcDNA3-GAL4-humanPPARgammaLBD). Beide Plasmide werden vorübergehend (= transient) in humane embryonale Nierenzellen (HEK-Zellen) transfiziert. In diesen Zellen wird dann das Fusionsprotein GAL4-humanPPARgammaLBD exprimiert, das an die GAL4-Bindungsstellen des Reporterplasmides bindet. In Anwesenheit eines PPARgamma-aktiven Liganden wird durch das aktivierte Fusionsprotein GAL4-humanPPARgammaLBD die Expression des Luziferase-Reportergens induziert, was sich nach Zugabe eines Luziferasesubtrates in Form eines Chemilumineszenzsignals nachweisen lässt. Im Unterschied zur stabil transfizierten PPARalpha-Reporterzellinie werden beim zellulären PPAR□-Test die beiden Komponenten (Luziferase-Reporterplasmid und PPARgamma-Expressionsplasmid) transient in HEK-Zellen transfiziert, weil die stabile und permanente Expression des PPARgamma-Fusionsproteins zelltoxisch ist.

### Konstruktion der Plasmide

Das Luziferase-Reporterplasmid pGL3basic-5xGAL4-TK basiert auf dem Vektor pGL3basic der Firma Promega. Zur Herstellung des Reporterplasmides wurden fünf Bindungsstellen des Hefe-Transkriptionsfaktors GAL4 (jede Bindungsstelle mit der Sequenz 5'-CTCGGAGGACAGTACTCCG-3'), 5'-oberhalb zusammen mit einem 160 bp langen Thymidinkinase-Promotorabschnitt (Genbank-Accession # AF027128) in pGL3basic einkloniert. 3'-unterhalb des Thymidinkinasepromotors liegt das gesamte Luziferasegen von Photinus pyralis (Genbank-Accession # M15077), welches bereits Bestandteil des verwendeten Plasmides pGL3basic ist. Die Klonierung und Sequenzierung des Reporterplasmides pGL3basic-5xGAL4-TK erfolgte analog wie bei Sambrook J. et. al. beschrieben (Molecular cloning, Cold Spring Harbor Laboratory Press, 1989).

Zur Herstellung des PPARgamma-Expressionsplasmides pcDNA3-GAL4-humanPPARgammaLBD wurde in das Plasmid pcDNA3 (Firma Invitrogen) 3'-unterhalb des Cytomegalovirus-Promotors zunächst die für die Aminosäuren 1-147 des Hefetranskriptionsfaktors GAL4 (Genbank-Accession # P04386) kodierende cDNA einkloniert. 3'-unterhalb der GAL4-DNA-Bindungsdomäne wurde anschließend die cDNA der Ligandenbindungsdomäne (LBD) des humanen PPARgamma-Rezeptors kloniert (Aminosäuren I152-Y475; Accession # g1480099). Klonierung und Sequenzierung des PPARgamma-Expressionsplasmides pcDNA3-GAL4-humanPPARgammaLBD erfolgten wiederum analog wie bei Sambrook J. et. al. beschrieben (Molecular cloning, Cold Spring Harbor Laboratory Press, 1989).

Neben dem Luziferase-Reporterplasmid pGL3basic-5xGAL4-TK und dem PPARgamma-Expressionsplasmid pcDNA3-GAL4-humanPPARgammaLBD werden für den zellulären PPARgamma-Test noch das Referenzplasmid pRL-CMV (Firma Promega), sowie das Plasmid pBluescript-SK(+) der Firma Stratagene verwendet. Alle vier Plasmide wurden vor der Transfektion in HEK-Zellen mit einem Plasmidpräparationskit der Firma Qiagen präpariert, der eine möglichst endotoxinfreie Plasmidqualität gewährleistet.

### Durchführung des Tests

Die Aktivität von PPARgamma-Agonisten wird in einem 4-Tagestest bestimmt, der nachfolgend beschrieben ist. Vor der Transfektion werden HEK-Zellen in DMEM-Medium (# 41965-039, Invitrogen) kultiviert, das mit folgenden Zusätzen versetzt ist: 10% FKS (#16000-044, Invitrogen), 1 % Penicillin-Streptomycin-Lösung (#15140-122, Invitrogen) und 2 mM L-Glutamin (#25030-024, Invitrogen).

### Tag 1

Zunächst wird Lösung A hergestellt, ein Transfektionsgemisch, das neben DMEM-Medium alle vier bereits beschriebenen Plasmide enthält. Für einen Test werden pro 96-well Mikrotiterplatte folgende Mengen zum Ansetzen von 3 ml Lösung A verwendet: 2622 µl antibiotika- und serumfreies DMEM-Medium (# 41965-039, Invitrogen), 100 µl Referenzplasmid pRL-CMV (1 ng/µl), 100 µl Luziferase-Reporterplasmid pGL3basic-5xGAL4-TK (10 ng/µl), 100 µl PPARgamma-Expressionsplasmid pcDNA3-GAL4-humanPPARgammaLBD (100 ng/µl) und 78 µl Plasmid pBluescript-SK(+) (500 ng/µl). Danach werden pro 96-well Mikrotiterplatte 2 ml Lösung B durch Mischen von 1,9 ml DMEM-Medium (# 41965-039, Invitrogen) mit 100 µl PolyFect-Transfektionsreagens (Firma Qiagen) hergestellt. Anschließend werden 3 ml Lösung A mit 2 ml Lösung B zu 5 ml Lösung C versetzt, durch mehrfaches Pipettieren gründlich gemischt und für 10 min bei Raumtemperatur inkubiert.

80% konfluente HEK-Zellen aus einer 175 cm² großen Zellkulturflasche werden einmal mit 15 ml PBS gewaschen (#14190-094, Invitrogen) und mit 3 ml Trypsinlösung (#25300-054, Invitrogen) für 2 min bei 37°C behandelt. Danach werden die Zellen in 15 ml DMEM-Medium (# 41965-039, Invitrogen) aufgenommen, welches mit 10% FKS (# 16000-044, Invitrogen), 1 % Penicillin-Streptomycin-Lösung (#15140-122, Invitrogen) und 2 mM L-Glutamin (#25030-024, Invitrogen) versetzt ist. Nach dem Zählen der Zellsuspension im Zellzählgerät wird die Suspension auf 250.000 Zellen/ml verdünnt. Für 1 Mikrotiterplatte werden 15 ml dieser Zellsuspension mit 5 ml der Lösung C vermengt. Pro well einer 96 well-Mikrotiterplatte mit klarem Plastikboden (#3610, Corning Costar) werden 200 µl der Suspension ausgesät. Die Platten werden für 24 h in einem Zellkulturbrutschrank bei 37°C und 5 % CO₂ inkubiert.

### Tag 2

Zu testende PPAR-Agonisten werden in einer Konzentration von 10 mM in DMSO gelöst. Diese Stocklösung wird in DMEM-Medium (# 41965-039, Invitrogen) verdünnt, welches mit 2 % Ultroser (#12039-012, Biosepra), 1 % Penicillin-Streptomycin-Lösung (#15140-122, Invitrogen) und 2 mM L-Glutamin (#25030-024, Invitrogen) versetzt ist. Testsubstanzen werden in insgesamt 11 verschiedenen Konzentrationen im Bereich von 10 µM bis 100 pM getestet. Potentere Verbindungen werden in Konzentrationsbereichen von 1 µM bis 10 pM geprüft.

Das Medium der an Tag 1 transfizierten und ausgesäten HEK-Zellen wird vollständig abgesaugt und die in Medium verdünnten Testsubstanzen sofort zu den Zellen zugegeben. Die Verdünnung und Zugabe der Substanzen erfolgt mit einem Roboter (Beckman FX). Das Endvolumen der in Medium verdünnten Testsubstanzen beträgt 100 µl pro well einer 96 well-Mikrotiterplatte. Jede Platte wird mit einem Standard PPARgamma-Agonisten belegt, der ebenfalls in 11 verschiedenen Konzentrationen verdünnt wird, um die Funktionsfähigkeit des Tests in jeder Einzelplatte nachzuweisen. Die Testplatten werden für 48 h in einem Brutschrank bei 37 °C und 5 % CO₂ inkubiert.

### Tag 4

Nach dem Absaugen des Mediums werden gemäß den Angaben des Herstellers pro well je 50 µl Dual-Glo^{™} Reagens (Dual-Glo^{™} Luciferase Assay System; Firma Promega) zugegeben, um die Zellen zu lysieren und das Substrat für die in den Zellen gebildete Firefly-Luziferase (Photinus pyralis) zur Verfügung zu stellen. Nach einer 10 minütigen Inkubation im Dunkeln bei Raumtemperatur wird die Firefly-Luziferasevermittelte Chemilumineszenz im Messgerät gemessen (1 sec. Meßzeit/well; Trilux der Firma Wallac). Danach wird pro well je 50 µl des Dual-Glo^{™} Stop & Glo Reagens (Dual-Glo^{™} Luciferase Assay System; Firma Promega) zugegeben, um die Aktivität der Firefly-Luziferase abzustoppen und das Substrat für die vom Referenzplasmid pRL-CMV aus exprimierten Renilla-Luciferase zur Verfügung zu stellen. Nach einer weiteren 10 minütigen Inkubation im Dunkeln bei Raumtemperatur wird erneut für 1 sec/well die durch die Renilla-Luziferase vermittelte Chemilumineszenz im Messgerät gemessen.

### Auswertung

Die Rohdaten des Lumineszenzmeßgerätes werden in ein Microsoft Excel-File transferiert. Für jeden Meßpunkt, der sich von einem well der Mikrotiterplatte ableitet, wird der Quotient Firefly/Renilla-Luciferaseaktivität bestimmt. Aus den Quotienten werden die Dosis-Wirkungskurven und EC50-Werte von PPAR-Agonisten mit dem Program XL.Fit nach Vorgabe des Herstellers (Firma IDBS) berechnet.

Mit den in dieser Anmeldung beschriebenen PPAR-Agonisten wurden PPARgamma-EC50-Werte im Bereich von 1 nM bis >10 µM gemessen.

Die nachfolgend aufgeführten Beispiele dienen zur Erläuterung der Erfindung, ohne diese jedoch einzuschränken.

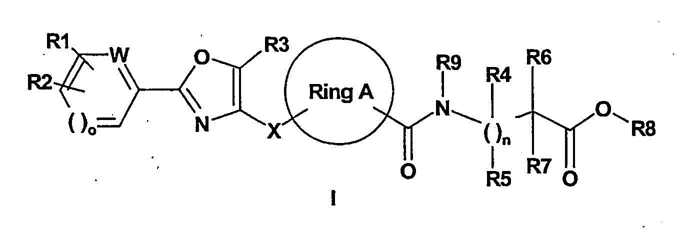

**Tabelle II:**

| Im folgenden sind: | | | | | | | | | |
|---|---|---|---|---|---|---|---|---|---|
| Ring A = cis-Cyclohexan-1,3-diyl, mit der Stereochemie nach Cahn-Ingold-Prelog, wie sie in den Beispielen angegeben ist; R4 = R5 = H, und R8 = H. | | | | | | | | | |
| **Bsp** | **R1** | **R2** | **R3** | **W** | **X** | **n** | **R6** | **R7** | **R9** |
| 1 | p-CH3 | H | CH3 | CH | -CH2O- | 0 | H | (S)-i-C3H7 | H |
| 2 | p-CH3 | H | CH3 | CH | -CH2O- | 0 | H | (S)-i-C4H9 | H |
| 3 | p-CH3 | H | CH3 | CH | -CH2O- | 0 | H | (S)-CH3 | H |
| 4 | p-CH3 | H | CH3 | CH | -CH2O- | 0 | H | (S)-PhCH2 | H |
| 5 | p-CH3 | H | CH3 | CH | -CH2O- | 0 | CH3 | CH3 | H |
| 6 | p-CH3 | H | CH3 | CH | -CH2O- | 0 | H | (R)-i-C3H7 | H |
| 7 | m- | H | CH3 | CH | -CH2O- | 0 | H | (2S)-Pyrrolidin-2-yl | |
| 8 | p-CH3 | H | CH3 | CH | -CH2O- | 0 | H | (2S)-Pyrrolidin-2-yl | |
| 9 | m- | H | CH3 | CH | -CH2O- | 0 | H | (S)-i-C3H7 | H |
| | | | | | | | | | |
| 10 | m-Br | H | CH3 | CH | -CH2O- | 0 | H | (S)-i-C3H7 | H |
| 11 | m-CF3 | H | CH3 | CH | -CH2O- | 0 | H | (S)-i-C3H7 | H |
| 12 | p-CH3 | H | i-C3H7 | CH | -CH2O- | 0 | H | (S)-i-C3H7 | H |
| 13 | m-OCH3 | H | i-C3H7 | CH | -CH2O- | 0 | H | (S)-i-C3H7 | H |
| 14 | m-OCH3 | H | i-C3H7 | CH | -CH2O- | 0 | H | (S)-i-C3H7 | H |
| 15 | m-OCH3 | H | CF3 | CH | -CH2O- | 0 | H | (S)-i-C3H7 | H |
| 16 | m-CF3 | H | CF3 | CH | -CH2O- | 0 | H | (S)-i-C3H7 | H |
| 17 | p-CH3 | H | CF3 | CH | -CH2O- | 0 | H | (S)-i-C3H7 | H |
| 18 | p-CH3 | H | Ph | CH | -CH2O- | 0 | H | (S)-i-C3H7 | H |
| 19 | m-OCH3 | H | Ph | CH | -CH2O- | 0 | H | (S)-i-C3H7 | H |
| 20 | m-OCH3 | H | Et | CH | -CH2O- | 0 | H | (S)-i-C3H7 | H |
| 21 | p-CH3 | H | Et | CH | -CH2O- | 0 | H | (S)-i-C3H7 | H |
| 22 | m-OCH3 | H | Cy | CH | -CH2O- | 0 | H | (S)-i-C3H7 | H |
| 23 | p-CH3 | H | Cy | CH | -CH2O- | 0 | H | (S)-i-C3H7 | H |
| 24 | p-CH3 | H | CH3 | CH | -CH2O- | 0 | Cyclohexyl | | H |
| 25 | p-CH3 | H | CH3 | CH | -CH2O- | 0 | Cyclopentyl | | H |
| 26 | m-OCH3 | H | CH3 | CH | -CH2O- | 0 | Cyclopentyl | | H |
| 27 | m-CF3 | H | CH3 | CH | -CH2O- | 0 | Cyclopentyl | | H |
| 28 | p-CH3 | H | CH3 | CH | -CH2O- | 0 | H | (S)-i-C3H7 | CH3 |
| 29 | p-CH3 | H | CH3 | CH | -CH2O- | 0 | H | (S)-i-C3H7 | PhCH2 |
| 30 | p-CH3 | H | CH3 | CH | -CH2O- | 0 | H | (S)-i-C3H7 | n-C3H7 |
| 31 | p-CH3 | H | C2H5 | CH | -CH2O- | 0 | Cyclopentyl | | H |
| 32 | m-OCH3 | m'-OCH3 | CH3 | CH | -CH2O- | 0 | Cyclopentyl | | H |
| 33 | m-CF3 | H | i-C3H7 | CH | -CH2O- | 0 | Cyclopentyl | | H |
| 34 | m-CF3 | H | C2H5 | CH | -CH2O- | 0 | Cyclopentyl | | H |
| 35 | m-CH3 | p-CH3 | i-C3H7 | CH | -CH2O- | 0 | Cyclopentyl | | H |
| 36 | p-OCH3 | H | CH3 | CH | -CH2O- | 0 | Cyclopentyl | | H |
| 37 | 2-Naphthyl | | C2H5 | CH | -CH2O- | 0 | Cyclopentyl | | H |
| 38 | m-OCH3 | H | C2H5 | CH | -CH2O- | 0 | Cyclopentyl | | H |
| 39 | p-i-C3H7 | H | CH3 | CH | -CH2O- | 0 | Cyclopentyl | | H |
| 40 | p-i-C3H7 | H | C2H5 | CH | -CH2O- | 0 | Cyclopentyl | | H |
| 41 | p-CH3 | H | CH3 | CH | -CH2OCH2- | 0 | H | (S)-i-C3H7 | H |
| 42 | m-CH3 | p-CH3 | C2H5 | CH | -CH2OCH2- | 0 | H | (S)-i-C3H7 | H |
| 43 | p-CF3 | H | i-C3H7 | CH | -CH2OCH2- | 0 | H | (S)-i-C3H7 | H |
| 44 | m-CF3 | H | CH3 | CH | -CH2OCH2- | 0 | H | (S)-i-C3H7 | H |
| 45 | p-i-C3H7 | H | C2H5 | CH | -CH2OCH2- | 0 | H | (S)-i-C3H7 | H |
| 46 | p-CH3 | H | C2H5 | CH | -CH2OCH2- | 0 | H | (S)-i-C3H7 | H |
| 47 | m-OCH3 | H | Cy | CH | -CH2OCH2- | 0 | H | (S)-i-C3H7 | H |
| 48 | m-OCH3 | H | CH3 | CH | -CH2OCH2- | 0 | H | (S)-i-C3H7 | H |
| 49 | p-i-C3H7 | H | CH3 | CH | -CH2OCH2- | 0 | H | (S)-i-C3H7 | H |
| 50 | p-CF3 | H | C2H5 | CH | -CH2OCH2- | 0 | H | (S)-i-C3H7 | H |

### Verfahren

Die erfindungsgemäßen Verbindungen der Formel I können entsprechend den folgenden Reaktionsschemata erhalten werden:

### Verfahren A:

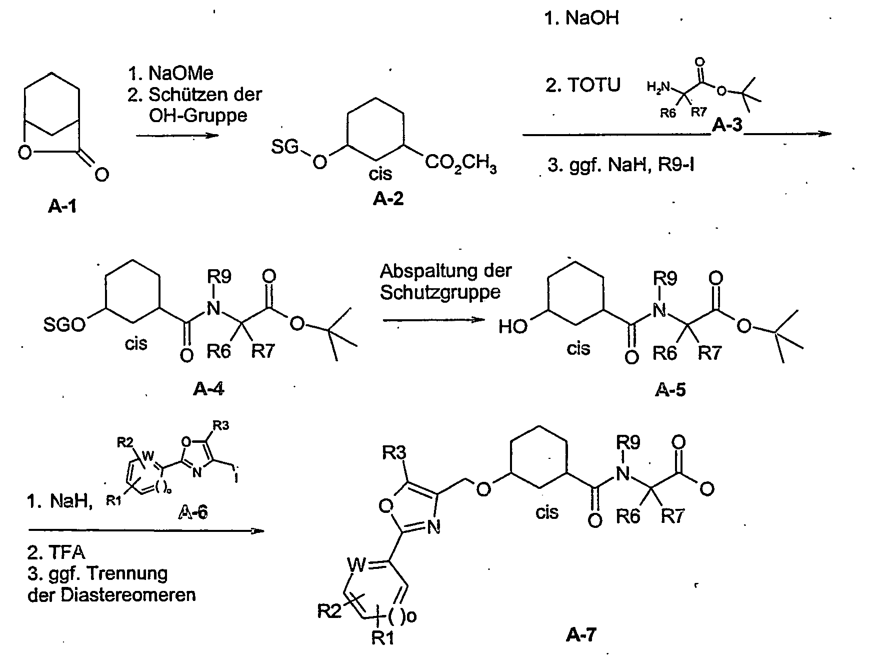

Die Verbindung A-1 wird bei Raumtemperatur in Methanol mit Natriummethanolat gerührt. Nach Aufarbeitung wird das Produkt an der Hydroxylgruppe geschützt (SG = Schutzgruppe), beispielsweise durch Umsetzen mit tert-Butyldiphenylsilylchlorid und Imidazol in Dimethylformamid bei Raumtemperatur oder mit Methoxymethylchlorid, Ethyldiisopropylamin in Dichlormethan. Dabei wird die Verbindung A-2 erhalten.

Die Verbindung A-2 wird in Isopropanol mit Natriumhydroxid 1 Stunde bei 60 °C gerührt und aufgearbeitet. Die so erhaltenen Carbonsäure wird in Dimethylformamid mit dem tert-Butylester einer α-Aminosäure der allgemeinen Formel A-3, worin R6 und R7 die oben beschriebenen Bedeutungen haben, Hydroxybenzotriazol, Diisopropylethylamin und O-[Cyan(ethoxycarbonyl)methylenamino]-1,1,3,3,-tetramethyluronium-tetrafluoroborat (TOTU) zum Produkt der allgemeinen Formel A-4, worin R9 = H ist, umgesetzt. In einigen Beispielen wird das Kupplungsprodukt mit Natriumhydrid und einem Alkyliodid der allgemeinen Formel R9-I, wobei R9 die oben beschriebene Bedeutung hat - außer R9 = H - , zur Verbindung der allgemeinen Formel A-4 umgesetzt.

Die Verbindung A-4 wird nun zur Verbindung A-5 O-entschützt, beispielsweise mit Tetrabutylammoniumfluorid in Tetrahydrofuran im Falle der tert-Butyldiphenylsilylschutzgruppe oder mit konzentrierter Salzsäure in Tetrahydrofuran im Falle der Methoxymethylschutzgruppe.

Die Verbindung der allgemeinen Formel A-5 wird mit Natriumhydrid und der Verbindung der allgemeinen Formel A-6, worin R1, R2, R3 und W die oben beschriebenen Bedeutungen haben, in Dimethylformamid umgesetzt. Das Produkt wird mehrere Stunden in Trifluoressigsäure gerührt, und anschließend werden, falls notwendig die Diastereomeren per präparativer HPLC getrennt. Dabei wird die Verbindung der allgemeinen Formel A-7 erhalten.

Nach diesem Verfahren können die Beispiele 1 bis 40 synthetisiert werden.

### Verfahren B:

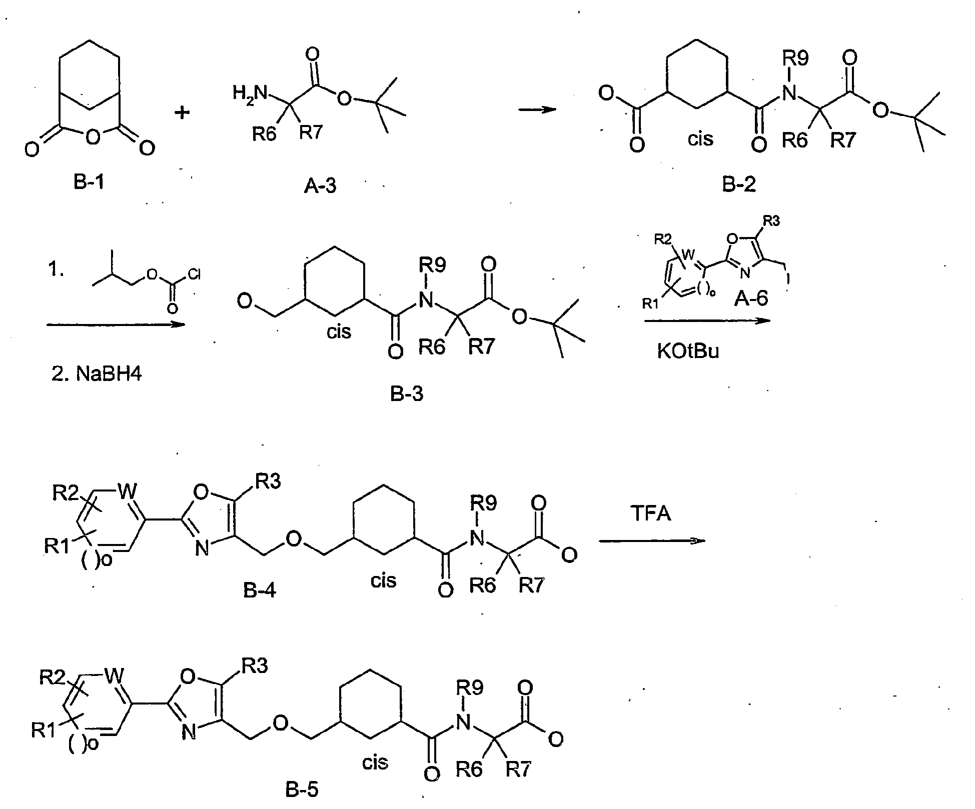

Die Verbindungen B-1 und eine Aminosäure der allgemeinen Formel A-3, worin R6, R7 und R9 die oben beschriebene Bedeutung haben, werden bei 0°C in Dimethylformamid vorgelegt und mit Triethylamin und katalytischen Mengen Dimethylaminopyridin versetzt. Es wird bei Raumtemperatur nachgerührt. Nach Aufarbeitung wird das Produkt der allgemeinen Formel B-2, beispielsweise durch Umsetzen mit Isobutylchloroformat und Triethylamin in Tetrahydrofuran, gefolgt von einer Zugabe von Natriumborhydrid, zur Verbindung der allgemeinen Formel B-3, worin R6, R7 und R9 die oben beschriebene Bedeutung haben, reduziert. Die Verbindung B-3 wird mit Kaliumtertbutylat und einem Alkyliodid der allgemeinen Formel A-6, worin R1, R2 und R3 die oben beschriebene Bedeutung haben, zur Verbindung der allgemeinen Formel B-4, worin R1, R2, R3, R6, R7 und R9 die oben beschriebene Bedeutung haben, umgesetzt. Die Verbindung B-4 wird nun zur Verbindung der allgemeinen Formel B-5, worin R1, R2, R3, R6, R7 und R9 die oben beschriebene Bedeutung haben, entschützt, beispielsweise durch Umsetztung mit Trifluoressigsäure in Dichlormethan.

Nach diesem Verfahren können die Beispiele 41 bis 50 synthetisiert werden.

### Verfahren C:

Dieses Verfahren dient zur Synthese des Bausteins A, worin R1, R2, W und R3 die oben genannten Bedeutungen haben.

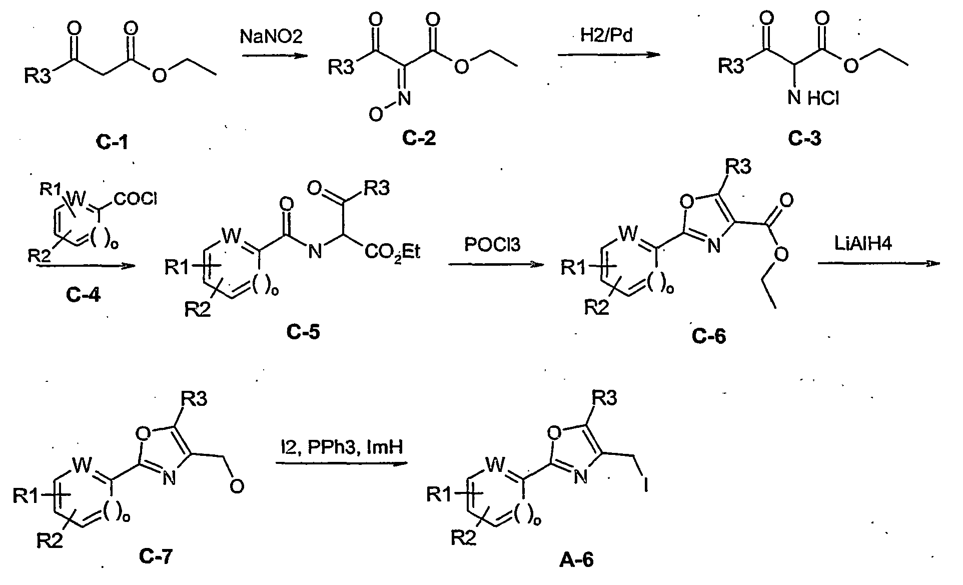

Der Ester C-1, worin R3 die oben genannte Bedeutung hat, wird mit Natriumnitrit und Salzsäure zum Oxim C-2 umgesetzt, welches durch Hydrierung mit Wasserstoff an Palladium/Kohle zum Amin C-3 reduziert wird.

Die Verbindung C-3 wird mit Säurechloriden der allgemeinen Formel C-4, worin R1, W und R2 die oben genannten Bedeutungen haben, und Base (beispielsweise Triethylamin) zur Verbindung C-5 umgesetzt.

Die Verbindung C-5 wird durch Erhitzen in Phosphorylchlorid zur Verbindung C-6, worin R1, R2, W und R3 die oben genannten Bedeutungen haben, umgesetzt.

Der Ester C-6 wird mit Lithiumaluminiumhydrid in einem etherischen Lösungsmittel (z. B) Diethylether zum Alkohol C-7 reduziert. Dieser wird (beispielsweise mit lod, Imidazol (ImH) und Triphenylphosphin) in das lodid A-6 überführt.

### Verfahren D:

Dieses Verfahren dient zur Synthese des Bausteins A-6, worin R1, R2, W und R3 die oben genannten Bedeutungen haben.

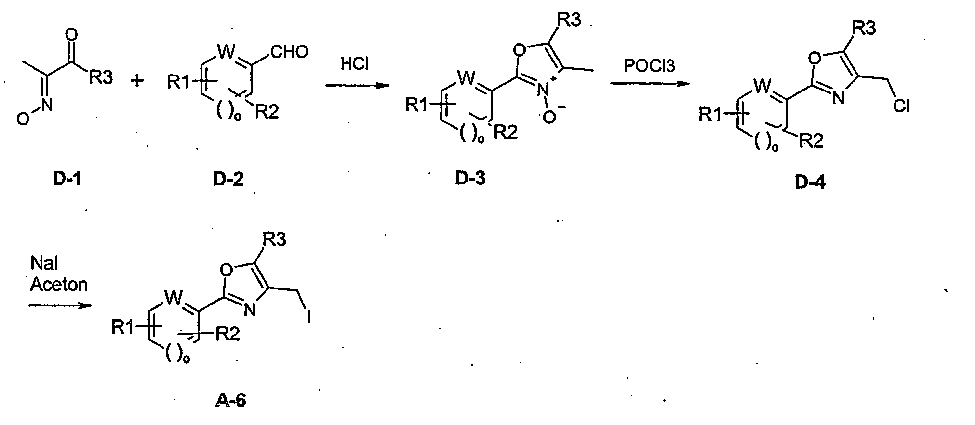

Die Verbindung D-1, worin R3 die oben genannte Bedeutung hat, wird mit dem Aldehyd D-2, worin R1, R2 und W die oben beschriebenen Bedeutungen haben, in Ethanol mit Chlorwasserstoff zur Verbindung D-3 umgesetzt.

Die Verbindung D-3 wird in Phosphorylchlorid zum Sieden erhitzt, wobei die Verbindung D-4 erhalten wird. Diese wird mit Natriumiodid in Aceton zum Sieden erhitzt. Dabei erhält man die Verbindung A-6.

Andere Verbindungen können entsprechend den oben genannten Verfahren hergestellt werden.

### Bausteinsynthese nach Verfahren C:

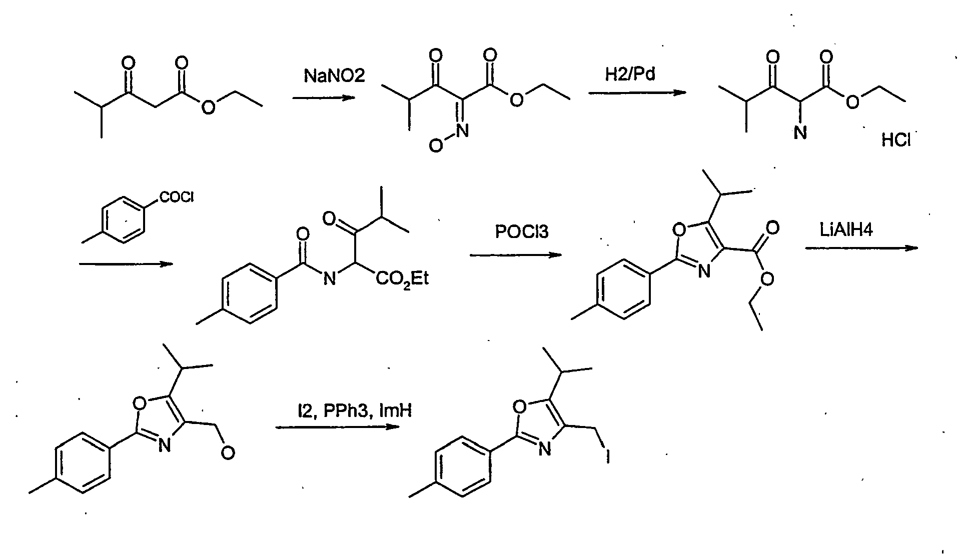

### 2-Hydroxyimino-4-methyl-3-oxo-pentansäureethylester

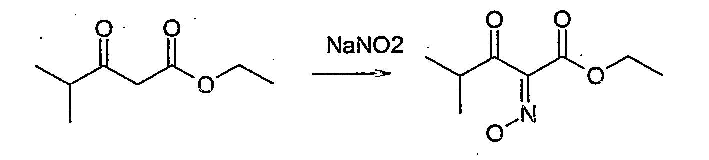

42.4 g 4-Methyl-3-oxo-pentansäureethylester werden in 100 ml Eisessig gelöst und bei 5°C mit 21 g Natriumnitrit, gelöst in 100 ml Wasser, versetzt. Man lässt innerhalb einer Stunde auf Raumtemperatur erwärmen, fügt sodann 100 ml Wasser hinzu und rührt eine weiter Stunde bei Raumtemperatur nach. Man extrahiert dreimal mit je 150 ml Methyl-tert-butylether, die vereinigten organischen Phasen werden mit 200 ml Wasser versetzt und durch Zugabe von festem NaHCO3 neutralisiert. Die organische Phase wird abgetrennt, mit gesättigter NaCl-Lösung gewaschen, über MgSO4 getrocknet und anschließend das Lösungsmittel im Vakuum entfernt. Man erhält 46 g 2-Hydroxyimino-4-methyl-3-oxo-pentansäure-ethylester als Öl. C8H13NO4 (187.20), MS(ESI) = 188 (M+H⁺).

### 2-Amino-4-methyl-3-oxo-pentansäureethylesterhydrochlorid

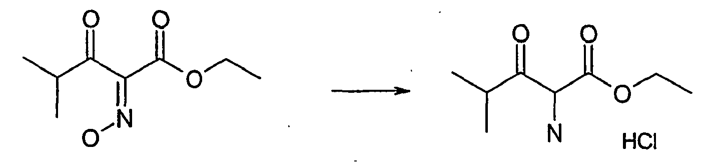

In 200 ml Ethanol werden 10 g HCl eingeleitet. 46 g 2-Hydroxyimino-4-methyl-3-oxo-pentansäureethylester werden darin gelöst und mit 5 g Pd(10% auf Kohle) versetzt und 8 Stunden unter einer Wasserstoffatmosphäre (5 bar) gerührt. Das Reaktionsgemisch wird über Celite filtriert und das Lösungsmittel im Vakuum entfernt. Man erhält 45 g 2-Amino-4-methyl-3-oxo-pentansäureethylester hydrochlorid als weißen Feststoff. C8H15NO3*HCl (209.5), MS(ESI) = 188 (M+H⁺).

### 4-Methyl-2-(4-methyl-benzoylamino)-3-oxo-pentansäureethylester

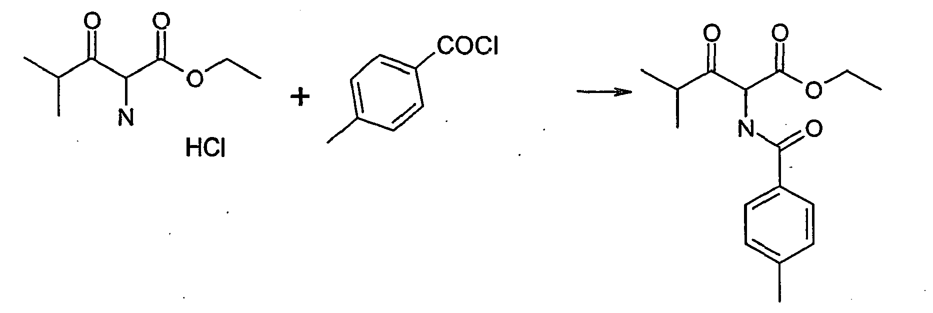

10 g 2-Amino-4-methyl-3-oxo-pentansäureethylesterhydrochlorid und 7.4 g 4-Methyl-benzoylchlorid werden in 250 ml Dichlormethan gelöst und bei 0°C langsam und tropfenweise mit 13.3 ml Triethylamin versetzt. Man rührt eine Stunde bei Raumtemperatur nach, dann wird mit Wasser gewaschen, die organische Phase abgetrennt, über MgSO4 getrocknet und anschließend das Lösungsmittel im Vakuum entfernt. Man erhält 13 g 4-Methyl-2-(4-methyl-benzoylamino)-3-oxo-pentansäureethylester als Öl.
C16H21NO4 (291.35), MS(ESI) = 292 (M+H⁺).

### 5-Isopropyl-2-p-tolyl-oxazol-4-carbonsäureethylester

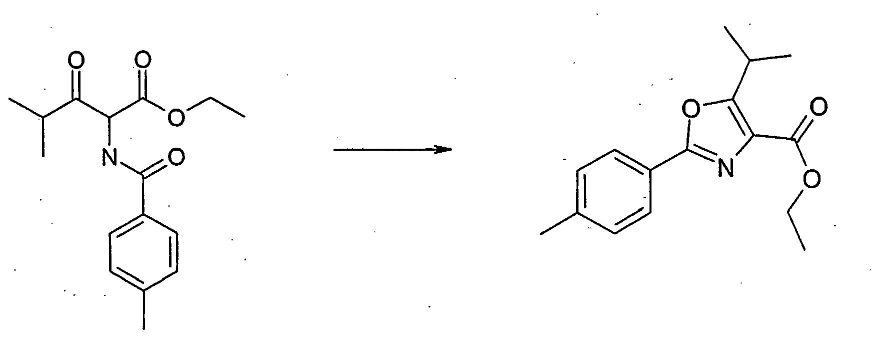

13 g 4-Methyl-2-(4-methyl-benzoylamino)-3-oxo-pentansäureethylester werden in 80 ml Phosphoroxychlorid 2h unter Rückfluss zum Sieden erhitzt. Das Phosphoroxychlorid wird im Vakuum entfernt und der resultierende Rückstand in 200 ml Dichlormethan gelöst , dreimal mit gesättigter NaHCO₃-Lösung gewaschen, über MgSO4 getrocknet und anschließend das Lösungsmittel im Vakuum entfernt. Man erhält 11 g 5-Isopropyl-2-p-tolyl-oxazol-4-carbonsäureethylester als bräunlichen Feststoff.C16H19NO3 (273.33), MS(ESI) = 292 (M+H⁺), Rf(n-Heptan:Ethylacetat) = 2:1) = 0.43.

### (5-Isopropyl-2-p-tolyl-oxazol-4-yl)-methanol

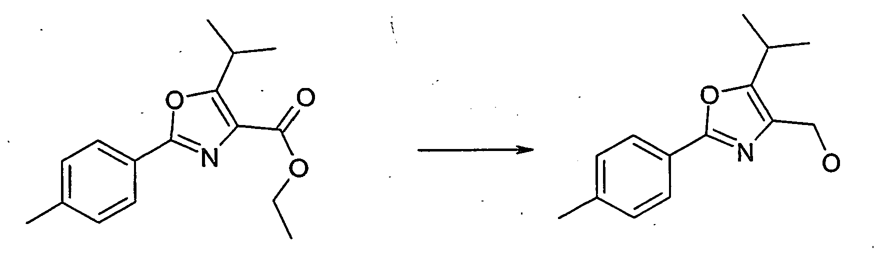

11 g 5-Isopropyl-2-p-tolyl-oxazol-4-carbonsäureethylester werden in 100 ml Tetrahydrofuran gelöst und bei 0°C mit 40 ml einer 1 molaren Lösung von Lithiumaluminiumhydrid in Tetrahydrofuran versetzt. Nach 30 min wird das Reaktionsgemisch mit 50 ml 1N HCl versetzt und fünfmal mit Ethylacetat extrahiert. Die vereinigten organischen Phasen werden über MgSO4 getrocknet und anschließend das Lösungsmittel im Vakuum entfernt. Der Rückstand wird an Kieselgel mit dem Laufmittel n-Heptan:Ethylacetat = 6:1 => 1:1 gereinigt. Man erhält 4.3 g (5-Isopropyl-2-p-tolyl-oxazol-4-yl)-methanol als hellgelben Feststoff. C14H17N02 (231.30), MS(ESI) = 232 (M+H⁺), Rf(n-Heptan:Ethylacetat) = 1:1) = 0.17.

### 4-lodmethyl-5-isopropyl-2-p-tolyloxazol

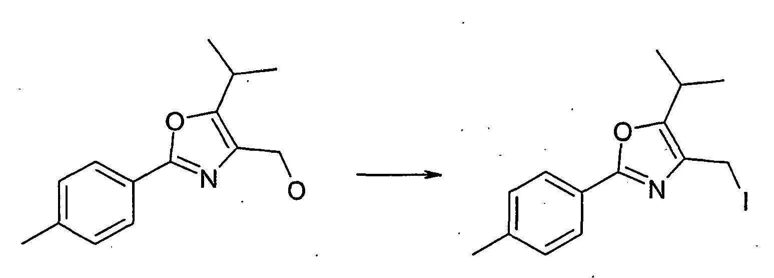

500 mg (5-Isopropyl-2-p-tolyl-oxazol-4-yl)-methanol werden zusammen mit 690 mg Triphenylphosphin und 600 mg Imidazol in 20 ml Toluol gelöst. Man gibt 715 mg lod hinzu und rührt 1 Stunde bei Raumtemperatur nach. Dann wird 10 ml gesättigte Natriumcarbonat-Lösung und 500 mg lod nachgegeben. Nach 10 Minuten wird die organische Phase abgetrennt und zweimal mit gesättigter Na2S2O3-Lösung gewaschen, über MgSO4 getrocknet und anschließend die lösungsmittel im Vakuum entfernt. Der Rückstand wird an Kieselgel mit dem Laufmittel n-Heptan:Ethylacetat = 10:1 gereinigt. Man erhält 400 mg 4-Iodmethyl-5-isopropyl-2-p-tolyl-oxazol als weißen Feststoff. C14H16INO (341.19), MS(ESI): 342 (M+H⁺), Rf(n-Heptan:Ethylacetat = 1:1) = 0.75.

Analog zur Bausteinsynthese nach Verfahren C wurde aus 2-Amino-4-methyl-3-oxo-pentansäureethylester hydrochlorid und 3-Methoxy-benzoylchlorid 4-lodmethyl-2-(3-methoxy-phenyl)-5-isopropyl-oxazol erhalten. 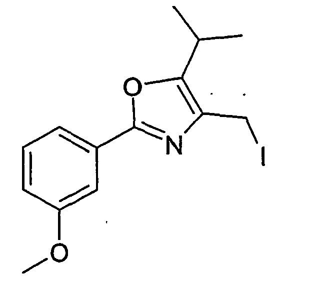 C14H16INO2 (357.19), MS(ESI): 358 (M+H⁺), Rf(n-Heptan:Ethylacetat = 1:1) = 0.60.

Analog zur Bausteinsynthese von 4-Iodmethyl-5-isopropyl-2-p-tolyl-oxazol wurde aus 4,4,4-Trifluoro-3-oxo-buttersäureethylester und 3-Methoxy-benzoylchlorid 4-Iodmethyl-2-(3-methoxy-phenyl)-5-trifluormethyl-oxazol erhalten. 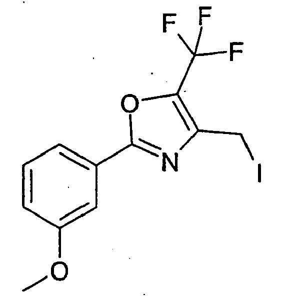 C12H9F3INO2 (383.11), MS(ESI): 384 (M+H⁺).

Analog zur Bausteinsynthese von 4-Iodmethyl-5-isopropyl-2-p-tolyl-oxazol wurde aus 4,4,4-Trifluoro-3-oxo-buttersäureethylester und 3-Trifluormethylbenzoylchlorid 4-Iodmethyl-2-(3-trifluormethyl -phenyl)-5-trifluormethyl-oxazol erhalten. 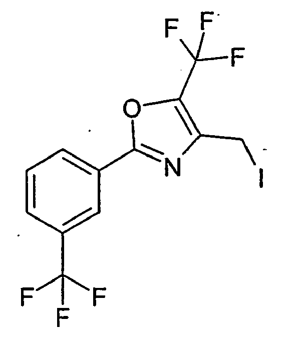 C12H6F6INO (421.08), MS(ESI): 422 (M+H⁺).

Analog zur Bausteinsynthese von 4-Iodmethyl-5-isopropyl-2-p-tolyl-oxazol wurde aus 4,4,4-Trifluoro-3-oxo-buttersäureethylester und 4-Methyl-benzoylchlorid 4-lodmethyl-5-trifluormethyl-2-p-tolyl-oxazol erhalten. 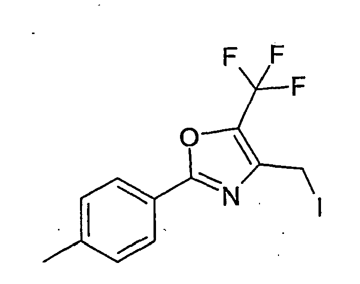 C12H9F3INO (367.11), MS(ESI): 368 (M+H⁺).

### Bausteinsynthese nach Verfahren D:

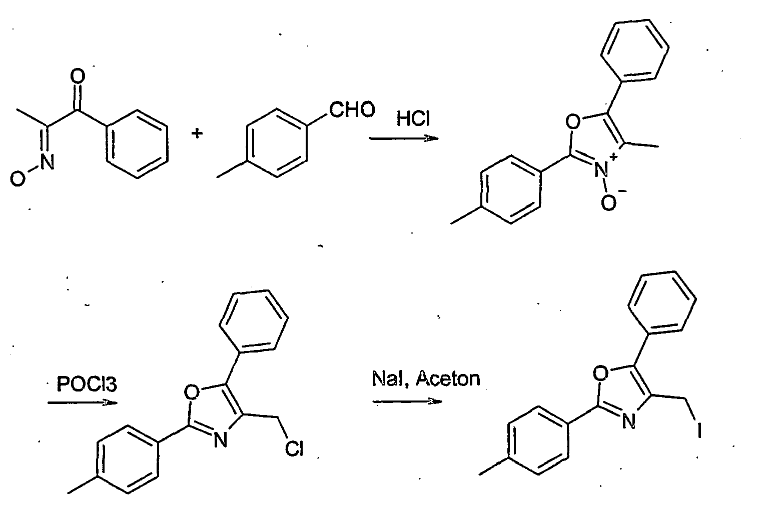

4-Methyl-5-phenyl-2-p-tolyl-oxazol 3-oxid

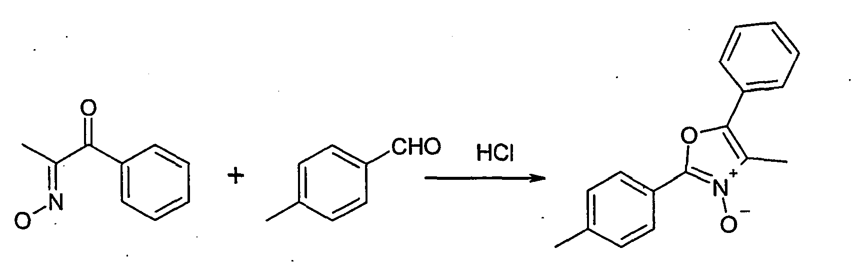

12.5 g 1-Phenyl-1,2-propandion-2-oxim und 10ml p-Toluolaldehyd werden in 50 ml Eisessig gegeben und 30 Minuten unter Eiskühlung HCl Gas durchgeleitet. Durch Zugabe von Methyl-tert-butylether wird das Produkt als Hydrochlorid ausgefällt, abgesaugt und der Niederschlag mit Methyl-tert-butylether gewaschen. Man suspendiert den Niederschlag in Wasser und stellt mit Ammoniak einen basischen pH-Wert ein. Es wird dreimal mit je 200 ml Dichlormethan extrahiert, die vereinigten organischen Phasen werden über MgSO4 getrocknet und anschließend das Lösungsmittel im Vakuum entfernt. Man erhält 6.4 g 4-Methyl-5-phenyl-2-p-tolyl-oxazol 3-oxid als weißen Feststoff. C17H15NO2 (265.31), MS(ESI) = 266 (M+H⁺).

### 4-Chlormethyl-5-phenyl-2-p-tolyl-oxazol

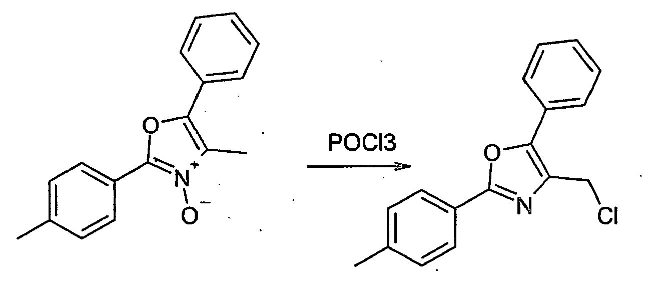

6.4 g 4-Methyl-5-phenyl-2-p-tolyl-oxazol 3-oxid werden in 50 ml Chloroform gelöst, mit 2.4 ml Phosphoroxychlorid versetzt und 30 Minuten unter Rückfluss zum Sieden erhitzt. Das Reaktionsgemisch wird auf 0°C abgekühlt, mit Ammoniak ein schwach alkalischer pH-Wert eingestellt und dreimal mit je 100 ml Ethylacetat extrahiert. Die vereinigten organischen Phasen werden mit Wasser gewaschen, über MgSO4 getrocknet und anschließend das Lösungsmittel im Vakuum entfernt. Man erhält 5.4 g 4-Chlormethyl-5-phenyl-2-p-tolyl-oxazol als gelben Feststoff. C17H14CINO (283.76), MS(ESI) = 284 (M+H⁺), Rf(n-Heptan:Ethylacetat) = 7:1) = 0.41.

### 4-Iodmethyl-5-phenyl-2-p-tolyl-oxazol

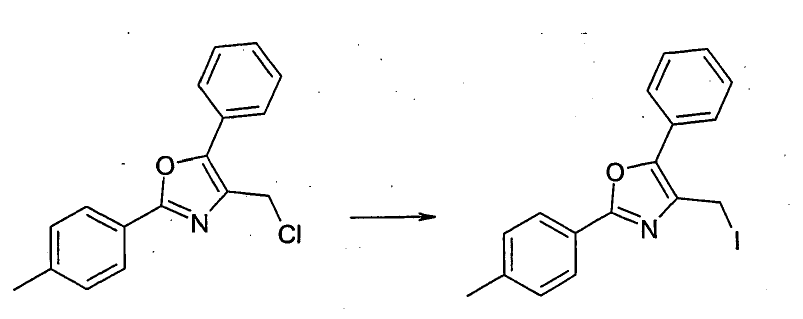

1.8 g 4-Chlormethyl-5-phenyl-2-p-tolyl-oxazol werden zusammen mit 3 g Natriumiodid in 150 ml Aceton 2 Stunden unter Rückfluss zum Sieden erhitzt. Nach dem Abkühlen des Reaktionsgemischs wird 300 ml Methyl-tert-butylether zugefügt, das Gemisch dreimal mit gesättigter Na2S2O3-Lösung gewaschen, über MgSO4 getrocknet und anschließend die Lösungsmittel im Vakuum entfernt. Man erhält 2.7 g 4-lodmethyl-5-phenyl-2-p-tolyl-oxazol als hellgelben Feststoff. C17H14INO (375.21), MS(ESI): 376 (M+H⁺).

Analog zur Bausteinsynthese von 4-Iodmethyl-5-phenyl-2-p-tolyl-oxazol wurde aus 1-Phenyl-1,2-propandion-2-oxim und m-Anisaldehyd 4-Iodmethyl-2-(3-methoxy-phenyl)-5-phenyl-oxazol erhalten. 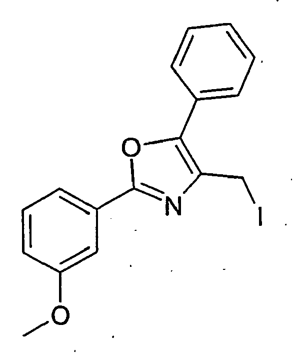 C17H14INO2 (391.21), MS(ESI): 392 (M+H⁺).

Analog zur Bausteinsynthese von 4-lodmethyl-5-phenyl-2-p-tolyl-oxazol wurde aus 1-Ethyl-1,2-propandion-2-oxim und m-Anisaldehyd 4-lodmethyl-5-ethyl-2-(3-methoxyphenyl)-oxazol erhalten. 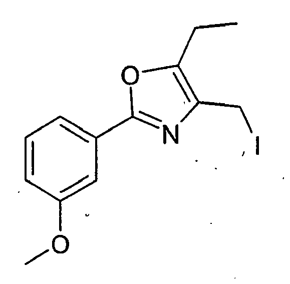 C13H14INO2 (343.17), MS(ESI): 344 (M+H⁺).

Analog zur Bausteinsynthese von 4-Iodmethyl-5-phenyl-2-p-tolyl-oxazol wurde aus 1-Ethyl-1,2-propandion-2-oxim und p-Toluolaldehyd 4-Iodmethyl-5-ethyl-2-p-tolyl-oxazol erhalten. 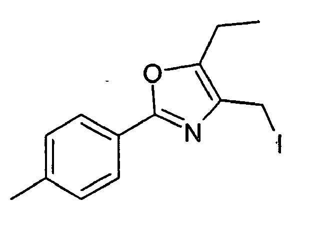 C13H14INO (327.17), MS(ESI): 328 (M+H⁺).

Analog zur Bausteinsynthese von 4-Iodmethyl-5-phenyl-2-p-tolyl-oxazol wurde aus 1-Cyclohexyl-1,2-propandion-2-oxim und m-Anisaldehyd 4-Iodmethyl-5-cyclohexyl-2-(3-methoxy-phenyl)-oxazol erhalten. 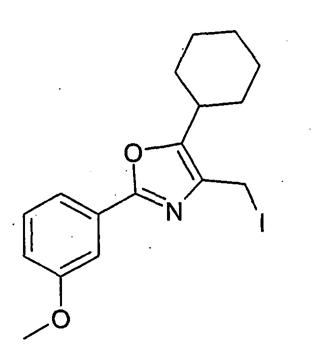 C17H20INO2 (397.26), MS(ESI): 398 (M+H⁺).

Analog zur Bausteinsynthese von 4-lodmethyl-5-phenyl-2-p-tolyl-oxazol wurde aus 1-Cyclohexyl-1,2-propandion-2-oxim und p-Toluolaldehyd 4-Iodmethyl-5-cyclohexyl-2-p-tolyl-oxazol erhalten. 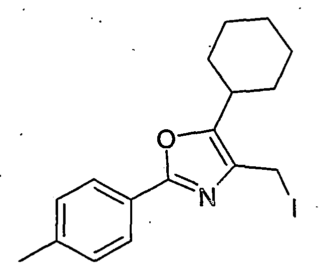 C17H20INO (381.26), MS(ESI): 382 (M+H⁺).

Analog zur Bausteinsynthese von 4-Iodmethyl-5-phenyl-2-p-tolyl-oxazol wurde aus Diacetylmonoxim und p-Toluolaldehyd 4-lodmethyl-5-methyl-2-p-tolyl-oxazol erhalten. 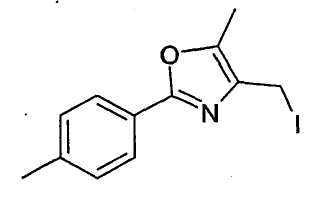 C12H12INO (313.14), MS(ESI): 314 (M+H⁺).

Analog zur Bausteinsynthese von 4-lodmethyl-5-phenyl-2-p-tolyl-oxazol wurde aus Diacetylmonoxim und m-Anisaldehyd 4-lodmethyl-2-(3-methoxy-phenyl)-5-methyl-oxazol erhalten. 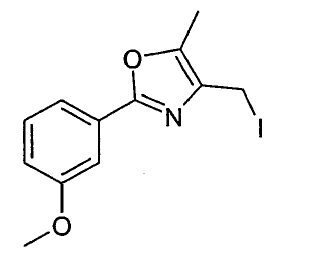 C12H12INO2 (329.14), MS(ESI): 330 (M+H⁺).

Analog zur Bausteinsynthese von 4-Iodmethyl-5-phenyl-2-p-tolyl-oxazol wurde aus Diacetylmonoxim und 3-Bromo-benzaldehyd 2-(3-Bromo-phenyl)-4-iodmethyl-5-methyl-oxazol erhalten. 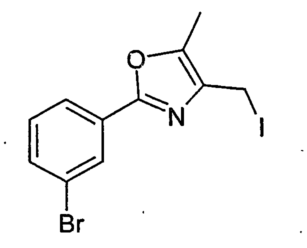 C11H9BrINO (377.01/379.01), MS(ESI): 378/380 (M+H⁺).

Analog zur Bausteinsynthese von 4-Iodmethyl-5-phenyl-2-p-tolyl-oxazol wurde aus Diacetylmonoxim und 3-Trifluormethylbenzaldehyd 4-lodmethyl-5-methyl-2-(3-trifluoromethyl-phenyl)-oxazol erhalten. 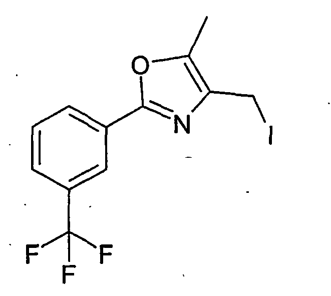 C12H9F3INO (367.11), MS(ESI): 368 (M+H⁺).

Analog zur Bausteinsynthese von 4-Iodmethyl-5-phenyl-2-p-tolyl-oxazol wurde aus Diacetylmonoxim und 4-Fluorbenzaldehyd 2-(4-Fluoro-phenyl)-4-iodmethyl-5-methyl-oxazol erhalten. 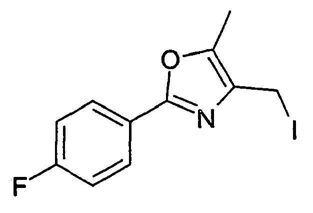 C11HH9FINO (317.10), MS(ESI):318 (M+H⁺).

Analog zur Bausteinsynthese von 4-Iodmethyl-5-phenyl-2-p-tolyl-oxazol wurde aus Diacetylmonoxim und 4-Methoxybenzaldehyd 4-Iodmethyl-2-(4-methoxy-phenyl)-5-methyl-oxazol erhalten. 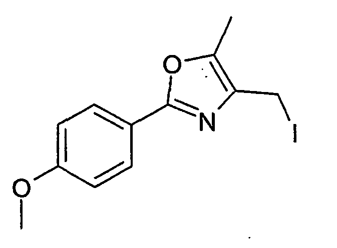 C12H12INO2 (329.14), MS(ESI):330 (M+H+).

Analog zur Bausteinsynthese von 4-Iodmethyl-5-phenyl-2-p-tolyl-oxazol wurde aus Diacetylmonoxim und 3-Trifluormethylbenzaldehyd 4-lodmethyl-5-methyl-2-(3-trifluoromethyl-phenyl)-oxazol erhalten. 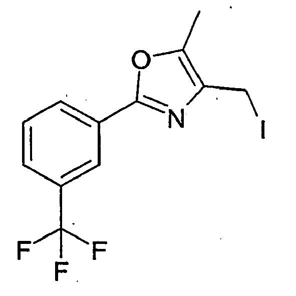 C12H9F3INO (367.11), MS(ESI):368 (M+H+).

Analog zur Bausteinsynthese von 4-Iodmethyl-5-phenyl-2-p-tolyl-oxazol wurde aus Diacetylmonoxim und 4-Trifluormethylbenzaldehyd 4-lodmethyl-5-methyl-2-(4-trifluoromethyl-phenyl)-oxazol erhalten. 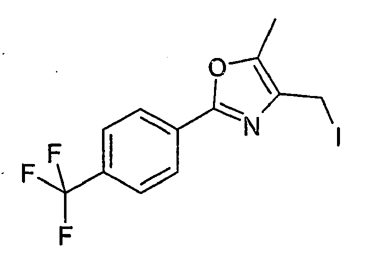 C12H9F3INO (367.11), MS(ESI):368 (M+H+).

Analog zur Bausteinsynthese von 4-lodmethyl-5-phenyl-2-p-tolyl-oxazol wurde aus Diacetylmonoxim und m-Toluolaldehyd 4-Iodmethyl-5-methyl-2-m-tolyl-oxazol erhalten. 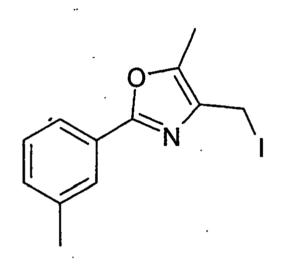 C12H12INO (313.14), MS(ESI):314 (M+H+).

Analog zur Bausteinsynthese von 4-Iodmethyl-5-phenyl-2-p-tolyl-oxazol wurde aus Diacetylmonoxim und 3-Trifluormethoxybenzaldehyd 4-lodmethyl-5-methyl-2-(3-trifluoromethoxy-phenyl)-oxazol erhalten. 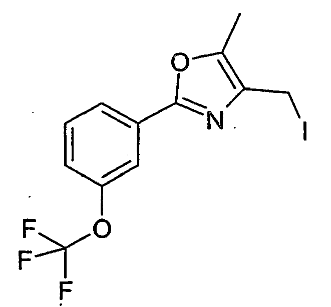 C12H9F3INO2 (383.11), MS(ESI):384 (M+H+).

Analog zur Bausteinsynthese von 4-Iodmethyl-5-phenyl-2-p-tolyl-oxazol wurde aus Diacetylmonoxim und 5-Methylfuran-2-carbaldehyd 4-lodmethyl-5-methyl-2-(5-methyl-furan-2-yl)-oxazol erhalten. 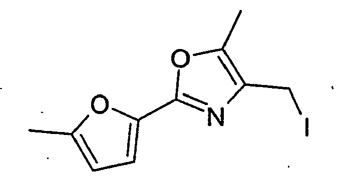 C10H10INO2 (303.11), MS(ESI):304 (M+H+).

Analog zur Bausteinsynthese von 4-lodmethyl-5-phenyl-2-p-tolyl-oxazol wurde aus Diacetylmonoxim und Thiophen-2-carbaldehyd 4-Iodmethyl-5-methyl-2-thiophen-2-yl-oxazol erhalten. 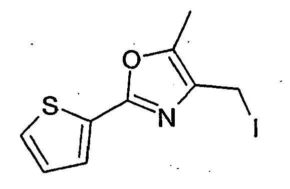 C9H8INOS (305.14), MS(ESI):306 (M+H+).

Analog zur Bausteinsynthese von 4-Iodmethyl-5-phenyl-2-p-tolyl-oxazol wurde aus Diacetylmonoxim und 4-Isopropylbenzaldehyd 4-Iodmethyl-2-(4-isopropyl-phenyl)-5-methyl-oxazol erhalten. 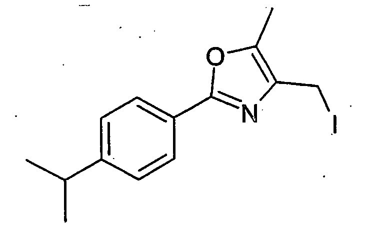 C14H16INO (341.19), MS(ESI):342 (M+H+).

Analog zur Bausteinsynthese von 4-lodmethyl-5-phenyl-2-p-tolyl-oxazol wurde aus 4-Methyl- pentan-2,3-dion-2-oxim und 3- Trifluoromethyl-benzaldehyd 4-lodomethyl-5-isopropyl-2-(3-trifluoromethyl-phenyl)-oxazol erhalten. 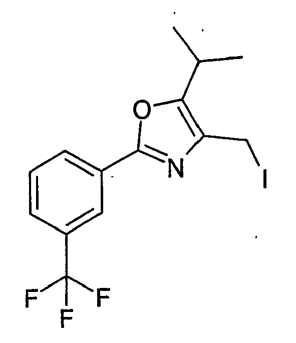 C14H13F3INO (395.17), MS(ESI):396 (M+H+).

Analog zur Bausteinsynthese von 4-Iodmethyl-5-phenyl-2-p-tolyl-oxazol wurde aus Pentan-2,3-dion 2-oxim und 3- Trifluoromethyl-benzaldehyd 5-Ethyl-4-iodomethyl-2-(3-trifluoromethyl-phenyl)-oxazol erhalten. 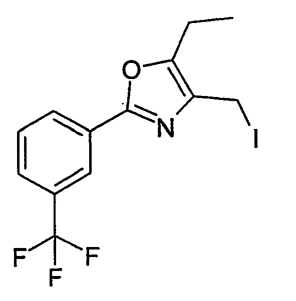 C13H11F3INO (381.14), MS(ESI):382 (M+H+).

Analog zur Bausteinsynthese von 4-Iodmethyl-5-phenyl-2-p-tolyl-oxazol wurde aus 4-Methyl- pentan-2,3-dion-2-oxim und 3,4-Dimethyl-benzaldehyd 2-(3,4-Dimethylphenyl)-4-iodomethyl-5-isopropyl-oxazol erhalten. 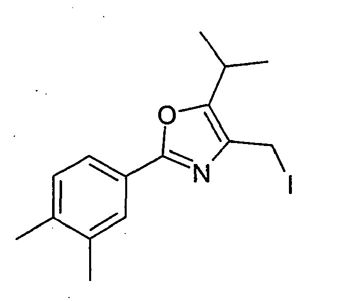 C15H18INO (355.22), MS(ESI):356 (M+H+).

Analog zur Bausteinsynthese von 4-Iodmethyl-5-phenyl-2-p-tolyl-oxazol wurde aus Pentan-2,3-dion 2-oxim und Naphthalen-2-carbaldehyd 5-Ethyl-4-iodomethyl-2-naphthale-2-yl-oxazol erhalten. 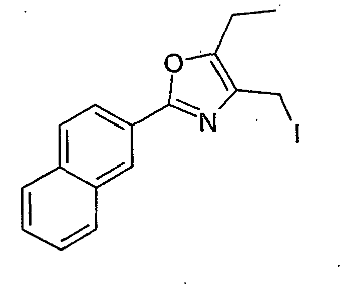 C16H14INO (363.20), NMS(ESI):364 (M+H+).

Analog zur Bausteinsynthese von 4-Iodmethyl-5-phenyl-2-p-tolyl-oxazol wurde aus Pentan-2,3-dion 2-oxim und 4-Isopropylbenzaldehyd 5-Ethyl-4-iodomethyl-2-(4-isopropyl-phenyl)-oxazol erhalten. 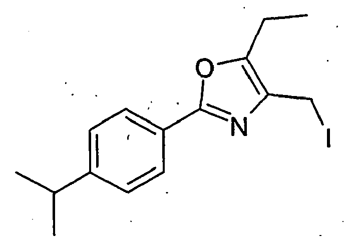 C15H18INO (355.22), MS(ESI):356 (M+H+).

Analog zur Bausteinsynthese von 4-Iodmethyl-5-phenyl-2-p-tolyl-oxazol wurde aus Pentan-2,3-dion 2-oxim und 3,4-Dimethyl-benzaldehyd 2-(3,4-Dimethyl-phenyl)-5-ethyl-4-iodomethyl-oxazol erhalten. 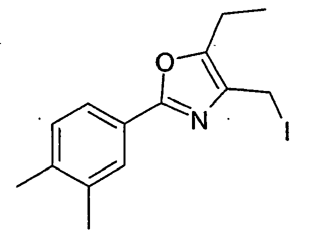 C14H16INO (341.19), MS(ESI):342 (M+H+).

Analog zur Bausteinsynthese von 4-Iodmethyl-5-phenyl-2-p-tolyl-oxazol wurde aus 4-Methyl- pentan-2,3-dion-2-oxim und 4-Trifluoromethyl-benzaldehyd 4-lodomethyl-5-isopropyl-2-(4-trifluoromethyl-phenyl)-oxazol erhalten. 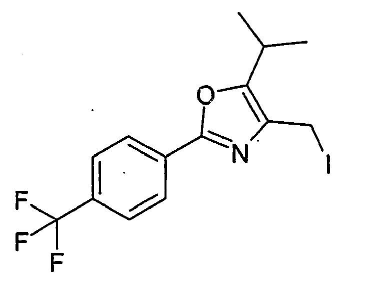 C14H13F3INO (395.17), MS(ESI):396 (M+H+).

### Beispiel 1

### (S)-3-Methyl-2-{[(1R,3S)/(1S,3R)-3-(5-methyl-2-p-tolyl-oxazol-4-ylmethoxy)-cyclohexancarbonyl]-amino}-buttersäure

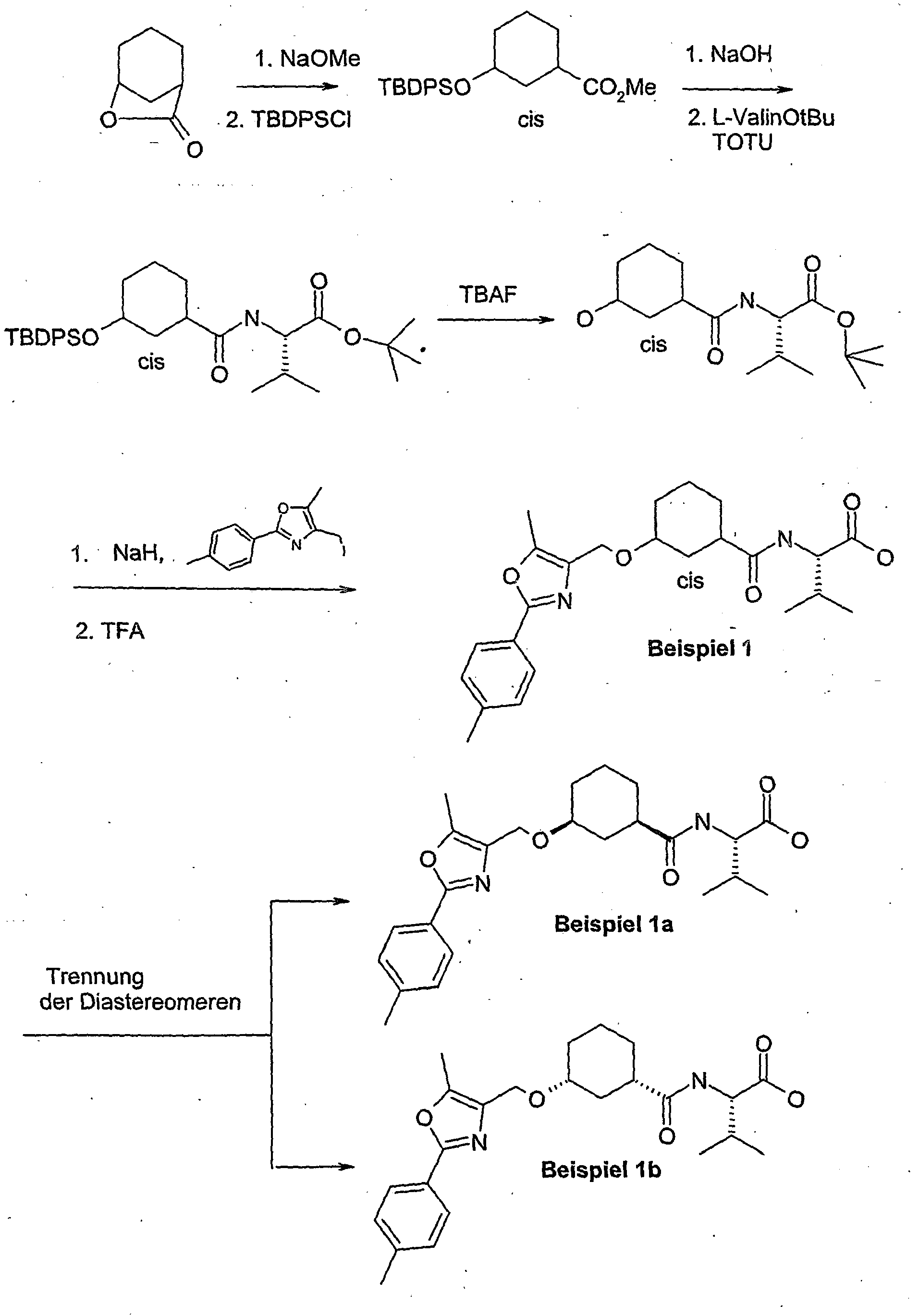

### (1R,3S)/(1S,3R)-3-(tert-Butyl-diphenyl-silanyloxy)-cyclohexancarbonsäuremethylester

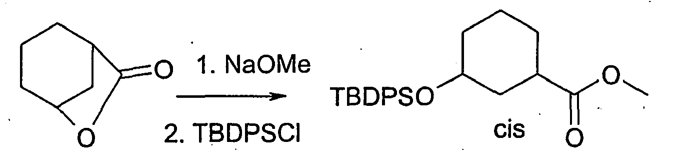

22 g 6-Oxa-bicyclo[3.2.1]octan-7-one werden in 200 ml Methanol gelöst und mit 10%iger Natriummethanolatlösung versetzt bis ein pH von 10 erreicht ist. Man rührt 30 Minuten bei Raumtemperatur nach, dann wird durch Zugabe von verdünnter Essigsäure neutralisiert und das Gemisch im Vakuum eingeengt. Der Rückstand wird in Ethylacetat gelöst, über MgSO4 getrocknet und anschließend im Vakuum eingeengt. Man erhält 21 g des Methylesters als farbloses Öl. Dieser wird in 200 ml Dimethylformamid gelöst und mit 43 g tert-Butyldiphenylsilylchlorid, 13 g Imidazol und 1 g Dimethylaminopyridin versetzt und 12 h bei Raumtemperatur gerührt. Das Lösungsmittel wird im Vakuum entfernt, der Rückstand in Methy-tert.-butylether aufgenommen und mit Wasser gewaschen. Die organische Phase wird über MgSO4 getrocknet und anschließend das Lösungsmittel entfernt. Man erhält 56.8 g ((1R,3S)/(1S,3R)-3-(tert-Butyl-diphenyl-silanyloxy)-cyclohexancarbonsäuremethylester als gelbes Öl. C24H32O3Si (396.61), MS(ESI): 397 (M+H⁺).

### 2-{[(1R,3S)/( 1 S,3R)-3-(tert-Butyl-diphenyl-silanyloxy)-cyclohexancarbonyl]-amino}-(3S)-methyl-buttersäure-tert-butylester

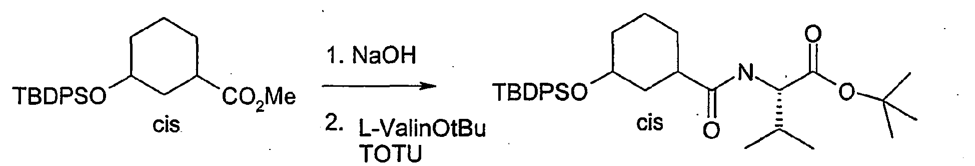

36.8 g ((1R,3S)/(1S,3R)-3-(tert-Butyl-diphenyl-silanyloxy)-cyclohexancarbonsäure werden in 150 ml i-Propanol gelöst und mit 8 g NaOH, gelöst in 50 ml Wasser, versetzt. Es wird 1 Stunde auf 60°C erwärmt. Das Reaktionsgemisch wird abgekühlt und durch Zugabe von 2N HCl neutralisiert. Das Reaktionsgemisch wird im Vakuum eingeengt und dreimal mit je 200 ml Etyhlacetat extrahiert. Die vereinigten organischen Phasen werden über MgSO4 getrocknet und anschließend das Lösungsmittel im Vakuum entfernt. Man erhält 34 g der freien Säure als farbloses Öl (Rf(Ethylacetat) = 0.63). Diese wird in 250 ml Dimethylformamid gelöst und mit 18,6 g L-Valin-tert.-butylesterhydrochlorid versetzt. Bei 0°C werden 29,1 g O-[Cyan(ethoxycarbonyl)methylenamino]-1,1,3,3,-tetramethyluronium-tetrafluoroborat zugegeben. Nach 10 Minuten wird das Eisbad entfernt und 23.9 g Hydroxybenzotriazol und 61,8 ml Hünigsbase zugegeben. Man rührt 2 Stunden bei Raumtemperatur nach. Das Lösungsmittel wird im Vakuum entfernt und der resultierende Rückstand in Ethylacetat gelöst und dreimal mit gesättigter NaHCO3-Lösung gewaschen. Die organische Phase wird über MgSO4 getrocknet und anschließend das Lösungsmittel entfernt. Der Rückstand wird an Kieselgel mit dem Eluens n-Heptan: Ethylacetat = 2:1 gereinigt. Man erhält 43,0 g 2-{[(1R,3S)/( 1S,3R)-3-(tert-Butyl-diphenyl-silanyloxy)-cyclohexancarbonyl]-amino}-(3S)-methyl-buttersäure-tert-butylester als gelbes Öl. C32H47NO4Si (537,82), MS(ESI): 538.

### 2-[((1R,3S)/(1S,3R)-3-Hydroxy-cyclohexancarbonyl)-amino]-(3S)-methyl-buttersäure-tert-butylester

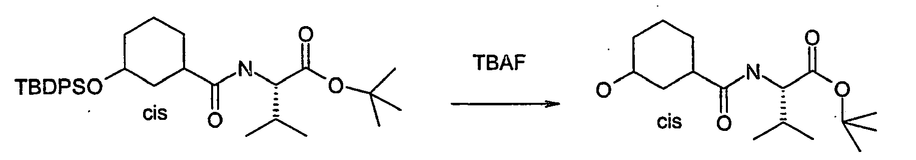

43,0 g 2-{[(1R,3S)/( 1S,3R)-3-(tert-Butyl-diphenyl-silanyloxy)-cyclohexancarbonyl]-amino}-(3S)-methyl-buttersäure-tert-butylester werden in 80 ml Tetrahydrofuran gelöst und mit 80 ml einer 1M Lösung von Tetrabutylammoniumfluorid in Tetrahydrofuran, versetzt. Man rührt 3h bei 60°C nach, dann wird im Vakuum eingeengt und der Rückstand an Kieselgel mit dem Eluens n-Heptan: Ethylacetat = 5:1 => 1:1 gereinigt. Man erhält 18 g eines weißen Feststoffs. Da dieser noch leicht verunreinigt ist werden 8g nochmals einer Kieselgelreinigung unterzogen. Man erhält 6,8 g 2-[((1R,3S)/( 1 S,3R)-3-Hydroxy-cyclohexancarbonyl)-amino]-(3S)-methyl-buttersäure-tert-butylester als weißen Feststoff. C16H29NO4 (299,41), MS(ESI): 300 (M+H⁺).

### (S)-3-Methyl-2-{[(1R,3S)/(1S,3R)-3-(5-methyl-2-p-tolyl-oxazol-4-ylmethoxy)-cyclohexancarbonyl]-amino}-buttersäure

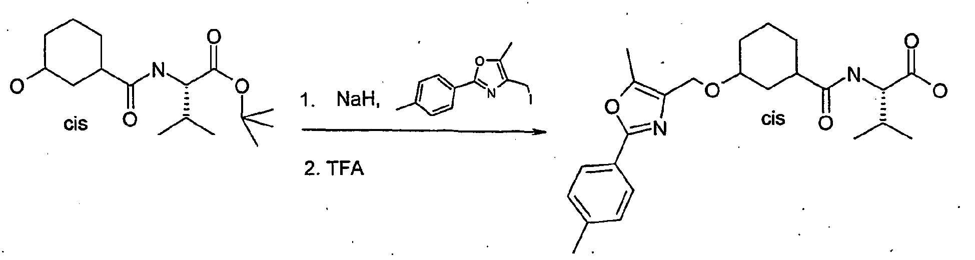

4,0 g 2-[((1R,3S)/( 1S,3R)-3-Hydroxy-cyclohexancarbonyl)-amino]-(3S)-methyl-buttersäure-tert-butylester und 6,3 g 4-Iodmethyl-5-methyl-2-p-tolyl-oxazol werden in 50 ml Dimethylformamid gelöst und portionsweise mit 800 mg Natriumhydrid (60%ig in Paraffinöl) versetzt. Man rührt 1 Stunde bei Raumtemperatur nach, danach wird das Reaktionsgemisch durch Zugabe von 200 ml Methyl-tert.-butyl-ether verdünnt und dreimal mit Wasser gewaschen. Die vereinigten organischen Phasen werden über MgSO4 getrocknet und anschließend das Lösungsmittel im Vakuum entfernt. Der resultierende Rückstand wird in 40 ml Dichlormethan gelöst und mit 20 ml Trifluoressigsäure versetzt. Man rührt 5 Stunden bei Raumtemperatur nach. Anschließend werden 100 ml Toluol zugefügt und die Lösungsmittel im Vakuum entfernt. Der Rückstand wird an Kieselgel mit dem Eluens n-Heptan: Ethylacetat = 2:1 => Ethylacetat gereinigt. Man erhält 1.5 g eines braunen Feststoffes. Dieser wird mittels RP-HPLC weiter gereinigt. Man erhält 1.0 g des Diastereomerengemisches (S)-3-Methyl-2-{[(1R,3S)/( 1 S,3R)-3-(5-methyl-2-p-tolyl-oxazol-4-ylmethoxy)-cyclohexancarbonyl]-amino}-buttersäure **(Beispiel 1)** als farblosen amorphen Feststoff. C24H32N2O5 (428.53), MS(ESI): 429 (M+H⁺).

Das Diastereomerengemisch (S)-3-Methyl-2-{[(1R,3S)/( 1S,3R)-3-(5-methyl-2-p-tolyl-oxazol-4-ylmethoxy)-cyclohexancarbonyl]-amino}-buttersäure aus Beispiel 1 kann durch chirale HPLC getrennt werden. Man erhält (S)-3-Methyl-2-{[(1R,3S)-3-(5-methyl-2-p-tolyl-oxazol-4-ylmethoxy)-cyclohexancarbonyl]-amino}-buttersäure (Beispiel 1 a) und (S)-3-Methyl-2-{[(1S,3R)-3-(5-methyl-2-p-tolyl-oxazol-4-ylmethoxy)-cyclohexancarbonyl]-amino}-buttersäure **(Beispiel 1b)** als farblose Lyophilisate. (Chiralpak AD/34 250x4,6; Eluens n-Heptan:Ethanol:Methanol = 20:1:1+0,1% Trifluoressigsäure; (S)-3-Methyl-2-{[(1R,3S)-3-(5-methyl-2-p-tolyl-oxazol-4-ylmethoxy)-cyclohexancarbonyl]-amino}-buttersäure **(Beispiel 1a)** ,Rt = 4,9 min; (S)-3-Methyl-2-{[(1S,3R)-3-(5-methyl-2-p-tolyl-oxazol-4-ylmethoxy)-cyclohexancarbonyl]-amino}-buttersäure **(Beispiel 1b)**, Rt = 5,7 min).

### Beispiel 2

### (S)-4-Methyl-2-{[(1R,3S)/(1S,3R)-3-(5-methyl-2-p-tolyl-oxazol-4-ylmethoxy)-cyclohexancarbonyl]-amino}-pentansäure

Analog zu Beispiel 1 wurde aus (1R,3S)/( 1S,3R)-3-(tert-Butyl-diphenyl-silanyloxy)-cyclohexancarbonsäure , 4-lodmethyl-5-methyl-2-p-tolyl-oxazol und (L)-Leucin-tert.-butylesterhydrochlorid das Diastereomerengemisch (S)-4-Methyl-2-{[(1R,3S)/( 1S,3R)-3-(5-methyl-2-p-tolyl-oxazol-4-ylmethoxy)-cyclohexancarbonyl]-amino}-pentansäure erhalten. 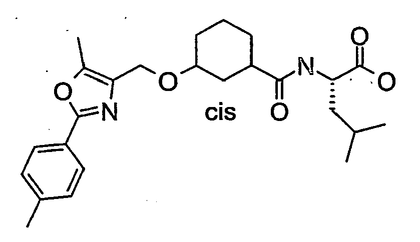 C25H34N2O5 (442.56), MS(ESI): 443 (M+H⁺).

### Beispiel 3

### (S)-2-{[(1R,3S)/(1S,3)-3-(5-Methyl-2-p-tolyl-oxazol-4-ylmethoxy)-cyclohexane-carbonyl]-amino}-propionsäure

Analog zu Beispiel 1 wurde aus (1R,3S)/( 1S,3R)-3-(tert-Butyl-diphenyl-silanyloxy)-cyclohexancarbonsäure, 4-lodmethyl-5-methyl-2-p-tolyl-oxazol und (L)-Alanin-tert.-butylesterhydrochlorid das Diastereomerengemisch (S)-2-{[(1R,3S)/( 1S,3)-3-(5-Methyl-2-p-tolyl-oxazol-4-ylmethoxy)-cyclohexane-carbonyl]-amino}-propionsäure erhalten. 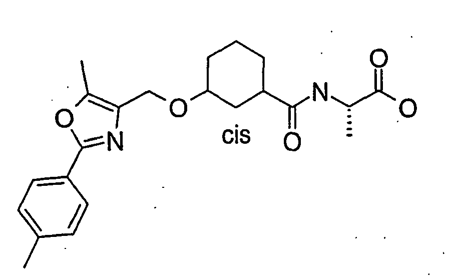 C22H28N2O5 (400.48), MS(ESI): 401 (M+H⁺).

### Beispiel 4

### (S)-2-{[(1R,3S)/(1S,3)-3-(5-Methyl-2-p-tolyl-oxazol-4-ylmethoxy)-cyclohexancarbonyl]-amino}-3-phenyl-propionsäure

Analog zu Beispiel 1 wurde aus (1R,3S)/( 1S,3R)-3-(tert-Butyl-diphenyl-silanyloxy)-cyclohexancarbonsäure, 4-Iodmethyl-5-methyl-2-p-tolyl-oxazol und (L)-Phenylalaninlanin-tert.-butylesterhydrochlorid das Diastereomerengemisch (S)-2-{[(1R,3S)/( 1 S,3)-3-(5-Methyl-2-p-tolyl-oxazol-4-ylmethoxy)-cyclohexancarbonyl]-amino}-3-phenyl-propionsäure erhalten. 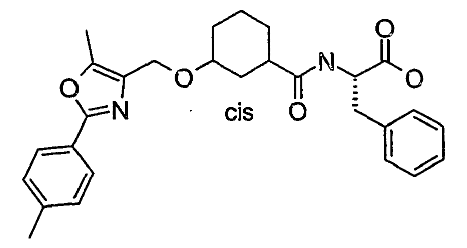 C28H32N2O5 (476.58), MS(ESI): 477 (M+H⁺).

### Beispiel 5

### 2-Methyl-2-{[(1R,3S)/(1S,3R)-3-(5-methyl-2-p-tolyl-oxazol-4-ylmethoxy)-cyclohexancarbonyl]-amino}-propionsäure

Analog zu Beispiel 1 wurde aus (1R,3S)/( 1S,3R)-3-(tert-Butyl-diphenyl-silanyloxy)-cyclohexancarbonsäure, 4-Iodmethyl-5-methyl-2-p-tolyl-oxazol und 2-Amino-2-methyl-propionsäure-tert.-butylesterhydrochlorid das Racemat 2-Methyl-2-{[(1R,3S)/( 1S,3R)-3-(5-methyl-2-p-tolyl-oxazol-4-ylmethoxy)-cyclohexancarbonyl]-amino}-propionsäure erhalten. 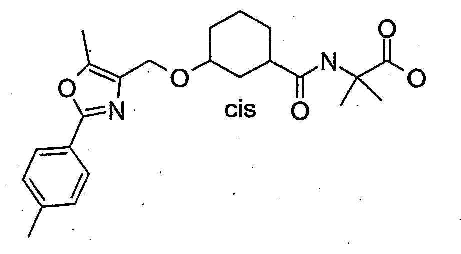 C23H30N2O5 (414.51), MS(ESI): 415 (M+H⁺).

### Beispiel 6

### (R)-3-Methyl-2-{[(1R,3S)/(1S,3R)-3-(5-methyl-2-p-tolyl-oxazol-4-ylmethoxy)-cyclohexancarbonyl]-amino}-buttersäure

Analog zu Beispiel 1 wurde aus (1R,3S)/( 1S,3R)-3-(tert-Butyl-diphenyl-silanyloxy)-cyclohexancarbonsäure, 4-Iodmethyl-5-methyl-2-p-tolyl-oxazol und (D)-Valin-tert.-butylesterhydrochlorid das Diastereomerengemisch (R)-3-Methyl-2-{[(1R,3S)/( 1S,3R)-3-(5-methyl-2-p-tolyl-oxazol-4-ylmethoxy)-cyclohexancarbonyl]-amino}buttersäure erhalten. 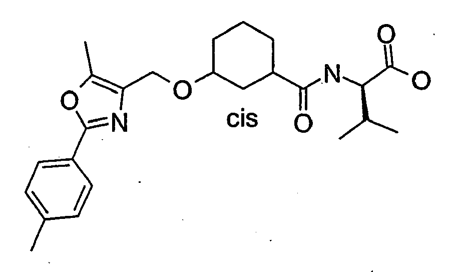 C24H32N2O5 (428.53), MS(ESI): 429 (M+H⁺).

### Beispiel 7

### (S)-1-{(1R,3S)/(1S,3R)-3-[2-(3-Methoxy-phenyl)-5-methyl-oxazol-4-ylmethoxy]-cyclohexancarbonyl}-pyrrolidine-2-carbonsäure

Analog zu Beispiel 1 wurde aus (1R,3S)/(1S,3R)-3-(tert-Butyl-diphenyl-silanyloxy)-cyclohexancarbonsäure, 4-Iodmethyl-2-(3-methoxy-phenyl)-5-methyl-oxazol und (L)-Prolin-tert.-butylesterhydrochlorid das Diastereomerengemisch (S)-1-{(1R, 3S)/(1S,3R)-3-[2-(3-Methoxy-phenyl)-5-methyl-oxazol-4-ylmethoxy]-cyclohexancarbonyl}-pyrrolidine-2-carbonsäure erhalten. 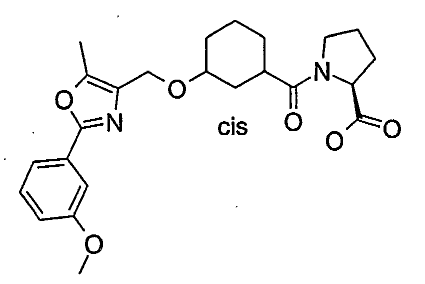 C24H30N2O6 (442.52), MS(ESI): 443 (M+H⁺).

### Beispiel 8

### (S)-1-[(1R,3S)/(1S,3R)-3-(5-Methyl-2-p-tolyl-oxazol-4-ylmethoxy)-cyclohexancarbonyl]-pyrrolidine-2-carbonsäure

Analog zu Beispiel 1 wurde aus (1R,3S)/(1S,3R)-3-(tert-Butyl-diphenyl-silanyloxy)-cyclohexancarbonsäure, 4-Iodmethyl-5-methyl-2-p-tolyl-oxazol und (L)-Prolin-tert.-butylesterhydrochlorid das Diastereomerengemisch (S)-1-[(1R,3S)/(1S,3R)-3-(5-Methyl-2-p-tolyl-oxazol-4-ylmethoxy)-cyclohexancarbonyl]-pyrrolidine-2-carbonsäure erhalten. 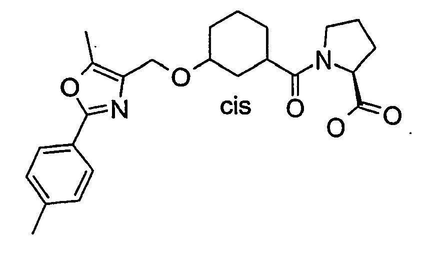 C24H30N2O5 (426.52), MS(ESI): 427 (M+H⁺).

### Beispiel 9

### (S)-2-({(1R,3S)/(1S,3R)-3-[2-(3-Methoxy-phenyl)-5-methyl-oxazol-4-ylmethoxy]-cyclohexancarbonyl}-amino)-3-methyl-buttersäure

Analog zu Beispiel 1 wurde aus (1R,3S)/( 1S,3R)-3-(tert-Butyl-diphenyl-silanyloxy)-cyclohexancarbonsäure, 4-Iodmethyl-2-(3-methoxy-phenyl)-5-methyl-oxazol und (L)-Valin-tert.-butylesterhydrochlorid das Diastereomerengemisch (S)-2-({(1R,3S)/( 1S,3R)-3-[2-(3-Methoxy-phenyl)-5-methyl-oxazol-4-ylmethoxy]-cyclohexancarbonyl}-amino)-3-methyl-buttersäure erhalten. 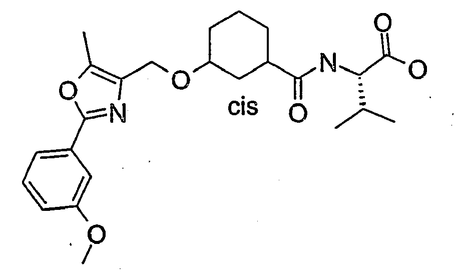 C24H32N2O6 (444.53), MS(ESI): 445 (M+H⁺).

### Beispiel 10

### (S)-2-({(1R,3S)/(1S,3R)-3-[2-(3-Bromo-phenyl)-5-methyl-oxazol-4-ylmethoxy]-cyclohexancarbonyl}-amino)-3-methyl-butyrisäure

Analog zu Beispiel 1 wurde aus (1R,3S)/( 1S,3R)-3-(tert-Butyl-diphenyl-silanyloxy)-cyclohexancarbonsäure, 4-Iodmethyl-2-(3-bromo-phenyl)-5-methyl-oxazol und (L)-Valin-tert.-butylesterhydrochlorid das Diastereomerengemisch (S)-2-({(1R,3S)/( 1 S,3R)-3-[2-(3-Bromo-phenyl)-5-methyl-oxazol-4-ylmethoxy]-cyclohexancarbonyl}-amino)-3-methyl-butyrisäure erhalten. 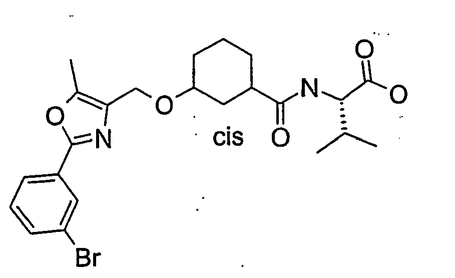 C23H29BrN2O5 (493.40), MS(ESI): 493 (M+H⁺).

### Beispiel 11

### (S)-3-Methyl-2-({(1R,3S)/(1S,3R)-3-[5-methyl-2-(3-trifluoromethyl-phenyl)-oxazol-4-ylmethoxy]-cyclohexancarbonyl}-amino)-buttersäure

Analog zu Beispiel 1 wurde aus (1R,3S)/( 1S,3R)-3-(tert-Butyl-diphenyl-silanyloxy)-cyclohexancarbonsäure, 4-Iodmethyl-2-(3-trifluoromethyl-phenyl)-5-methyl-oxazol und (L)-Valin-tert.-butylesterhydrochlorid das Diastereomerengemisch (S)-3-Methyl-2-({(1R,3S)/(1S,3R)-3-[5-methyl-2-(3-trifluoromethyl-phenyl)-oxazol-4-ylmethoxy]-cyclohexancarbonyl}-amino)-buttersäure erhalten. 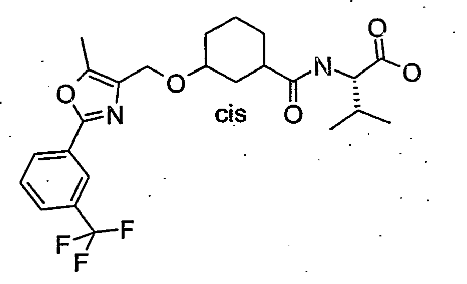 C24H29F3N2O5 (482.50), MS(ESI): 483 (M+H⁺).

### Beispiel 12

### (S)-2-{[(1R,3S)/(1S,3R)-3-(5-Isopropyl-2-p-tolyl-oxazol-4-ylmethoxy)-cyclohexancarbonyl]-amino}-3-methyl-buttersäure

Analog zu Beispiel 1 wurde aus aus 2-[((1R,3S)/(1S,3R)-3-Hydroxy-cyclohexancarbonyl)amino]-(3S)-methyl-buttersäure-tert-butylester und 4-Iodmethyl-2-(4-methyl-phenyl)-5-isopropyl-oxazol das Diastereomerengemisch (S)-2-{[(1R,3S)/(1S,3R)-3-(5-Isopropyl-2-p-tolyl-oxazol-4-ylmethoxy)-cyclohexancarbonyl]-amino}-3-methyl-buttersäure **(Beispiel 12)** erhalten. 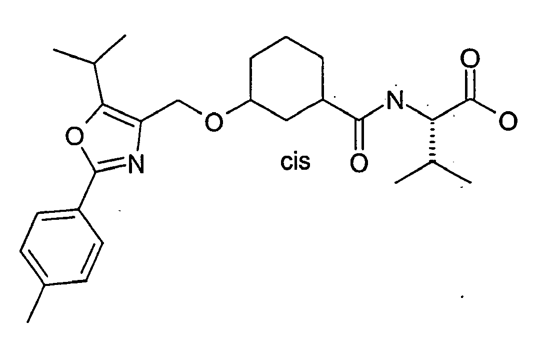 C26H36N2O5 8456.59), MS(ESI): 457 (M+H⁺).

Das Diastereomerengemisch (S)-2-{[(1R,3S) /( 1S,3R)-3-(5-Isopropyl-2-p-tolyl-oxazol-4-ylmethoxy)-cyclohexancarbonyl]-amino}-3-methyl-buttersäure aus **Beispiel 12** kann durch chirale HPLC unter angepaßten Bedingungen analog zu Beispiel 1 getrennt werden. Man erhält (S)-2-{[(1R,3S) -3-(5-Isopropyl-2-p-tolyl-oxazol-4-ylmethoxy)-cyclohexancarbonyl]-amino}-3-methyl-buttersäure **(Beispiel 12a)** und (S)-2-{[( 1S,3R)-3-(5-Isopropyl-2-p-tolyl-oxazol-4-ylmethoxy)cyclohexancarbonyl]-amino}-3-methyl-buttersäure **(Beispiel 12b)** als farblose Lyophilisate.

### Beispiel 13

### (S)-2-({(1R,3S)/(1S,3R)-3-[5-Isopropyl-2-(3-methoxy-phenyl)-oxazol-4-ylmethoxy]-cyclohexancarbonyl}-amino)-3-methyl-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(3-methoxy-phenyl)-5-isopropyl-oxazol das Diastereomerengemisch (S)-2-({(1R,3S)/(1S,3R)-3-[5-Isopropyl-2-(3-methoxy-phenyl)-oxazol-4-ylmethoxy]-cyclohexancarbonyl}-amino)-3-methyl-buttersäure erhalten. **(Beispiel 13)** 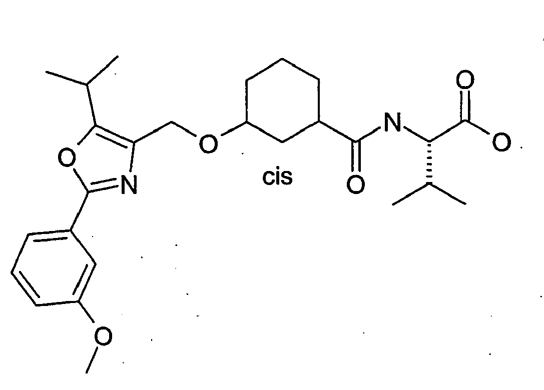 C26H36N2O6 (472.59), MS(ESI): 473 (M+H⁺).

Das Diastereomerengemisch (S)-2-({(1R,3S)/(1S,3R)-3-[5-Isopropyl-2-(3-methoxyphenyl)-oxazol-4-ylmethoxy]-cyclohexancarbonyl}-amino)-3-methyl-buttersäure aus **Beispiel 13** kann durch chirale HPLC unter angepaßten Bedingungen analog zu Beispiel 1 getrennt werden. Man erhält (S)-2-({(1R,3S)-3-[5-Isopropyl-2-(3-methoxyphenyl)-oxazol-4-ylmethoxy]-cyclohexancarbonyl}-amino)-3-methyl-buttersäure **(Beispiel 13a)** und (S)-2-({(1S,3R)-3-[5-Isopropyl-2-(3-methoxy-phenyl)-oxazol-4-ylmethoxy]-cyclohexancarbonyl}-amino)-3-methyl-buttersäure **(Beispiel 13b)** als farblose Lyophilisate.

### Beispiel 14

### (S)-2-({(R,3S)/( 1S,3R)-3-[5-Isopropyl-2-(3-methoxy-phenyl)-oxazol-4-ylmethoxy]-cyclohexancarbonyl}-amino)-3-methyl-buttersäure

Analog zu Beispiel 1 wurde aus (1R,3S)/( 1S,3R)-3-(tert-Butyl-diphenyl-silanyloxy)-cyclohexancarbonsäure, 4-lodmethyl-2-(3-methoxy-phenyl)-5-isopropyl-oxazol und (L)-Valin-tert.-butylesterhydrochlorid das Diastereomerengemisch (S)-2-({(1R,3S)/( 1S,3R)-3-[5-Isopropyl-2-(3-methoxy-phenyl)-oxazol-4-ylmethoxy]-cyclohexancarbonyl}-amino)-3-methyl-buttersäure erhalten. 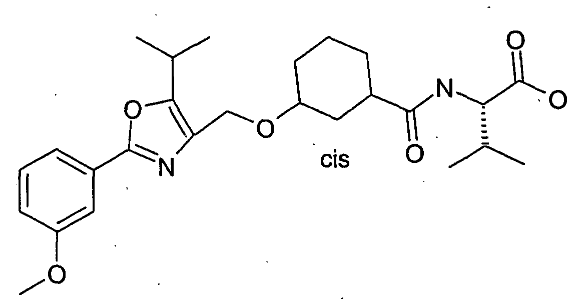 C26H36N2O6 (472.59), MS(ESI): 473 (M+H⁺).

### Beispiel 15

### (S)-2-({(1R,3S/(1S,3R)-3-[2-(3-Methoxy-phenyl)-5-trifluoromethyl-oxazol-4-ylmethoxy]-cyclohexancarbonyl}-amino)-3-methyl-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(3-methoxy-phenyl)-5-trifluormethyl-oxazol das Diastereomerengemisch (S)-2-({(1R,3S/(1S,3R)-3-[2-(3-Methoxy-phenyl)-5-trifluoromethyl-oxazol-4-ylmethoxy]-cyclohexancarbonyl}-amino)-3-methyl-buttersäure erhalten. 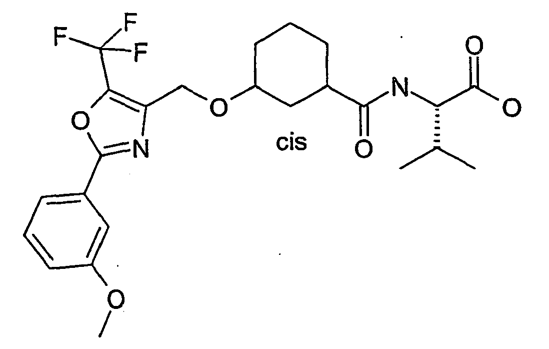 C24H29F3N2O6 (498.50), MS(ESI): 499 (M+H⁺).

Das Diastereomerengemisch ((SF2-({(1R,3S) /( 1S,3R)-3-[2-(3-Methoxy-phenyl)-5-trifluoromethyl-oxazol-4-ylmethoxy]-cyclohexancarbonyl}-amino)-3-methyl-buttersäure aus **Beispiel 15** kann durch chirale HPLC unter angepaßten Bedingungen analog zu Beispiel 1 getrennt werden. Man erhält (S)-2-({(1R,3S) -3-[2-(3-Methoxy-phenyl)-5-trifluoromethyl-oxazol-4-ylmethoxy]-cyclohexancarbonyl}-amino)-3-methyl-buttersäure

### (Beispiel 15a) und (S)-2-({( 1S,3R)-3-[2-(3-Methoxy-phenyl)-5-trifluoromethyl-oxazol-4-ylmethoxy]-cyclohexancarbonyl}-amino)-3-methyl-buttersäure (Beispiel 15b) als farblose Lyophilisate.

### Beispiel 16

### (S)-3-Methyl-2-({(1R,3S)/(1S,3R)-3-[5-trifluoromethyl-2-(3-trifluoromethyl-phenyl)-oxazol-4-ylmethoxy]-cyclohexancarbonyl}-amino)-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(3-trifluormethyl - phenyl)-5-trifluormethyl-oxazol das Diastereomerengemisch (S)-3-Methyl-2-({(1R,3S)/(1S,3R)-3-[5-trifluoromethyl-2-(3-trifluoromethyl-phenyl)-oxazol-4-ylmethoxy]-cyclohexancarbonyl}-amino)-buttersäure erhalten. 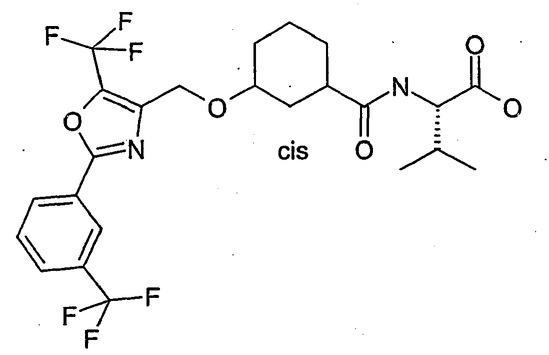 C24H26F6N2O5 (536.48), MS(ESI): 537 (M+H⁺).

Das Diastereomerengemisch (S)-3-Methyl-2-({(1R,3S)/(1S,3R)-3-[5-trifluoromethyl-2-(3-trifluoromethyl-phenyl)-oxazol-4-ylmethoxy]-cyclohexancarbonyl}amino)-buttersäure aus **Beispiel 16** kann durch chirale HPLC unter angepaßten Bedingungen analog zu Beispiel 1 getrennt werden. Man erhält (S)-3-Methyl-2-({(1R,3S)-3-[5-trifluoromethyl-2-(3-trifluoromethyl-phenyl)-oxazol-4-ylmethoxy]-cyclohexancarbonyl}-amino)-buttersäure **(Beispiel 16a)** und (S)-3-Methyl-2-({( 1S,3R)-3-[5-trifluoromethyl-2-(3-trifluoromethyl-phenyl)-oxazol-4-ylmethoxy]-cyclohexancarbonyl}-amino)-buttersäure **(Beispiel 16b)** als farblose Lyophilisate.

### Beispiel 17

### (S)-3-Methyl-2-{[(1R,3S)/(1S,3R)-3-(2-p-tolyl-5-trifluoromethyl-oxazol-4-ylmethoxy)-cyclohexancarbonyl]-amino}-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(4-methyl-phenyl)-5-trifluormethyl-oxazol das Diastereomerengemisch (S)-3-Methyl-2-{[(1R,3S)/(1S,3R)-3-(2-p-tolyl-5-trifluoromethyl-oxazol-4-ylmethoxy)-cyclohexancarbonyl]-amino}-buttersäure erhalten. 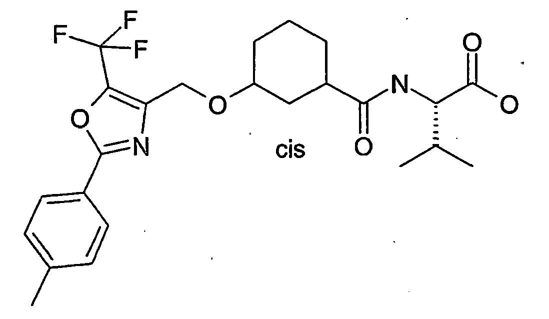 C24H29F3N2O5 (482.50), MS(ESI): 483 (M+H⁺).

Das Diastereomerengemisch (S)-3-Methyl-2-{[(1R,3S)/(1S,3R)-3-(2-p-tolyl-5-trifluoromethyl-oxazol-4-ylmethoxy)-cyclohexancarbonyl]-amino}-buttersäure aus **Beispiel 17** kann durch chirale HPLC unter angepaßten Bedingungen analog zu Beispiel 1 getrennt werden. Man erhält (S)-3-Methyl-2-{[(1R,3S)-3-(2-p-tolyl-5-trifluoromethyl-oxazol-4-ylmethoxy)-cyclohexancarbonyl]-amino}-buttersäure (Beispiel 17a) und (S)-3-Methyl-2-{[( 1S,3R)-3-(2-p-tolyl-5-trifluoromethyl-oxazol-4-ylmethoxy)-cyclohexancarbonyl]-amino}-buttersäure **(Beispiel 17b)** als farblose Lyophilisate.

### Beispiel 18

### (S)-3-Methyl-2-{[(1R,3S)/(1S,3R)-3-(5-phenyl-2-p-tolyl-oxazol-4-ylmethoxy)-cyclohexancarbonyl]-amino}-buttersäure

Analog zu Beispiel 1 wurde aus 2-[((1R,3S) /( 1 S,3R)-3-Hydroxy-cyclohexancarbonyl)-amino]-(3S)-methyl-buttersäure-tert-butylester und 4-Iodmethyl-2-(4-methyl-phenyl)-5-phenyl-oxazol das Diastereomerengemisch (S)-3-Methyl-2-{[(1R,3S)/(1S,3R)-3-(5-phenyl-2-p-tolyl-oxazol-4-ylmethoxy)-cyclohexancarbonyl]-amino}-buttersäure erhalten. 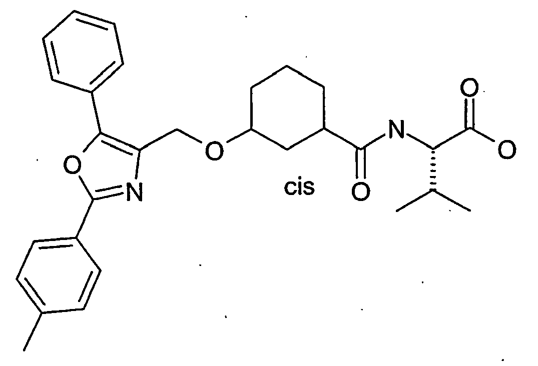 C29H34N2O5 (490.60), MS(ESI): 491 (M+H⁺).

Das Diastereomerengemisch (S)-3-Methyl-2-{[ (1R,3S) /(1S,3R)-3-(5-phenyl-2-p-tolyl-oxazol-4-ylmethoxy)-cyclohexancarbonyl]-amino}-buttersäure aus **Beispiel 18** kann durch chirale HPLC unter angepaßten Bedingungen analog zu Beispiel 1 getrennt werden. Man erhält (S)-3-Methyl-2-{[(1R,3S) -3-(5-phenyl-2-p-tolyl-oxazol-4-ylmethoxy)-cyclohexancarbonyl]-amino}-buttersäure **(Beispiel 18a)** und (S)-3-Methyl-2-{[(1S,3R)-3-(5-phenyl-2-p-tolyl-oxazol-4-ylmethoxy)-cyclohexancarbonyl]-amino}-buttersäure **(Beispiel 18b)** als farblose Lyophilisate.

### Beispiel 19

### (S)-2-({(1R,3S)/(1S,3R)-3-[2-(3-Methoxy-phenyl)-5-phenyl-oxazol-4-ylmethoxy]-cyclohexancarbonyl}-amino)-3-methyl-buttersäure

Analog zu Beispiel 1 wurde aus (1R,3S)/(1S,3R)-3-(tert-Butyl-diphenyl-silanyloxy)-cyclohexancarbonsäure, 4-lodmethyl-2-(3-methoxy-phenyl)-5-phenyl-oxazol und (L)-Valin-tert.-butylesterhydrochlorid das Diastereomerengemisch (S)-2-({(1R,3S) /(1S,3R)-3-[2-(3-Methoxy-phenyl)-5-phenyl-oxazol-4-ylmethoxy]-cyclohexancarbonyl}-amino)-3-methyl-buttersäure erhalten. 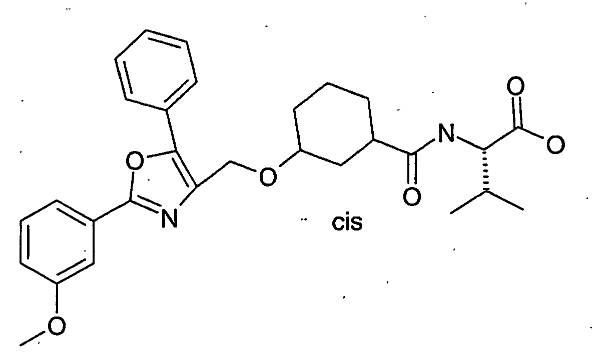 C29H34N2O6 (506.60), MS(ESI): 507 (M+H⁺).

### Beispiel 20

### (S)-2-({(1R,3S)/(1S,3R)-3-[2-(3-Methoxy-phenyl)-5-ethyl-oxazol-4-ylmethoxy]-cyclohexancarbonyl}-amino)-3-methyl-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(3-methoxy-phenyl)-5-ethyl-oxazol das Diastereomerengemisch (S)-2-({(1R,3S)/(1S,3R)-3-[2-(3-Methoxyphenyl)-5-ethyl-oxazol-4-ylmethoxy]-cyclohexancarbonyl}-amino)-3-methyl-buttersäure erhalten. 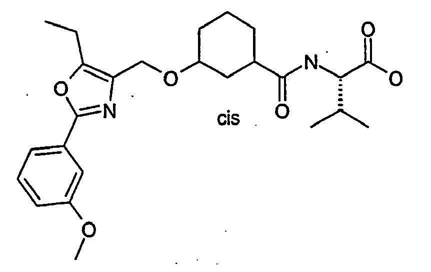 C25H34N2O6 (458.56), MS(ESI): 459 (M+H⁺).

Das Diastereomerengemisch (S)-2-({(1R,3S) /( 1S,3R)-3-[2-(3-Methoxy-phenyl)-5-ethyl-oxazol-4-ylmethoxy]-cyclohexancarbonyl}amino)-3-methyl-buttersäure aus **Beispiel 20** kann durch chirale HPLC unter angepaßten Bedingungen analog zu Beispiel 1 getrennt werden. Man erhält (S)-2-({(1R,3S)-3-[2-(3-Methoxy-phenyl)-5-ethyl-oxazol-4-ylmethoxy]-cyclohexancarbonyl}-amino)-3-methyl-buttersäure **(Beispiel 20a)** und (S)-2-({( 1 S,3R)-3-[2-(3-Methoxy-phenyl)-5-ethyl-oxazol-4-ylmethoxy]-cyclohexancarbonyl}-amino)-3-methyl-buttersäure **(Beispiel 20b)** als farblose Lyophilisate.

### Beispiel 21

### (S)-3-Methyl-2-{[(1R,3S)/(1S,3R)-3-(5-ethyl-2-p-tolyl-oxazol-4-ylmethoxy)-cyclohexancarbonyl]-amino}-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(4-methyl-phenyl)-5-ethyl-oxazol das Diastereomerengemisch (S)-3-Methyl-2-{[(1R,3S)/(1S,3R)-3-(5-ethyl-2-p-tolyl-oxazol-4-ylmethoxy)-cyclohexancarbonyl]-amino}-buttersäure erhalten. 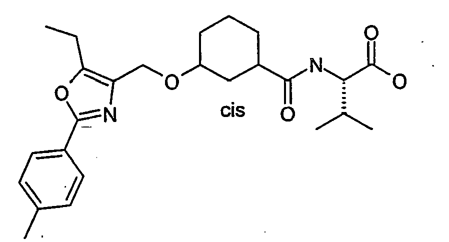 C25H34N2O5 (442.56), MS(ESI): 443 (M+H⁺).

Das Diastereomerengemisch (S)-3-Methyl-2-{[(1R,3S)/(1 S,3R)-3-(5-ethyl-2-p-tolyl-oxazol-4-ylmethoxy)-cyclohexancarbonyl]-amino}-buttersäure aus **Beispiel 21** kann durch chirale HPLC unter angepaßten Bedingungen analog zu Beispiel 1 getrennt werden. Man erhält (S)-3-Methyl-2-{[(1R,3S) -3-(5-ethyl-2-p-tolyl-oxazol-4-ylmethoxy)-cyclohexancarbonyl]-amino}-buttersäure **(Beispiel 21a)** und (S)-3-Methyl-2-{[( 1S,3R)-3-(5-ethyl-2-p-tolyl-oxazol-4-ylmethoxy)-cyclohexancarbonyl]-amino}-buttersäure **(Beispiel 21b)** als farblose Lyophilisate.

### Beispiel 22

### (S)-2-({(1R,3S)/(1S,3R)-3-[2-(3-Methoxy-phenyl)-5-cyclohexyl-oxazol-4-ylmethoxy]-cyclohexancarbonyl}-amino)-3-methyl-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(3-methoxy-phenyl)-5-cyclohexyl -oxazol das Diastereomerengemisch (S)-2-({(1R,3S)/(1S,3R)-3-[2-(3-Methoxy-phenyl)-5-cyclohexyl-oxazol-4-ylmethoxy]-cyclohexancarbonyl}-amino)-3-methyl-buttersäure erhalten. 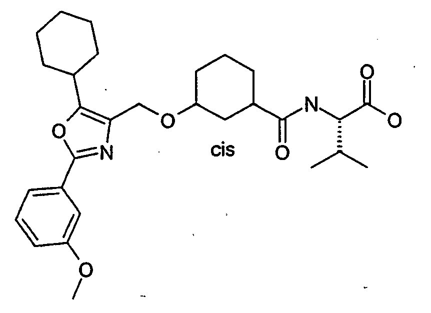 C29H40N2O6 (512.65), MS(ESI): 513 (M+H⁺).

Das Diastereomerengemisch (S)-2-({(1R,3S)/(1S,3R)-3-[2-(3-Methoxy-phenyl)-5-cyclohexyl -oxazol-4-ylmethoxy]-cyclohexancarbonyl}-amino)-3-methyl-buttersäure aus **Beispiel 22** kann durch chirale HPLC unter angepaßten Bedingungen analog zu Beispiel 1 getrennt werden. Man erhält (S)-2-({(1R,3S)-3-[2-(3-Methoxy-phenyl)-5-cyclohexyl-oxazol-4-ylmethoxy]-cyclohexancarbonyl}-amino)-3-methyl-buttersäure **(Beispiel 22a)** und (S)-2-({(1S,3R)-3-[2-(3-Methoxy-phenyl)-5-cyclohexyl -oxazol-4-ylmethoxy]-cyclohexancarbonyl}-amino)-3-methyl-buttersäure **(Beispiel 22b)** als farblose Lyophilisate.

### Beispiel 23

### (S)-3-Methyl-2-{[(1R,3S)/(1S,3R)-3-(5-cyclohexyl-2-p-tolyl-oxazol-4-ylmethoxy)-cyclohexancarbonyl]-amino}-buttersäure

Analog zu Beispiel 1 wurde aus 2-[((1R,3S)/(1S,3R)-3-Hydroxy-cyclohexancarbonyl)-amino]-(3S)-methyl-buttersäure-tert-butylester und 4-Iodmethyl-2-(4-methyl-phenyl)-5-cyclohexyl-oxazol das Diastereomerengemisch (S)-3-Methyl-2-{[(1R,3S)/(1S,3R)-3-(5-cyclohexyl-2-p-tolyl-oxazol-4-ylmethoxy)-cyclohexancarbonyl]-amino}-buttersäure erhalten. 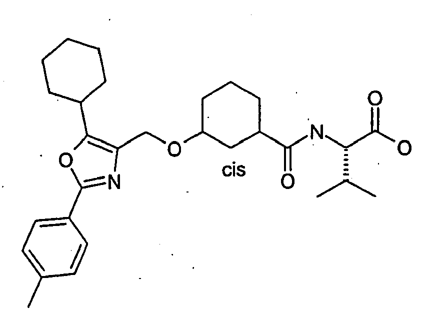 C29H40N2O5 (496.65), MS(ESI): 497 (M+H⁺).

Das Diastereomerengemisch (S)-3-Methyl-2-{[(1R,3S) /( 1S,3R)-3-(5-cyclohexyl-2-p-tolyl-oxazol-4-ylmethoxy)-cyclohexancarbonyl]-amino}-buttersäure aus **Beispiel 23** kann durch chirale HPLC unter angepaßten Bedingungen analog zu Beispiel 1 getrennt werden. Man erhält (S)-3-Methyl-2-{[(1R,3S) -3-(5-cyclohexyl-2-p-tolyl-oxazol-4-ylmethoxy)-cyclohexancarbonyl]-amino}-buttersäure **(Beispiel 23a)** und (S)-3-Methyl-2-{[(1S,3R)-3-(5-cyclohexyl-2-p-tolyl-oxazol-4-ylmethoxy)-cyclohexancarbonyl]-amino}-buttersäure **(Beispiel 23b)** als farblose Lyophilisate.

### Beispiel 24

### 1-{[(1R,3S)/(1 S,3R)-3-(5-Methyl-2-p-tolyl-oxazol-4-ylmethoxy)-cyclohexancarbonyl]-amino}-cyclohexane-carbonsäure

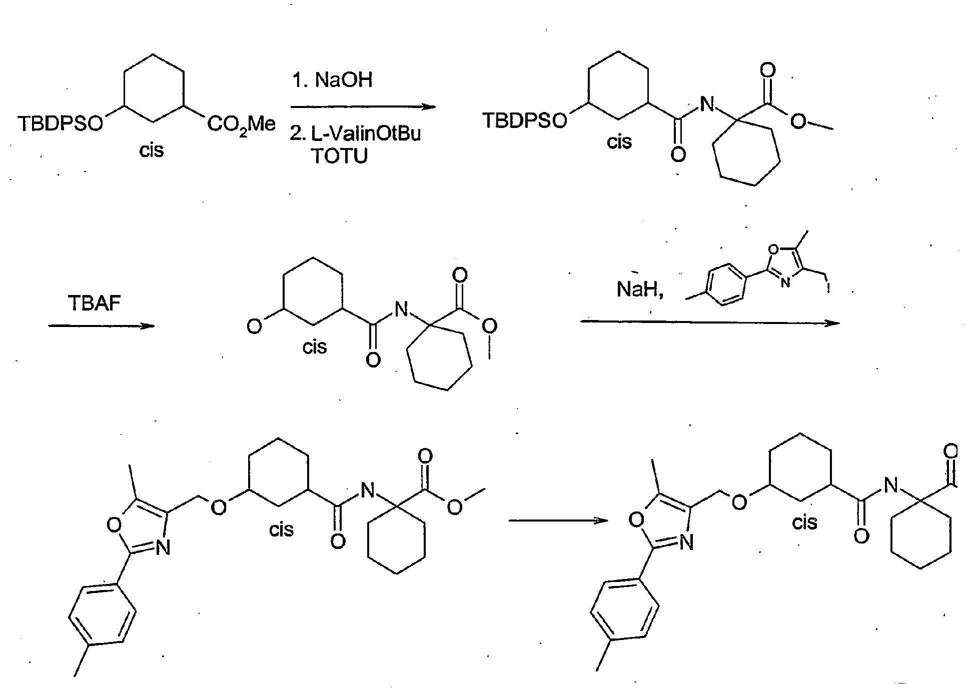

Analog zu Beispiel 1 wurde aus (1R,3S)/( 1S,3R)-3-(tert-Butyl-diphenyl-silanyloxy)-cyclohexancarbonsäure, 1-Amino-cyclohexancarbonsäuremethylesterhydrochlorid und 4-lodmethyl-5-methyl-2-p-tolyl-oxazol das Racemat 1-{[(1R,3S)/( 1S,3R)-3-(5-Methyl-2-p-tolyl-oxazol-4-ylmethoxy)-cyclohexancarbonyl]-amino}-cyclohexancarbonsäuremethylester erhalten. C27H36N2O5(468,60), ), MS(ESI): 469.

### 1-{[(1R,3S)/(1S,3R)-3-(5-Methyl-2-p-tolyl-oxazol-4-ylmethoxy)-cyclohexancarbonyl]-amino}-cyclohexane-carbonsäure

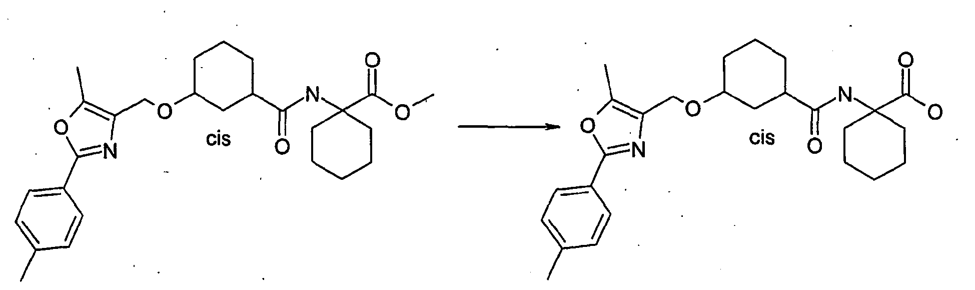

500 mg des Racemats 1-{[(1R,3S)/(1S,3R)-3-(5-Methyl-2-p-tolyl-oxazol-4-ylmethoxy)-cyclohexancarbonyl]-amino}-cyclohexancarbonsäuremethylester werden in 10 ml tert.-Butanol gelöst und mit 1 ml 10 N KOH versetzt. Es wird 1 Tag unter Rückfluss zum Sieden erhitzt. Nach Neutralisation mit 2N HCl wird die organische Phase abgetrennt und die wässrige Phase dreimal mit je 20 ml Ethylacetat extrahiert. Die vereinigten organischen Phasen werden über Magnesiumsulfat getrocknet und anschließend die Lösungsmittel im Vakuum entfernt. Der resultierende Rückstand wird durch RP-HPLC gereinigt. Man erhält 170 mg 1-{[(1R,3S)/( 1S,3R)-3-(5-Methyl-2-p-tolyl-oxazol-4-ylmethoxy)-cyclohexancarbonyl]-amino}-cyclohexane-carbonsäure als farbloses Lyophilisat. C26H34N2O5 (454.47), MS(ESI): 455( M + H⁺).

### Beispiel 25

### 1-{[(1R,3S)/(1S,3R)-3-(5-Methyl-2-p-tolyl-oxazol-4-ylmethoxy)-cyclohexancarbonyl]-amino}-cyclopentancarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 4-Iodmethyl-5-methyl-2-p-tolyl-oxazol das Racemat 1-{[(1R,3S)/(1S,3R)-3-(5-Methyl-2-p-tolyl-oxazol-4-ylmethoxy)-cyclohexancarbonyl]-amino}-cyclopentancarbonsäure erhalten. 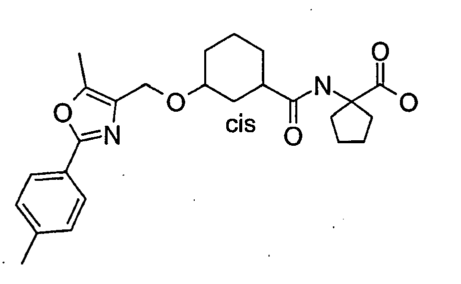 C25H32N2O5 (440.54), MS(ESI): 441 ( M + H⁺).

### Beispiel 26

### 1-({(1R,3S)/(1S,3R)-3-[2-(3-Methoxy-phenyl)-5-methyl-oxazol-4-ylmethoxy]-cyclohexancarbonyl}-amino)-cyclopentancarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 4-Iodmethyl-2-(3-methoxy-phenyl)-5-methyl-oxazol das Racemat 1-({(1R,3S)/(1S,3R)-3-[2-(3-Methoxy-phenyl)-5-methyl-oxazol-4-ylmethoxy]-cyclohexancarbonyl}-amino)-cyclopentancarbonsäure erhalten. 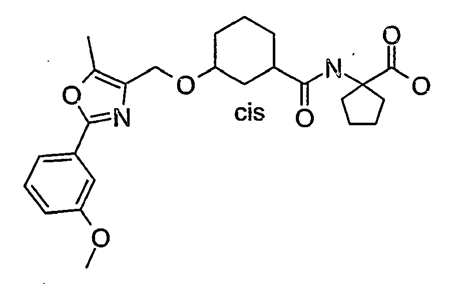 C25H32N2O6 (456.54), MS(ESI): 457( M + H⁺).

### Beispiel 27

### 1-({(1R,3S)/(1S,3R)-3-[5-Methyl-2-(3-trifluoromethyl-phenyl)-oxazol-4-ylmethoxy]-cyclohexancarbonyl}-amino)-cyclopentancarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 4-lodmethyl-2-(3-trifluormethyl-phenyl)-5-methyl-oxazol das Racemat 1-({(1R,3S)/(1S,3R)-3-[5-Methyl-2-(3-trifluoromethyl-phenyl)-oxazol-4-ylmethoxy]-cyclohexancarbonyl}-amino)-cyclopentancarbonsäure erhalten. 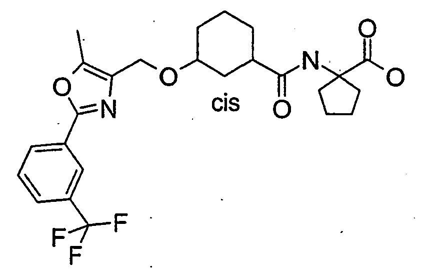 C25H29F3N2O5 (494.52), MS(ESI): 495( M + H⁺).

### Beispiel 28

### 3-Methyl-2-{methyl-[(1R,3S)/(1S,3R)-(5-methyl-2-p-tolyl-oxazol-4-ylmethoxy)-cyclohexancarbonyl]-amino}-buttersäure

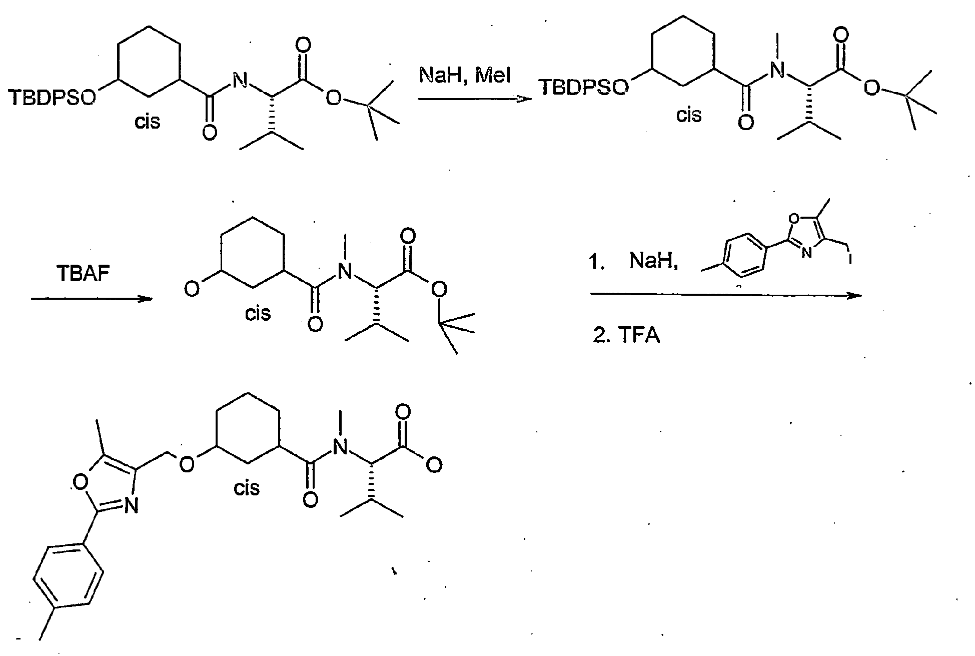

### 2-{[(1R,3S)/( 1S,3R)-3-(tert-Butyl-diphenyl-silanyloxy)-cyclohexancarbonyl]-methyl-amino}-3-methyl-buttersäure-tert-butylester

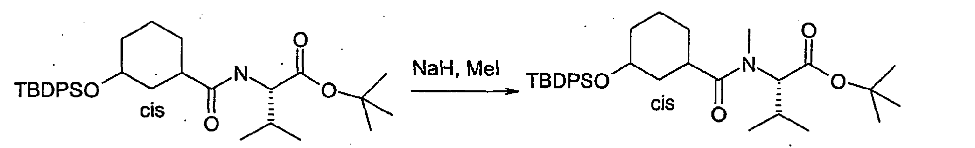

220 mg Natriumhydrid (60%ig in Paraffinöl) werden in 20 ml Dimethylformamid suspendiert. Zu dieser Suspension werden 2 g des Diastereomerengemisches 2-{[(1R,3S)/(1S,3R)-3-(tert-Butyl-diphenyl-silanyloxy)-cyclohexancarbonyl]-amino}-(3S)-methyl-buttersäure-tert-butylester gelöst in 10 ml Dimethylformamid, zugegeben. Man rührt 15 Minuten bei Raumtemperatur nach, dann wird 0,5 ml Methyliodid zugetropft. Nach 2 Stunden Rühren bei Raumtemperatur wird das Reaktionssgemisch mit 200 ml Ethylacetat verdünnt und dreimal mit Wasser und gesättigter Nach-Lösung gewaschen. Die vereinigten organischen Phasen werden über MgSO4 getrocknet und anschließend das Lösungsmittel im Vakuum entfernt. Man erhält 2,3 g 2-{[(1R,3S)/( 1 S,3R)-3-(tert-Butyl-diphenyl-silanyloxy)-cyclohexancarbonyl]-methyl-amino}-3-methyl-buttersäure-tert-butylester als gelbes Öl. C33H49NO4Si (551,85), MS(ESI): 552( M + H⁺).

### 2-[((1R,3S)/(1S,3R)-3-Hydroxy-cyclohexancarbonyl)-methyl-amino]-3-methyl-buttersäure -tert-butylester

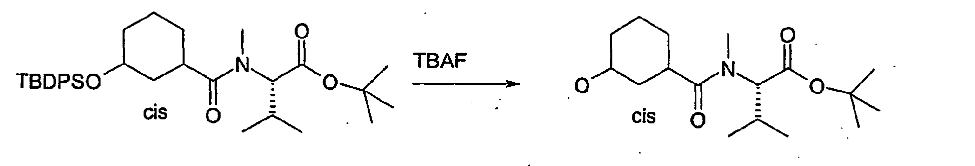

2,3 g des Diastereomerengemisches 2-{[(1R,3S)/(1S,3R)-3-(tert-Butyl-diphenyl-silanyloxy)-cyclohexancarbonyl]-methyl-amino}-3-methyl-buttersäure-tert-butylester werden in 10 ml Tetrahydrofuran gelöst und mit 6 ml einer 1 M Lösung von Tetrabutylammoniumfluorid in Tetrahydrofuran, versetzt. Man rührt 2 Stunden bei 60°C nach, dann wird im Vakuum eingeengt und der Rückstand an Kieselgel mit dem Eluens n-Heptan: Ethylacetat = 2:1 => Ethylacetat gereinigt. Man erhält 970 mg 2-[((1R,3S)/( 1 S,3R)-3-Hydroxy-cyclohexancarbonyl)-methyl-amino]-3-methyl-buttersäure -tert-butylester als gelbes Öl. C17H31NO4 (313,44), MS(ESI): 314( M + H⁺), Rf(n-Heptan:Ethylacetat = 1:1) = 0,18.

### 3-Methyl-2-{methyl-[(1R,3S)/(1S,3R)-(5-methyl-2-p-tolyl-oxazol-4-ylmethoxy)-cyclohexancarbonyl]-amino}-buttersäure

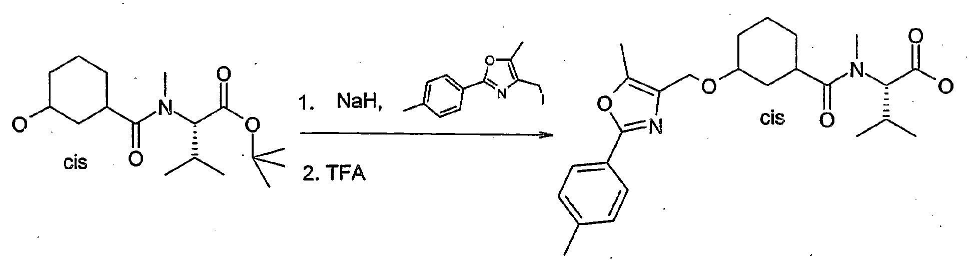

150 mg Natriumhydrid (60%ig in Paraffinöl) werden in 5 ml Dimethylformamid suspendiert. Zu dieser Suspension werden 970 mg des Diastereomerengemisches 2-[((1R,3S)/(1S,3R)-3-Hydroxy-cyclohexancarbonyl)-methyl-amino]-3-methyl-butersäure -tert-butylester, gelöst in 10 ml Dimethylformamid, zugegeben. Man rührt 15 Minuten bei Raumtemperatur nach, dann werden 1.5 g 4-Iodmethyl-5-methyl-2-p-tolyl-oxazol, gelöst in 10 ml Dimethylformamid, zugetropft. Nach 2 Stunden Rühren bei Raumtemperatur wird das Reaktionsgemisch mit 200 ml Methyl-tert.-butyl-ether verdünnt und dreimal mit Wasser und gesättigter NaCl-Lösung gewaschen. Die vereinigten organischen Phasen werden über MgSO4 getrocknet und anschließend das Lösungsmittel im Vakuum entfernt. Der resultierende Rückstand wird in 10 ml Dichlormethan gelöst und mit 5 ml Trifluoressigsäure versetzt. Man rührt 2 Stunden bei Raumtemperatur nach. Dann wird 50 ml Toluol zugegeben und die Lösungsmittel im Vakuum entfernt. Der Rückstand wird mittels RP-HPLC gereinigt. Nach Gefriertrocknung erhält man 105 mg 3-Methyl-2-{methyl-[(1R,3S)/(1S,3R)-(5-methyl-2-p-tolyl-oxazol-4-ylmethoxy)-cyclohexancarbonyl]-amino}-buttersäure als weißes Lyophilisat. C25H34N2O5 (442,56), MS(ESI): 443( M + H⁺).

### Beispiel 29

### (S)-2-{Benzyl-[(1R,3S)/(1S,3R)-3-(5-methyl-2-p-tolyl-oxazol-4-ylmethoxy)-cyclohexancarbonyl]-amino}-3-methyl-buttersäure

Analog zu Beispiel 28 wurde aus dem Diastereomerengemisch 2-{[(1R,3S)/( 1S,3R)-3-(tert-Butyl-diphenyl-silanyloxy)-cyclohexancarbonyl]-amino}-(3S)-methyl-buttersäure-tert-butylester , Benzylbromid und 4-Iodmethyl-5-methyl-2-p-tolyl-oxazol das Diastereomerengemisch (S)-2-{Benzyl-[(1R,3S)/(1S,3R)-3-(5-methyl-2-p-tolyl-oxazol-4-ylmethoxy)-cyclohexancarbonyl]-amino}-3-methyl-buttersäure erhalten. 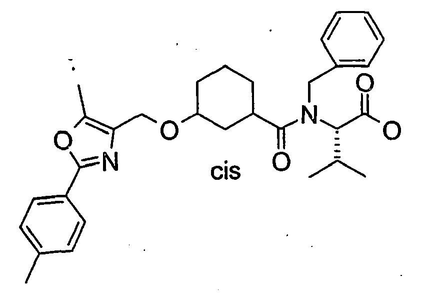 C31 H38N2O5 (518.66), MS(ESI): 519( M + H⁺).

### Beispiel 30

### (S)-3-Methyl-2-{[(1R,3S)/(1R,3S)-3-(5-methyl-2-p-tolyl-oxazol-4-ylmethoxy)-cyclohexancarbonyl]-propyl-amino}-buttersäure

Analog zu Beispiel 28 wurde aus dem Diastereomerengemisch 2-{[(1R,3S)/( 1S,3R)-3-(tert-Butyl-diphenyl-silanyloxy)-cyclohexancarbonyl]-amino}-(3S)-methyl-buttersäure-tert-butylester und Allylbromid das Diastereomerengemisch 2-[Allyl-((1R,3S)/(1S,3R)-3-hydroxy-cyclohexancarbonyl)-amino]-3-methyl-buttersäure-tert-butyl ester erhalten. 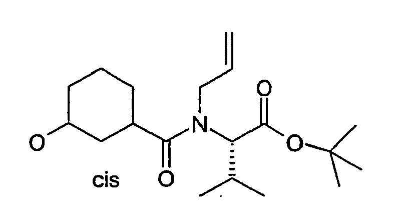 C19H33NO4 (339,48), MS(ESI): 340( M + H⁺).

### 2-[((1R,3S)/(1S,3R)-3-Hydroxy-cyclohexancarbonyl)-propyl-amino]-3-methyl-buttersäure-tert-butyl ester

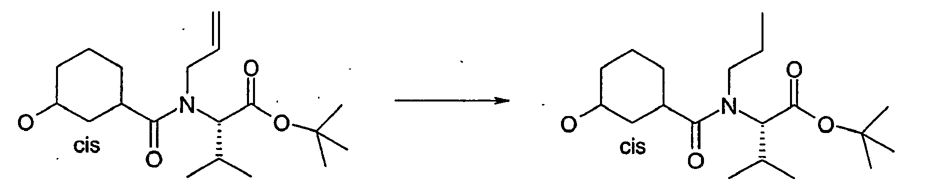

1 g des Diastereomerengemisches 2-[Allyl-((1R,3S)/(1S,3R)-3-hydroxy-cyclohexancarbonyl)-amino]-3-methyl-buttersäure-tert-butylester werden in 30 ml Methanol gelöst und mit 100 mg Palladium (10% auf Kohle) versetzt. Es wird 3h unter einer Wasserstoffatmosphäre bei 5 bar gerührt. Anschließend wird über Celite filtriert und das Lösungsmittel imVakuum entfernt. Man erhält 1g 2-[((1R,3S)/(1S,3R)-3-Hydroxy-cyclohexancarbonyl)-propyl-amino]-3-methyl-buttersäure-tert-butylester als farbloses Öl. C19H35NO4 (341,50), MS(ESI): 342( M + H⁺).

Analog zu Beispiel 28 wurde aus dem Diastereomerengemisch 2-[((1R,3S)/(1S,3R)-3-Hydroxy-cyclohexancarbonyl)-propyl-amino]-3-methyl-buttersäure-tert-butylester und 4-Iodmethyl-5-methyl-2-p-tolyl-oxazol das Diastereomerengemisch (S)-3-Methyl-2-{[(1R,3S)/(1R,3S)-3-(5-methyl-2-p-tolyl-oxazol-4-ylmethoxy)-cyclohexancarbonyl]-propyl-amino}-buttersäure erhalten. 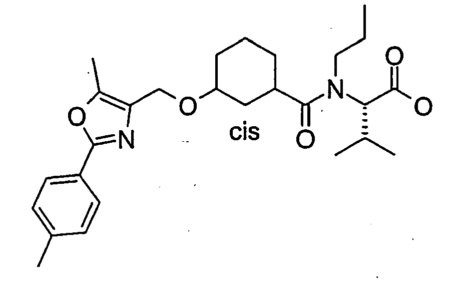 C27H38N2O5 (470.61), MS(ESI): 342( M + H⁺).

### Beispiel 31

### 1-{[(1R,3S)/(1S,3R)-3-(5-Ethyl-2-p-tolyl-oxazol-4-ylmethoxy)-cyclohexancarbonyl]-amino}-cyclopentancarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 4-Iodmethyl-5-ethyl-2-p-tolyl-oxazol das Racemat 1-{[(1R,3S)/(1S,3R)-3-(5-Ethyl-2-p-tolyl-oxazol-4-ylmethoxy)-cyclohexancarbonyl]-amino}-cyclopentancarbonsäure erhalten. 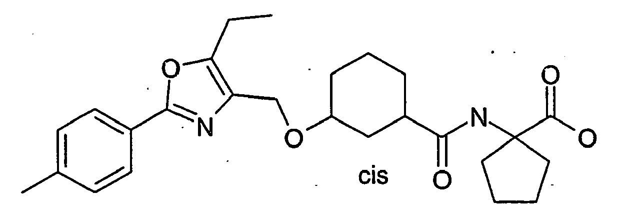 C26H34N2O5 (454.57), MS(ESI): 455( M + H⁺).

### Beispiel 32

### 1-{[(1R,3S)/(1S,3R)-3-[2-(3,5-Dimethoxy-phenyl)-5-methyl-oxazol-4-ylmethoxy]-cyclohexanecarbonyl}-amino)- cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 2-(3,5-Dimethoxy-phenyl)-4-iodomethyl-5-methyl-oxazol das Racemat 1-{[(1R,3S)/(1S,3R)-3-[2-(3,5-Dimethoxy-phenyl)-5-methyl-oxazol-4-ylmethoxy]-cyclohexanecarbonyl}-amino)- cyclopentanecarbonsäure erhalten. 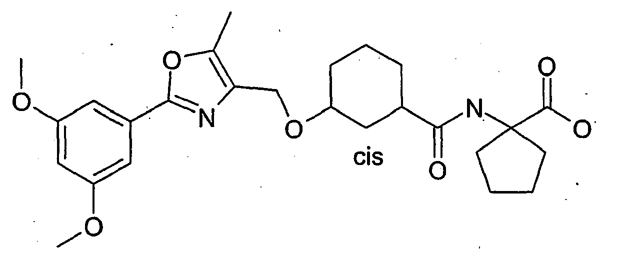 C26H34N2O7 (486.57), MS(ESI): 487( M + H⁺).

### Beispiel 33

### 1-{[(1R,3S)/(1S,3R)-({3-[5-Isopropyl-2-(3-trifluoromethyl-phenyl)-oxazol-4-ylmethoxy]-cyclohexanecarbonyl}- amino)-cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 4-Iodomethyl-5-isopropyl-2-(3-trifluoromethyl-phenyl)-oxazol das Racemat 1-{[(1R,3S)/(1S,3R)-({3-[5-Isopropyl-2-(3-trifluoromethyl-phenyl)-oxazol-4-ylmethoxy]-cyclohexanecarbonyl}- amino)-cyclopentanecarbonsäure erhalten. 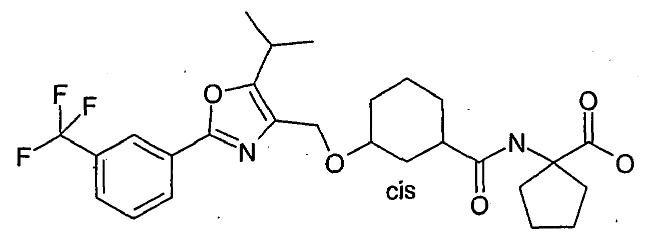 C27H33F3N2O5 (522.57), MS(ESI): 523(M + H⁺).

### Beispiel 34

### 1-{[(1R,3S)/(1S,3R)-({3-[5-Ethyl-2-(3-trifluoromethyl-phenyl)-oxazol-4-ylmethoxy]-cyclohexanecarbonyl}-amino)-cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 5-Ethyl-4-iodomethyl-2-(3-trifluoromethyl-phenyl)-oxazol das Racemat 1-{[(1R,3S)/(1S,3R)-({3-[5-Ethyl-2-(3-trifluoromethyl-phenyl)-oxazol-4-ylmethoxy]-cyclohexanecarbonyl}-amino)-cyclopentanecarbonsäure erhalten. 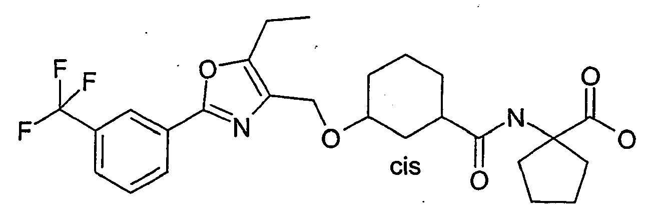 C26H31 F3N2O5 (508.54), MS(ESI): 509( M + H⁺).

### Beispiel 35

### 1-{(1R,3S)/(1S,3R)-({3-[2-(3,4-Dimethyl-phenyl)-5-isopropyl-oxazol-4-ylmethoxy]-cyclohexanecarbonyl}-amino)-cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 2-(3,4-Dimethyl-phenyl)-4-iodomethyl-5-isopropyl-oxazol das Racemat 1-{[(1R,3S)/(1S,3R)-({3-[2-(3,4-Dimethyl-phenyl)-5-isopropyl-oxazol-4-ylmethoxy]-cyclohexanecarbonyl}-amino)-cyclopentanecarbonsäure erhalten. 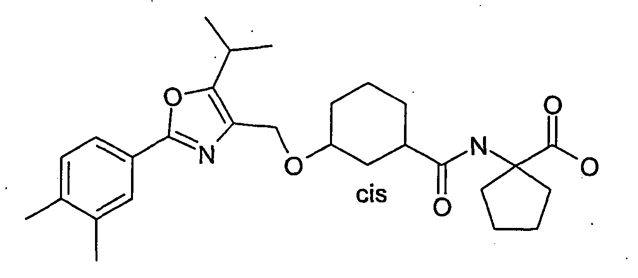 C28H38N2O5 (482.63), MS(ESI): 483( M + H⁺).

### Beispiel 36

### 1-{[(1R,3S)/(1S,3R)-({3-[2-(4-Methoxy-phenyl)-5-methyl-oxazol-4-ylmethoxy]-cyclohexanecarbonyl}-amino)- cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 4-Iodomethyl-2-(4-methoxy-phenyl)-5-methyl-oxazol das Racemat 1-{[(1R,3S)/(1S,3R)-({3-[2-(4-Methoxy-phenyl)-5-methyl-oxazol-4-ylmethoxy]-cyclohexanecarbonyl}-amino)- cyclopentanecarbonsäure erhalten. 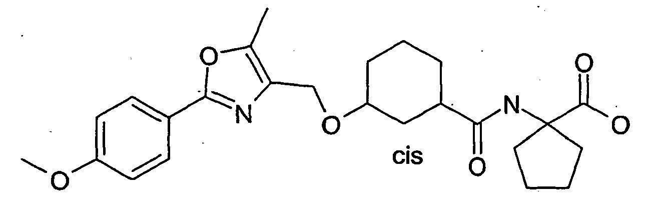 C25H32N2O6 (456.54), MS(ESI): 457( M + H⁺).

### Beispiel 37

### 1-{[(1R,3S)/(1S,3R)-{[3-(5-Ethyl-2-naphthalen-2-yl-oxazol-4-ylmethoxy)-cyclohexanecarbonyl]-amino}- cyclopentanecarbonsäure

Analog zu Beispiel 24 wurde aus (1R,3S)/( 1S,3R)-3-(tert-Butyl-diphenyl-silanyloxy)-cyclohexancarbonsäure, 1-Amino-cyclopentancarbonsäuremethylesterhydrochlorid und 5-Ethyl-4-iodomethyl-2-naphthalen-2-yl-oxazol das Racemat 1-{[(1R,3S)/(1S,3R)-{[3-(5-Ethyl-2-naphthalen-2-yl-oxazol-4-ylmethoxy)-cyclohexanecarbonyl]-amino}-cyclopentanecarbonsäure erhalten. 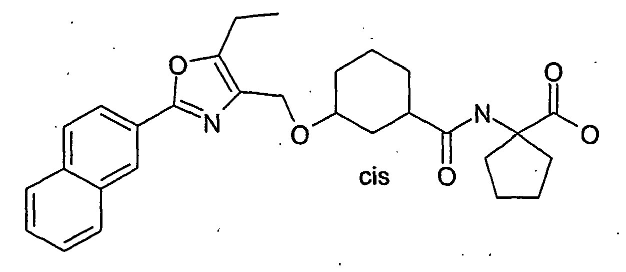 C29H34N2O5 (490.60), MS(ESI): 491 ( M + H⁺).

### Beispiel 38

### 1-{[(1R,3S)/(1S,3R)-({3-[5-Ethyl-2-(3-methoxy-phenyl)-oxazol-4-ylmethoxy]-cyclohexanecarbonyl}-amino)- cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 5-Ethyl-4-iodomethyl-2-(3-methoxy-phenyl)-oxazol das Racemat 1-{[(1R,3S)/(1S,3R)-({3-[5-Ethyl-2-(3-methoxy-phenyl)-oxazol-4-ylmethoxy]-cyclohexanecarbonyl}-amino)- cyclopentanecarbonsäure erhalten. 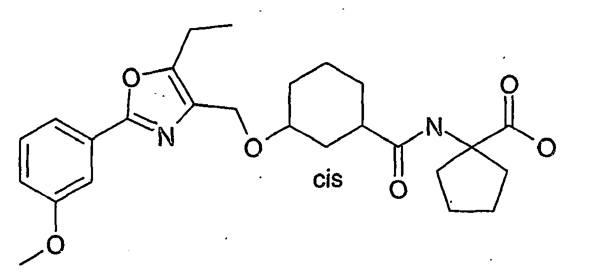 C26H34N2O6 (470.57), MS(ESI): 471 ( M + H⁺).

### Beispiel 39

### 1-{[(1R,3S)/(1S,3R)-({3-[2-(4-Isopropyl-phenyl)-5-methyl-oxazol-4-ylmethoxy]-cyclohexanecarbonyl}-amino)- cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 4-Iodomethyl-2-(4-isopropyl-phenyl)-5-methyl-oxazol das Racemat 1-{[(1R,3S)/(1S,3R)-({3-[2-(4-Isopropyl-phenyl)-5-methyl-oxazol-4-ylmethoxy]-cyclohexanecarbonyl}-amino)- cyclopentanecarbonsäure erhalten. 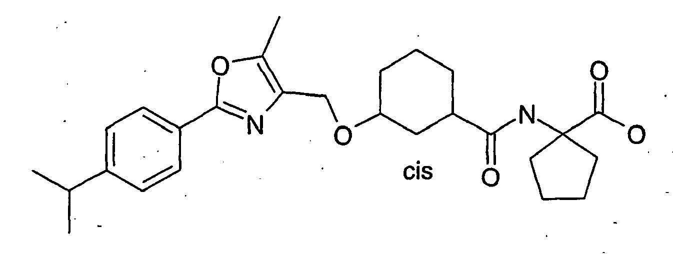 C27H36N2O5 (468.60), MS(ESI): 469( M + H⁺).

### Beispiel 40

### 1-{[(1R,3S)/(1S,3R)-({3-[5-Ethyl-2-(4-isopropyl-phenyl)-oxazol-4-ylmethoxy]-cyclohexanecarbonyl}-amino)- cyclopentanecarbonsäure

Analog zu Beispiel 24 wurde aus (1R,3S)/(1S,3R)-3-(tert-Butyl-diphenyl-silanyloxy)-cyclohexancarbonsäure, 1-Amino-cyclopentancarbonsäuremethylesterhydrochlorid und 5-Ethyl-4-iodomethyl-2-(4-isopropyl-phenyl)-oxazol das Racemat 1-{[(1R,3S)/(1S,3R)-({3-[5-Ethyl-2-(4-isopropyl-phenyl)-oxazol-4-ylmethoxy]-cyclohexanecarbonyl}-amino)- cyclopentanecarbonsäure erhalten. 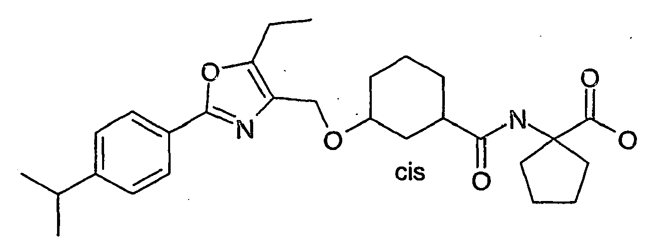 C28H38N2O5 (482.63), MS(ESI): 483( M + H⁺).

### Beispiel 41

### (S)-(3-Methyl-2-{[(1R,3S)/(1S,3R)-3-(5-methyl-2-p-tolyl-oxazol-4-ylmethoxymethyl)-cyclohexan carbonyl]- amino}-butyrsäure

### (S)-(1 R,3S)/(1S,3R)-3-(1-tert-Butoxycarbonyl-2-methyl-propylcarbamoyl)-cyclohexanecarbonsäure

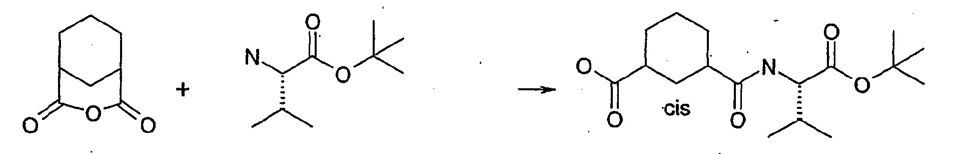

5,0 g 3-Oxa-bicyclo[3.3.1]nonane-2,4-dion und 7,1 g L-Valin-tert.-butylesterhydrochlorid werden bei 0°C in 50 ml Dimethylformamid vorgelegt und mit 4,5 ml Triethylamin und einer Spatelspitze Dimethylaminopyridin versetzt. Man rührt eine Stunde bei Raumtemperatur nach, danach wird das Reaktionsgemisch durch Zugabe von 200 ml Ethylacetat verdünnt und fünfmal mit Wasser, einnormaler Salzsäure und gesättigter Natriumchloridlösung gewaschen. Die organische Phase wird über Magnesiumsulfat getrocknet und anschließend das Lösungsmittel im Vakuum entfernt. Als Rückstand erhält man 7,0 g des gewünschten Diastereomerengemischs (S)-(1R,3S)/(1S,3R)-3-(1-tert-Butoxycarbonyt-2-methyl-propylcarbamoyl)-cyclohexanecarbonsäure als farbloses Öl.
C17H29NO5 (327.42), MS(ESI): 272 (M-tert-Butyl+2H⁺).

### (S)-(2-[((1R,3S)/(1S,3R)-3-Hydroxymethyl-cyclohexanecarbonyl)-amino]-3-methyl-butyrsäure- tert-butylester

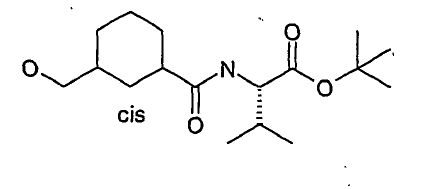

7,0 g (S)-(1R,3S)/(1S,3R)-3-(1-tert-Butoxycarbonyl-2-methyl-propylcarbamoyl)-cyclohexanecarbonsäure werden in 200 ml Tetrahydrofuran gelöst und mit 4,05 ml Triethylamin versetzt. Man rührt eine Stunde bei Raumtemperatur nach, dann wird das Reaktionsgemisch auf -78°C abgekühlt und mit 3,58 ml Isobutylchloroformat versetzt. Das Reaktionsgemisch wird über Nacht auf Raumtemperatur erwärmt, über Celite filtriert und mit wenig kaltem Tetrahydrofuran nachgewaschen. Unter Eiskühlung werden 2,75 g Natriumborhydrid zum Filtrat gegeben , gefolgt von einer tropfenweise Zugabe von 10 ml Wasser. Nach zweistündigem Rühren bei Raumtemperatur wird das Reaktionsgemisch über Celite filtriert und das Filtrat im Vakuum eingeengt. Der Rückstand wird dreimal mit jeweils 150 ml Ethylacetat extrahiert, die vereinigten organischen Extrakte werden über Magnesiumsulfat getrocknet und anschließend das Lösungsmittel im Vakuum entfernt. Als Rückstand erhält man 6,7 g des gewünschten Diastereomerengemischs (S)-(2-[((1R,3S)/(1 S,3R)-3-Hydroxymethyl-cyclohexanecarbonyl)-amino]-3-methyl-butyrsäure- tert-butylester als farbloses Öl. C17H31 NO4 (313.44), MS(ESI): 258 (M-tert-Butyl+2H⁺).

### (S)-(3-Methyl-2-{[(1R,3S)/( 1S,3R)-3-(5-methyl-2-p-tolyl-oxazol-4-ylmethoxymethyl)-cyclohexan carbonyl]- amino}-butyrsäure-tert-butylester

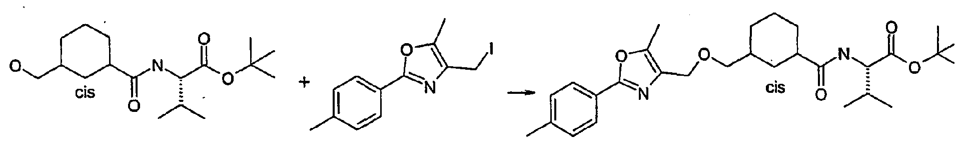

200 mg (S)-(2-[((1R,3S)/( 1S,3R)-3-Hydroxymethyl-cyclohexanecarbonyl)-amino]-3-methyl-butyrsäure- tert-butylester und 260 mg 4-lodomethyl-5-methyl-2-p-tolyl-oxazol werden in 30 ml Chlorbenzol gelöst und mit 86 mg Kalium-tert.-butylat versetzt. Man rührt eine Stunde bei Raumtemperatur nach, danach werden 100 ml Ethylacetat zugefügt und das Reaktionsgemisch mit 40 ml einer einmolaren Salzsäurelösung und dreimal mit jeweils 50 ml gesättigter Natriumchloridlösung gewaschen. Die organische Phase wird über Magnesiumsulfat getrocknet und anschließend das Lösungsmittel im Vakuum entfernt. Der Rückstand wird mittels HPLC gereinigt. Nach Gefriertrocknung erhält man 150 mg des gewünschten Diastereomerengemischs (S)-(3-Methyl-2-{[(1R,3S)/(1S,3R)-3-(5-methyl-2-p-tolyl-oxazol-4-ylmethoxymethyl)-cyclohexancarbonyl]-amino}-butyrsäure-tert-butylester als gelbes Öl.
C29H42N2O5 (498.67), MS(ESI): 499 (M+H⁺).

### (S)-(3-Methyl-2-{[(1R,3S)/(1S,3R)-3-(5-methyl-2-p-tolyl-oxazol-4-ylmethoxymethyl)-cyclohexan carbonyl]- amino}-butyrsäure

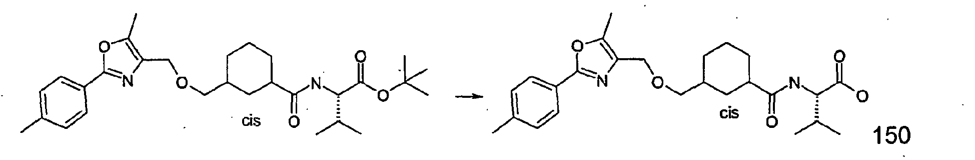

mg (S)-(3-Methyl-2-{[(1R,3S)/( 1 S,3R)-3-(5-methyl-2-p-tolyl-oxazol-4-ylmethoxymethyl)-cyclohexan carbonyl]- amino}-butyrsäure-tert-butylester werden in 10 ml Dichlormethan gelöst und mit 2 ml Trifluoressigsäure versetzt. Nach zweistündigem Rühren bei Raumtemperatur werden 50 ml Toluol zugefügt und die Lösungsmittel im Vakuum entfernt. Der resultierende Rückstand wird in Wasser aufgenommen und lyophilisiert. Man erhält 130 mg des gewünschten Diastereomerengemischs (S)-(3-Methyl-2-{[(1R,3S)/(1S,3R)-3-(5-methyl-2-p-tolyl-oxazol-4-ylmethoxymethyl)-cyclohexan carbonyl]- amino}-butyrsäure als weißen Feststoff.
C25H34N2O5 (442.56), MS(ESI): 443 (M+H⁺).

### Beispiel 42

### (S)-(2-({(1R,3S)/(1S,3R)-3-[2-(3,4-Dimethyl-phenyl)-5-ethyl-oxazol-4-ylmethoxy methyl]-cyclohexan carbonyl}- amino)-3-methyl-butyrsäure

Analog zu Beispiel 41 wurde aus 3-Oxa-bicyclo[3.3.1]nonane-2,4-dion, L-Valin-tert.-butylesterhydrochlorid, und 2-(3,4-Dimethyl-phenyl)-5-ethyl-4-iodomethyl-oxazol das Diastereomerengemisch (S)-(2-({(1R,3S)/( 1 S,3R)-3-[2-(3,4-Dimethyl-phenyl)-5-ethyl-oxazol-4-ylmethoxy methyl]-cyclohexan carbonyl}- amino)-3-methyl-butyrsäure hergestellt. 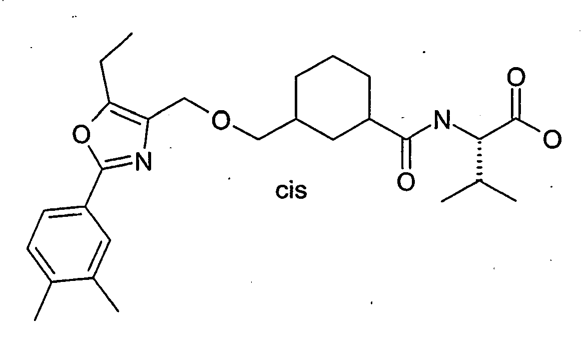 C27H38N2O5 (470.61), MS(ESI): 471 (M+H⁺).

### Beispiel 43

(S)-(2-({1,3-(1R,3S)/(1S,3R)-3-[5-Isopropyl-2-(4-trifluoromethyl-phenyl)-oxazol-4-ylmethoxymethyl]- cyclohexanecarbonyl}-amino)-3-methyl-butyrsäure Analog zu Beispiel 41 wurde aus 3-Oxa-bicyclo[3.3.1]nonane-2,4-dion, L-Valin-tert.-butylesterhydrochlorid, und 4-Iodomethyl-5-isopropyl-2-(4-trifluoromethyl-phenyl)-oxazol das Diastereomerengemisch (S)-(2-({1,3-(1R,3S)/(1S,3R)-3-[5-Isopropyl-2-(4-trifluoromethyl-phenyl)-oxazol-4-ylmethoxymethyl]-cyclohexanecarbonyl}-amino)-3-methyl-butyrsäure hergestellt. 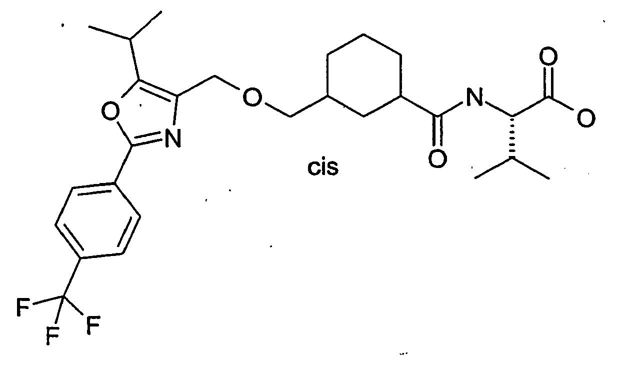 C27H35F3N2O5 (524.59), MS(ESI): 525 (M+H⁺).

### Beispiel 44

### (S)-(3-Methyl-2-({(1R,3S)/( 1 S,3R)-3-[5-methyl-2-(3-trifluoromethyl-phenyl)-oxazol-4-ylmethoxymethyl]- cyclohexanecarbonyl}-amino)-butyrsäure

Analog zu Beispiel 41 wurde aus 3-Oxa-bicyclo[3.3.1]nonane-2,4-dion, L-Valin-tert.-butylesterhydrochlorid, und 4-Iodomethyl-5-methyl-2-(3-trifluoromethyl-phenyl)-oxazol das Diastereomerengemisch ((S)-(3-Methyl-2-({(1R,3S)/( 1S,3R)-3-[5-methyl-2-(3-trifluoromethyl-phenyl)-oxazol-4-ylmethoxymethyl]- cyclohexanecarbonyl}-amino)-butyrsäure hergestellt. 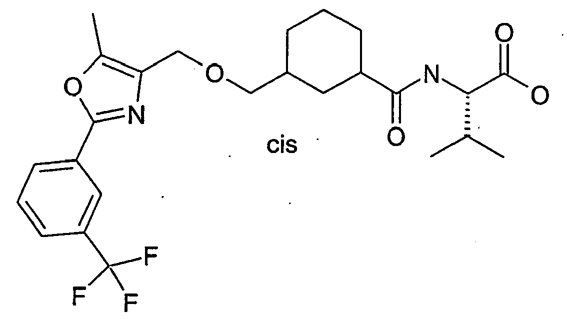 C25H31 F3N2O5 (496.53), MS(ESI): 497 (M+H⁺).

### Beispiel 45

### (S)-2-({ (1R,3S)/( 1 S,3R)- 3-[5-Ethyl-2-(4-isopropyl-phenyl)-oxazol-4-ylmethoxymethyl]-cyclohexanecarbonyl}- amino)-3-methyl-butyrsäure

Analog zu Beispiel 41 wurde aus 3-Oxa-bicyclo[3.3.1]nonane-2,4-dion, L-Valin-tert.-butylesterhydrochlorid, und 5-Ethyl-4-iodomethyl-2-(4-isopropyl-phenyl)-oxazol das Diastereomerengemisch (S)-2-({ (1R,3S)/( 1S,3R)- 3-[5-Ethyl-2-(4-isopropyl-phenyl)-oxazol-4-ylmethoxymethyl]-cyclohexanecarbonyl}- amino)-3-methyl-butyrsäure hergestellt. 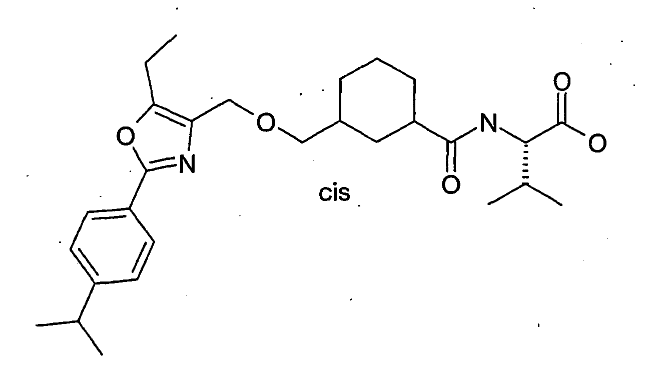 C28H40N2O5 (484.64), MS(ESI): 485 (M+H⁺).

### Beispiel 46

### (S)-(2-{[(1R,3S)/( 1S,3R)-3-(5-Ethyl-2-p-tolyl-oxazol-4-ylmethoxymethyl)-cyclohexan carbonyl]-amino}-3-methyl- butyrsäure

Analog zu Beispiel 41 wurde aus 3-Oxa-bicyclo[3.3.1]nonane-2,4-dion, L-Valin-tert.-butylesterhydrochlorid, und 5-Ethyl-4-iodomethyl-2-p-tolyl-oxazol das Diastereomerengemisch (S)-(2-{[(1R,3S)/( 1 S,3R)-3-(5-Ethyl-2-p-tolyl-oxazol-4-yfmethoxymethyl)-clyclohexan carbonyl]-amino}-3-methyl- butyrsäure hergestellt. 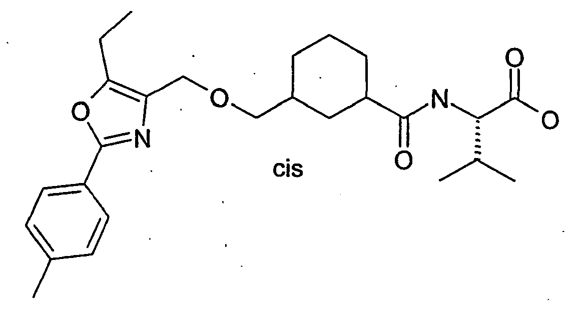 C26H36N2O5 (456.59), MS(ESI): 457 (M+H⁺).

### Beispiel 47

### (S)-(2-({(1R,3S)/( 1 S,3R)-3-[5-Cyclohexyl-2-(3-methoxy-phenyl)-oxazol-4-ylmethoxy methyl]-cyclohexan carbonyl}- amino)-3-methyl-butyrsäure

Analog zu Beispiel 41 wurde aus 3-Oxa-bicyclo[3.3.1]nonane-2,4-dion, L-Valin-tert.-butylesterhydrochlorid, und 5-Cyclohexyl-4-iodomethyl-2-(3-methoxy-phenyl)- oxazol das Diastereomerengemisch (S)-(2-({(1R,3S)/( 1S,3R)-3-[5-Cyclohexyl-2-(3-methoxyphenyl)-oxazol-4-ylmethoxy methyl]-cyclohexan carbonyl}- amino)-3-methyl-butyrsäure hergestellt. 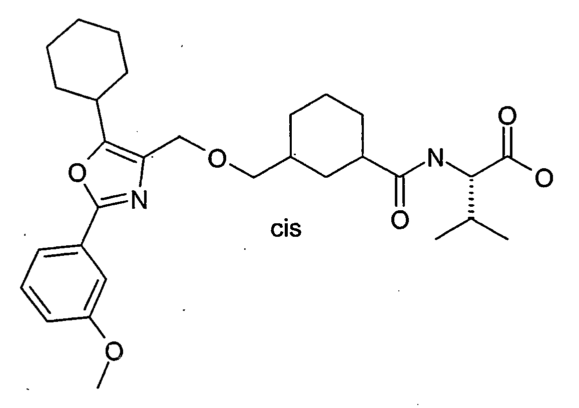 C30H42N2O6 (526.68), MS(ESI): 527 (M+H⁺).

### Beispiel 48

### (S)-(2-({(1R,3S)/( 1S,3R)-3-[2-(3-Methoxy-phenyl)-5-methyl-oxazol-4-yl methoxy methyl]-cyclohexan carbonyl}- amino)-3-methyl-butyrsäure

Analog zu Beispiel 41 wurde aus 3-Oxa-bicyclo[3.3.1]nonane-2,4-dion, L-Valin-tert.-butylesterhydrochlorid, und 4-lodomethyl-2-(3-methoxy-phenyl)-5-methyl-oxazol das Diastereomerengemisch (S)-(2-({(1R,3S)/( 1 S,3R)-3-[2-(3-Methoxy-phenyl)-5-methyl-oxazol-4-ylmethoxymethyl]-cyclohexan carbonyl}- amino)-3-methyl-butyrsäure hergestellt. 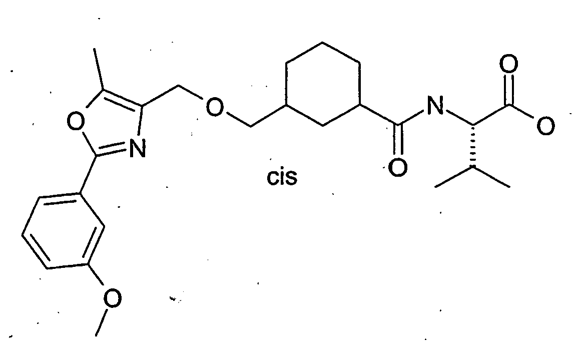 C25H34N2O6 (458.56), MS(ESI): 459 (M+H⁺).

### Beispiel 49

### (S)-(2-({3-[2-(4-Isopropyl-phenyl)-5-methyl-oxazol-4-ylmethoxy methyl]-cyclohexan carbonyl}- amino)-3-methyl-butyrsäure

Analog zu Beispiel 41 wurde aus 3-Oxa-bicyclo[3.3.1]nonane-2,4-dion, L-Valin-tert.-butylesterhydrochlorid, und 4-Iodomethyl-2-(4-isopropyl-phenyl)-5-methyl-oxazol das Diastereomerengemisch (S)-(2-({3-[2-(4-Isopropyl-phenyl)-5-methyl-oxazol-4-ylmethoxy methyl]-cyclohexan carbonyl}- amino)-3-methyl-butyrsäure hergestellt. 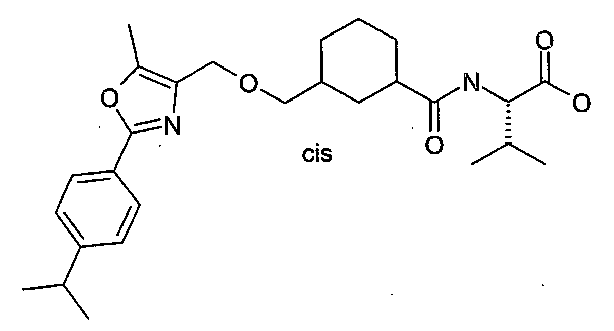 C27H38N2O5 (470.61), MS(ESI): 471 (M+H⁺).

### Beispiel 50

### (S)-(2-({(1R,3S)/( 1 S,3R)-3-[2-(4-Trifluormethyl-phenyl)-5-ethyl-oxazol-4-ylmethoxy methyl]-cyclohexan carbonyl}- amino)-3-methyl-butyrsäure

Analog zu Beispiel 41 wurde aus 3-Oxa-bicyclo[3.3.1]nonane-2,4-dion, L-Valin-tert.-butylesterhydrochlorid, und 5-Ethyl-4-iodomethyl-2-(4-trifluoromethyl-phenyl)-oxazol das Diastereomerengemisch (S)-(2-({(1R,3S)/(1S,3R)-3-[2-(4-Trifluormethyl-phenyl)-5-ethyl-oxazol-4-ylmethoxy methyl]-cyclohexan carbonyl}- amino)-3-methyl-butyrsäure hergestellt. 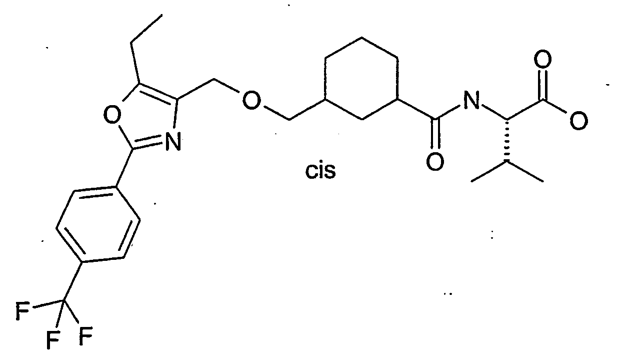 C26H33F3N2O5 (510.56), MS(ESI): 511 (M+H⁺).

## Patentansprüche

1. Verbindungen der Formel I 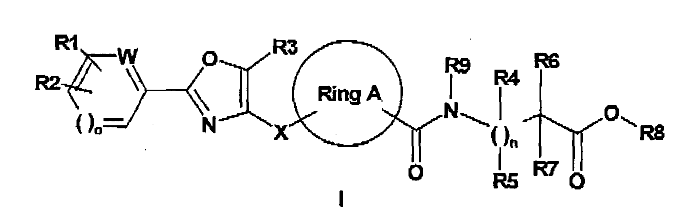 worin bedeuten:
Ring A Cyclohexan-1,3-diyl;
R1, R2 unabhängig voneinander H, F, Cl, Br, CF₃, OCF₃, (C1-C6)-Alkyl, O-(C1- C6)-Alkyl, SCF₃, SF₅, OCF₂-CHF₂, (C6-C10)-Aryl, (C6-C10)-Aryloxy, OH, NO₂; oder
R1 und R2 zusammen genommen mit dem Phenyl-, Pyridin-, 1-H-Pyrrol-, Thiophen- oder Furan-Ring kondensiertes, teilweise oder nicht gesättigtes bicyclisches (C6-C10)-Aryl, (C5-C11)-Heteroaryl;
R3 H, (C1-C6)-Alkyl, (C3-C8)-Cycloalkyl, (C1-C3)-Alkyl-(C3-C8)-Cycloalkyl, Phenyl, (C1-C3)-Alkyl-Phenyl, (C5-C6)-Heteroaryl, (C1-C3)-Alkyl-(C5- C6)-Heteroaryl oder (C1-C3)-Alkyl, das durch F ganz oder teilweise substituiert ist;
o 0 oder 1;
W CH, N, falls o = 1;
w O, S, NR10, falls o = 0;
X (C1-C6)-Alkandiyl, wobei in der Alkandiylgruppe ein oder mehrere Kohlenstoffatome durch Sauerstoffatome ersetzt sein können;
n 0 - 2;
R4 H, (C1-C6)-Alkyl;
R5 H, (C1-C6)-Alkyl;
R6 H, (C1-C6)-Alkyl, F;
R7 H, F, (C1-C6)-Alkyl, (C1-C6)-Alkoxy, (C2-C6)-Alkenyl, (C2-C6)-Alkinyl, (C3-C8)-Cycloalkyl, Phenyl, wobei Alkyl gegebenenfalls substituiert sein kann durch ein oder mehrere Reste aus der Reihe Hydroxy, Phenyl, (C5- C11)-Heteroaryl, (C1-C6)-Alkoxy und NR11R12, und Phenyl gegebenfalls substituiert sein kann durch ein oder mehrere Reste aus der Reihe Hydroxy, (C1-C6)-Alkoxy, F und CF₃; mit der Maßgabe, dass R7 ungleich NR11R12 oder (C1-C6)-Alkoxy, falls R6 = F ist;
R7 und R9 zusammen mit den sie tragenden Atomen Pyrrolidin oder Piperidin, falls n = 0;
R6 und R7 zusammen mit dem sie tragenden C-Atom (C3-C8)-Cycloalkyl;
R8 H, (C1-C6)-Alkyl;
R9 H, (C1-C6)-Alkyl, (C2-C6)-Alkenyl, (C2-C6)-Alkinyl, (C1-C4)-Alkyl-(C6- C10)-Aryl, (C1-C4)-Alkyl-(C5-C11)-Heteroaryl, (C1-C4)-Alkyl-O-(C1-C4)- Alkyl, Phenyl-(C1-C4)-Alkyl, (C5-C6)-Heteroaryl-(C1-C4)-Alkyl;
R10 H, (C1-C6)-Alkyl-Phenyl, (C1-C6)-Alkyl;
R11 H, (C1-C6)-Alkyl-Phenyl, (C1-C6)-Alkyl;
R12 H, (C1-C6)-Alkyl-Phenyl, (C1-C6)-Alkyl;
sowie deren physiologisch verträgliche Salze.

2. Verbindungen der Formel I gemäß Anspruch 1, worin bedeuten
Ring A Cyclohexan-1,3-diyl;
X (C1-C6)-Alkandiyl, wobei in der Alkandiylgruppe das C1- oder C2- Kohlenstoffatom (zum Ring A) durch Sauerstoffatom ersetzt ist.

3. Verbindungen der Formel I gemäß einem oder mehren der Ansprüche 1 bis 2, worin bedeuten
Ring A cis-Cyclohexan-1,3-diyl;
R1 Br, CF₃, OCF₃, (C1-C6)-Alkyl, O-(C1-C6)-Alkyl;
R2 H, (C1-C6)-Alkyl, O-(C1-C6)-Alkyl oder
R1 und R2 zusammen mit dem Phenylring = Naphthyl;
R3 CF₃, (C1-C6)-Alkyl, (C3-C6)-Cycloalkyl, Phenyl;
W CH, falls o = 1;
X CH₂O, CH₂-O-CH₂;
n 0
R6 H, (C1-C6)-Alkyl;
R7 (C1-C6)-Alkyl, wobei Alkyl gegebenenfalls substituiert sein kann durch Phenyl;
R7 und R9 zusammen mit den sie tragenden Atomen Pyrrolidin, falls n = 0;
R6 und R7 zusammen mit dem sie tragenden C-Atom (C3-C6)-Cycloalkyl;
R8 H,
R9 H, (C1-C6)-Alkyl, Benzyl;
sowie deren physiologisch verträgliche Salze.

4. Arzneimittel enthaltend eine oder mehrere der Verbindungen der Formel I gemäß einem oder mehreren der Ansprüche 1 bis 3.

5. Arzneimittel enthaltend eine oder mehrere der Verbindungen der Formel I gemäß einem oder mehreren der Ansprüche 1 bis 3 und einen oder mehrere Wirkstoffe, die günstige Wirkungen auf Stoffwechselstörungen oder damit assozierte Erkrankungen haben.

6. Arzneimittel enthaltend eine oder mehrere der Verbindungen der Formel I gemäß einem oder mehreren der Ansprüche 1 bis 3 und einen oder mehrere Antidiabetika.

7. Arzneimittel enthaltend eine oder mehrere der Verbindungen der Formel I gemäß einem oder mehreren der Ansprüche 1 bis 3 und einen oder mehrere Lipidmodulatoren.

8. Verwendung der Verbindungen der Formel I gemäß einem oder mehreren der Ansprüche 1 bis 3 zur Herstellung eines Arzneimittels zur Behandlung und/oder Prävention von Störungen des Fettsäurestoffwechsels und Glucoseverwertungsstörungen.

9. Verwendung der Verbindungen der Formel I gemäß einem oder mehreren der Ansprüche 1 bis 3 zur Herstellung eines Arzneimittels zur Behandlung und/oder Prävention von Störungen, bei denen Insulin Resistenz eine Rolle spielt.

10. Verwendung der Verbindungen der Formel I gemäß einem oder mehreren der Ansprüche 1 bis 3 zur Herstellung eines Arzneimittels zur Behandlung und/oder Prävention von Diabetes mellitus und der damit verbundenen Folgeerkrankungen.

11. Verwendung der Verbindungen der Formel I gemäß einem oder mehreren der Ansprüche 1 bis 3 zur Herstellung eines Arzneimittels zur Behandlung und/oder Prävention von Dyslipidämien und deren Folgen.

12. Verwendung der Verbindungen der Formel I gemäß einem oder mehreren der Ansprüche 1 bis 3 zur Herstellung eines Arzneimittels zur Behandlung und/oder Prävention von Zuständen, die mit dem Metabolischen Syndrom assoziiert sind.

13. Verwendung der Verbindungen gemäß einem oder mehreren der Ansprüche 1 bis 3 in Kombination mit mindestens einem weiteren Wirkstoff zur Herstellung eines Arzneimittels zur Behandlung und/oder Prävention von Störungen des Fettsäurestoffwechsels und Glucoseverwertungsstörungen.

14. Verwendung der Verbindungen gemäß einem oder mehreren der Ansprüche 1 bis 3 in Kombination mit mindestens einem weiteren Wirkstoff zur Herstellung eines Arzneimittels zur Behandlung und /oder Prävention von Störungen, bei denen Insulin Resistenz eine Rolle spielt.

15. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 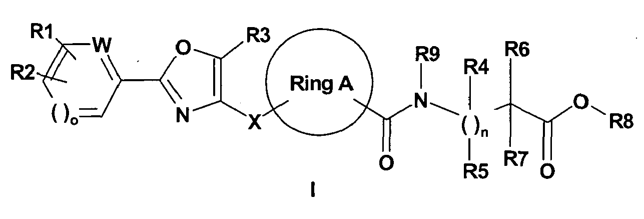 in which:
Ring A is cyclohexane-1,3-diyl;
R1, R2 independently of one another are H, F, Cl, Br, CF₃, OCF₃, (C1-C6)-alkyl, O-(C1-C6)-alkyl, SCF₃, SF₅, OCF₂-CHF₂, (C6-C10)-aryl, (C6-C10)- aryloxy, OH, NO₂; or
R1 and R2 together with the phenyl, pyridine, 1H-pyrrole, thiophene or furan ring form fused, partially or unsaturated bicyclic (C6-C10)-aryl, (C5-C11)-heteroaryl;
R3 is H, (C1-C6)-alkyl, (C3-C8)-cycloalkyl, (C1- C3)-alkyl-(C3-C8)-cycloalkyl, phenyl, (C1- C3)-alkyl-phenyl, (C5-C6)-heteroaryl, (C1- C3)-alkyl-(C5-C6)-heteroaryl or (C1-C3)-alkyl which is fully or partially substituted by F;
O is 0 or 1;
W is CH or N if o = 1;
W is O, S or NR10 if o = 0;
X is (C1-C6)-alkanediyl, where in the alkanediyl group one or more carbon atoms may be replaced by oxygen atoms;
n is 0-2;
R4 is H or (C1-C6)-alkyl;
R5 is H or (C1-C6)-alkyl;
R6 is H, (C1-C6)-alkyl or F;
R7 is H, F, (C1-C6)-alkyl, (C1-C6)-alkoxy, (C2- C6)-alkenyl, (C2-C6)-alkynyl, (C3-C8)- cycloalkyl, phenyl, where alkyl may be unsubstituted or substituted by one or more radicals selected from the group consisting of hydroxyl, phenyl, (C5-C11)-heteroaryl, (C1-C6)-alkoxy and NR11R12, and phenyl may be unsubstituted or substituted by one or more radicals from the group consisting of hydroxyl, (C1-C6)-alkoxy, F and CF₃; with the proviso that R7 is not NR11R12 or (C1-C6)- alkoxy if R6 = F;
R7 and R9 together with the atoms that carry them are pyrrolidine or piperidine if n = 0;
R6 and R7 together with the carbon atom that carries them are (C3-C8)-cycloalkyl;
R8 is H, (C1-C6)-alkyl;
R9 is H, (C1-C6)-alkyl, (C2-C6)-alkenyl, (C2- C6)-alkynyl, (C1-C4)-alkyl-(C6-C10)-aryl, (C1-C4)-alkyl-(C5-C11)-heteroaryl, (C1-C4)- alkyl-O-(C1-C4)-alkyl, phenyl-(C1-C4)-alkyl, (C5-C6)-heteroaryl-(C1-C4)-alkyl;
R10 is H, (C1-C6)-alkyl-phenyl, (C1-C6)-alkyl;
R11 is H, (C1-C6)-alkyl-phenyl, (C1-C6)-alkyl;
R12 is H, (C1-C6)-alkyl-phenyl, (C1-C6)-alkyl;
and its physiologically acceptable salts.

2. A compound of the formula I as claimed in claim 1 in which
Ring A is cyclohexane-1,3-diyl;
X is (C1-C6)-alkanediyl, where in the alkanediyl group the C1 or C2 carbon atom (to Ring A) is replaced by an oxygen atom.

3. A compound of the formula I as claimed in one or more of claims 1 to 2, in which
Ring A is cis-cyclohexane-1,3-diyl
R1 is Br, CF₃, OCF₃, (C1-C6)-alkyl, O-(C1-C6)- alkyl;
R2 is H, (C1-C6)-alkyl, O-(C1-C6)-alkyl or
R1 and R2 together with the phenyl ring = naphthyl;
R3 is CF₃, (C1-C6)-alkyl, (C3-C8)-cycloalkyl, phenyl;
W is CH if o = 1;
X is CH₂O or CH₂-O-CH₂;
n is 0;
R6 is H or (C1-C6)-alkyl;
R7 is (C1-C6)-alkyl, where alkyl may be unsubstituted or substituted by phenyl;
R7 and R9 together with the atoms that carry them are pyrrolidine if n = 0;
R6 and R7 together with the carbon atom that carries them are (C3-C6)-cycloalkyl;
R8 is H;
R9 is H, (C1-C6)-alkyl or benzyl;
and its physiologically acceptable salts.

4. A pharmaceutical, comprising one or more compounds of the formula I as claimed in one or more of claims 1 to 3.

5. A pharmaceutical, comprising one or more compounds of the formula I as claimed in one or more of claims 1 to 3 and one or more active compounds having favorable effects on metabolic disorders or diseases associated therewith.

6. A pharmaceutical, comprising one or more compounds of the formula I as claimed in one or more of claims 1 to 3 and one or more antidiabetics.

7. A pharmaceutical, comprising one or more compounds of the formula I as claimed in one or more of claims 1 to 3 and one or more lipid modulators.

8. The use of the compounds of the formula I as claimed in one or more of claims 1 to 3 for preparing a pharmaceutical for the treatment and/or prevention of disorders of the fatty acid metabolism and glucose utilization disorders.

9. The use of the compounds of the formula I as claimed in one or more of claims 1 to 3 for preparing a pharmaceutical for the treatment and/or prevention of disorders where insulin resistance is involved.

10. The use of the compounds of the formula I as claimed in one or more of claims 1 to 3 for preparing a pharmaceutical for the treatment and/or prevention of diabetes mellitus and its sequelae.

11. The use of the compounds of the formula I as claimed in one or more of claims 1 to 3 for preparing a pharmaceutical for the treatment and/or prevention of dyslipidemias and their sequelae.

12. The use of the compounds of the formula I as claimed in one or more of claims 1 to 3 for preparing a pharmaceutical for the treatment and/or prevention of states associated with metabolic syndrome.

13. The use of the compounds as claimed in one or more of claims 1 to 3 in combination with at least one further active compound for preparing a pharmaceutical for the treatment and/or prevention of disorders of the fatty acid metabolism and glucose utilization disorders.

14. The use of the compounds as claimed in one or more of claims 1 to 3 in combination with at least one further active compound for preparing a pharmaceutical for the treatment and/or prevention of disorders in which insulin resistance is involved.

15. A process for preparing a pharmaceutical comprising one or more of the compounds as claimed in one or more of claims 1 to 3, which comprises mixing the active compound with a pharmaceutically acceptable carrier and bringing this mixture into a form suitable for administration.

## Revendications

1. Composés de formule I 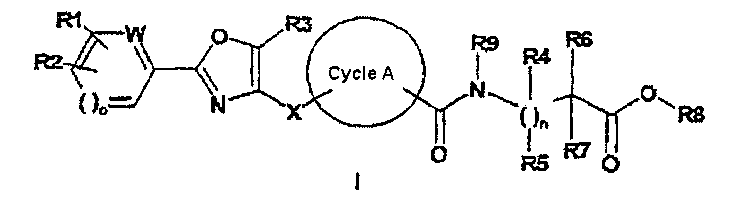 dans laquelle
cycle A signifie cyclohexane-1,3-diyle ;
R1, R2 signifient, indépendamment l'un de l'autre, H, F, Cl, Br, CF₃, OCF₃, (C₁-C₆)-alkyle, O-(C₁-C₆)- alkyle, SCF₃, SF₅, OCF₂-CHF₂, (C₆-C₁₀)-aryle, (C₆- C₁₀)-aryloxy, OH, NO₂ ; ou
R1 et R2 pris ensemble avec le cycle phényle, pyridine, 1-H-pyrrole, thiophène ou furanne, signifient (C₆-C₁₀)-aryle, (C₅-C₁₁) -hétéroaryle bicyclique condensé, partiellement saturé ou non saturé ;
R3 signifie H, (C₁-C₆)-alkyle, (C₃-C₈)-cycloalkyle, (C₁-C₃)-alkyl-(C₃-C₈)-cycloalkyle, phényle, (C₁-C₃)- alkyl-phényle, (C₅-C₆)-hétéroaryle, (C₁-C₃)-alkyl- (C₅-C₆)-hétéroaryle ou (C₁-C₃)-alkyle, qui est totalement ou partiellement substitué par F ;
o vaut 0 ou 1 ;
W signifie CH, N, si o = 1 ;
W signifie O, S, NR10, si o = 0 ;
X signifie (C₁-C₆)-alcanediyle, où dans le groupe alcanediyle, un ou plusieurs atomes de carbone peuvent être remplacés par des atomes d'oxygène ;
n vaut 0-2 ;
R4 signifie H, (C₁-C₆)-alkyle ;
R5 signifie H, (C₁-C₆)-alkyle ;
R6 signifie H, (C₁-C₆)-alkyle, F ;
R7 signifie H, F, (C₁-C₆)-alkyle, (C₁-C₆)-alcoxy, (C₂- C₆)-alcényle, (C₂-C₆)-alcynyle, (C₃-C₈)-cycloalkyle, phényle, où alkyle peut le cas échéant être substitué par un ou plusieurs radicaux de la série hydroxy, phényle, (C₅-C₁₁)-hétéroaryle, (C₁-C₆)- alcoxy et NR11R12, et phényle peut le cas échéant être substitué par un ou plusieurs radicaux de la série hydroxy, (C₁-C₆)-alcoxy, F et CF₃ ; à condition que R7 soit différent de NR11R12 ou de (C₁-C₆)-alcoxy, si R6 = F ;
R7 et R9 signifient, ensemble avec les atomes qui les portent, pyrrolidine ou pipéridine, si n = 0 ;
R6 et R7 signifient, ensemble avec l'atome de C qui les porte, (C₃-C₈)-cycloalkyle ;
R8 signifie H, (C₁-C₆)-alkyle ;
R9 signifie H, (C₁-C₆)-alkyle, (C₂-C₆)-alcényle, (C₂- C₆)-alcynyle, (C₁-C₄)-alkyl-(C₆-C₁₀)-aryle, (C₁-C₄)- alkyl-(C₅-C₁₁)-hétéroaryle, (C₁-C₄)-alkyl-O-(C₁-C₄)- alkyle, phényl-(C₁-C₄)-alkyle, (C₅-C₆)-hétéroaryl- (C₁-C₄)-alkyle ;
R10 signifie H, (C₁-C₆)-alkyl-phényle, (C₁-C₆)alkyle ;
R11 signifie H, (C₁-C₆)-alkyl-phényle, (C₁-C₆)alkyle ;
R12 signifie H, (C₁-C₆)-alkyl-phényle, (C₁-C₆)alkyle ;
ainsi que leurs sels physiologiquement acceptables.

2. Composés de formule I selon la revendication 1, dans laquelle
cycle A signifie cyclohexane-1,3-diyle ;
X signifie (C₁-C₆)-alcanediyle, où dans le groupe alcanediyle, l'atome de carbone C₁ ou C₂ (par rapport au cycle A) est remplacé par un atome d'oxygène.

3. Composés de formule I selon l'une ou plusieurs des revendications 1 à 2, dans laquelle
cycle A signifie cis-cyclohexane-1,3-diyle ;
R1 signifie Br, CF₃, OCF₃, (C₁-C₆)-alkyle, O-(C₁-C₆)- alkyle ;
R2 signifie H, (C₁-C₆)-alkyle, O-(C₁-C₆)-alkyle ou
R1 et R2 ensemble avec le cycle phényle = naphtyle ;
R3 signifie CF₃, (C₁-C₆)-alkyle, (C₃-C₆)-cycloalkyle, phényle ;
W signifie CH, si o = 1 ;
X signifie CH₂O, CH₂-O-CH₂ ;
n = 0
R6 signifie H, (C₁-C₆)-alkyle ;
R7 signifie (C₁-C₆)-alkyle, où alkyle peut le cas échéant être substitué par phényle ;
R7 et R9 signifient, ensemble avec les atomes qui les portent, pyrrolidine, si n = 0 ;
R6 et R7 signifient, ensemble avec l'atome de C qui les porte, (C₃-C₆)-cycloalkyle ;
R8 signifie H,
R9 signifie H, (C₁-C₆)-alkyle, benzyle ;
ainsi que leurs sels physiologiquement acceptables.

4. Médicament contenant un ou plusieurs des composés de formule I selon l'une ou plusieurs des revendications 1 à 3.

5. Médicament contenant un ou plusieurs des composés de formule I selon l'une ou plusieurs des revendications 1 à 3 et une ou plusieurs substances actives, qui ont des effets favorables sur les troubles du métabolisme ou les maladies qui y sont associées.

6. Médicament contenant un ou plusieurs des composés de formule I selon l'une ou plusieurs des revendications 1 à 3 et un ou plusieurs antidiabétiques.

7. Médicament contenant un ou plusieurs des composés de formule I selon l'une ou plusieurs des revendications 1 à 3 et un ou plusieurs modulateurs de lipides.

8. Utilisation des composés de formule I selon l'une ou plusieurs des revendications 1 à 3 pour la préparation d'un médicament destiné au traitement et/ou à la prévention de troubles du métabolisme des acides gras et de troubles de l'utilisation du glucose.

9. Utilisation des composés de formule I selon l'une ou plusieurs des revendications 1 à 3 pour la préparation d'un médicament destiné au traitement et/ou à la prévention de troubles dans lesquels la résistance à l'insuline joue un rôle.

10. Utilisation des composés de formule I selon l'une ou plusieurs des revendications 1 à 3 pour la préparation d'un médicament destiné au traitement et/ou à la prévention du diabète sucré et des maladies secondaires qui y sont liées.

11. Utilisation des composés de formule I selon l'une ou plusieurs des revendications 1 à 3 pour la préparation d'un médicament destiné au traitement et/ou à la prévention de dyslipidémies et de leurs conséquences.

12. Utilisation des composés de formule I selon l'une ou plusieurs des revendications 1 à 3 pour la préparation d'un médicament destiné au traitement et/ou à la prévention d'états qui sont associés au syndrome métabolique.

13. Utilisation des composés selon l'une ou plusieurs des revendications 1 à 3 en combinaison avec au moins une autre substance active pour la préparation d'un médicament destiné au traitement et/ou à la prévention de troubles du métabolisme des acides gras et de troubles de l'utilisation du glucose.

14. Utilisation des composés selon l'une ou plusieurs des revendications 1 à 3 en combinaison avec au moins une autre substance active pour la préparation d'un médicament destiné au traitement et/ou à la prévention de troubles dans lesquels la résistance à l'insuline joue un rôle.

15. Procédé pour la préparation d'un médicament contenant un ou plusieurs des composés selon l'une ou plusieurs des revendications 1 à 3, **caractérisé en ce que** la substance active est mélangée avec un support pharmaceutiquement acceptable et ce mélange est amené dans une forme appropriée pour l'administration.
